# EUROPEAN PATENT APPLICATION

(11) **EP 4 624 899 A1**
(43) Date of publication of application: **01.10.2025**
(21) Application number: 25165729.2
(22) Date of filing: 24.03.2025
(51) Int. Cl.: G01N 21/00, C21D 1/26, A61B 5/00

(54) **PHOTOBLEACHED IMAGING APPARATUS OR CATHETER, AND METHODS FOR USING SAME OR PERFORMING PHOTO-BLEACHING FOR SAME**

(30) Priority: 27.03.2024 US 202463570514 P
(71) Applicant: Canon U.S.A., Inc., Melville, NY 11747 (US)
(72) Inventor: YAMADA, Daisuke, Irvine, California, 92612 (US)
(74) Representative: TBK

(57) **Abstract**

One or more devices, systems, methods and storage mediums for performing photo-bleaching and/or performing intravascular imaging and/or optical coherence tomography (OCT) while detecting and/or characterizing one or more tissues are provided. Examples of applications include imaging, evaluating and diagnosing biological objects, such as, but not limited to, for Gastro-intestinal, cardio and/or ophthalmic applications, and being obtained via one or more optical instruments, such as, but not limited to, optical probes, catheters, capsules and needles (*e.g.,* a biopsy needle). Preferably, the intravascular imaging devices, systems, methods, and storage mediums involve photo-bleaching feature(s) and/or include or involve a method, such as, but not limited to, using one or more images to detect and/or characterize the one or more tissues and/or to perform coregistration. Photo-bleached devices or systems may be used for improved imaging, including for fluorescence devices or systems.

## Description

### CROSS-REFERENCE TO RELATED APPLICATION(S)

This application relates, and claims priority, to U.S. Patent Application Serial No. 63/570,514, filed March 27, 2024, the entire disclosure of which is incorporated by reference herein in its entirety.

### FIELD OF THE DISCLOSURE

This present disclosure generally relates to computer imaging, reducing and/or stabilizing background noise for imaging, and/or to the field of medical imaging, particularly to photo-bleached devices/apparatuses, systems, methods, and storage mediums for performing tissue characterization and/or imaging in one or more images and/or for using one or more imaging modalities, including but not limited to, angiography, Optical Coherence Tomography (OCT), Multi-modality OCT (MM-OCT), near-infrared fluorescence (NIRF), near-infrared auto-fluorescence (NIRAF), OCT-NIRAF, fluorescence, white light back-reflection, near-infrared spectroscopy (NIRS), robot imaging, robot imaging, continuum robot imaging, etc. Examples of OCT applications include imaging, evaluating, and diagnosing biological objects, including, but not limited to, for gastro-intestinal, cardio, and/or ophthalmic applications, and being obtained via one or more optical instruments, including, but not limited to, one or more optical probes, one or more catheters, one or more endoscopes, one or more capsules, and one or more needles *(e.g.,* a biopsy needle). One or more devices, systems, methods and storage mediums for characterizing, examining and/or diagnosing, and/or measuring viscosity of, a sample or object (e.g., tissue, an organ, a portion of a patient, etc.) using an apparatus or system that uses and/or controls one or more imaging modalities are discussed herein.

### BACKGROUND

Fiber optic catheters and endoscopes have been developed to access to internal organs. For example, in cardiology, OCT has been developed to see (e.g., capture and visualize) depth resolved images of vessels with a catheter. The catheter, which may include a sheath, a coil and an optical probe, may be navigated to a coronary artery.

Optical coherence tomography (OCT) is a technique for obtaining high-resolution cross-sectional images of tissues or materials, and OCT enables real time visualization. The aim of the OCT techniques is to measure the time delay of light by using an interference optical system or interferometry, such as via Fourier Transform or Michelson interferometers. A light from a light source delivers and splits into a reference arm and a sample (or measurement) arm with a splitter (*e.g*., a beamsplitter). A reference beam is reflected from a reference mirror (partially reflecting or other reflecting element) in the reference arm while a sample beam is reflected or scattered from a sample in the sample arm. Both beams combine (or are recombined) at the splitter and generate interference patterns. The output of the interferometer is detected with one or more detectors, such as, but not limited to, photodiodes or multi-array cameras, in one or more devices, such as, but not limited to, a spectrometer (*e.g*., a Fourier Transform infrared spectrometer). The interference patterns are generated when the path length of the sample arm matches that of the reference arm to within the coherence length of the light source. By evaluating the output beam, a spectrum of an input radiation may be derived as a function of frequency. The frequency of the interference patterns corresponds to the distance between the sample arm and the reference arm. The higher frequencies are, the greater are the differences in path length. Single mode fibers may be used for OCT optical probes, and double clad fibers may be used for fluorescence and/or spectroscopy. A multi-modality system, such as, but not limited to, an OCT, fluorescence, and/or spectroscopy system with an optical probe, is developed to obtain multiple information at the same time.

Spectrally encoded endoscope (SEE) is an endoscope technology which uses a broadband light source, a rotating or oscillating grating and a spectroscopic detector to encode spatial information from a sample. When illuminating light to the sample, the light is spectrally dispersed along one illumination line, such that the dispersed light illuminates a specific position of the illumination line with a specific wavelength. When the reflected light from the sample is detected with a spectrometer, the intensity distribution is analyzed as the reflectance along the line where the wavelength encodes the spatial information. By rotating or oscillating the grating to scan the illumination line, a two-dimensional image of the sample is obtained.

In order to acquire cross-sectional images of tubes and cavities such as vessels, and/or esophagus and nasal cavities, the optical probe is rotated with a fiber optic rotary joint (FORJ). A FORJ is the interface unit that operates to rotate one end of a fiber and/or an optical probe. In general, a free space beam coupler is assembled to separate a stationary fiber and a rotor fiber inside the FORJ. Besides, the optical probe may be simultaneously translated longitudinally during the rotation so that helical scanning pattern images are obtained. This translation is most commonly performed by pulling the tip of the probe back along a guidewire towards a proximal end and, therefore, referred to as a pullback.

A multi-modality system such as an OCT, fluorescence, and/or spectroscopy system with an optical probe is developed to obtain multiple information at the same time.

In a catheter or endoscope based fluorescence system, a catheter may emit light (which may be or cause a catheter background noise) when an excitation light couples into an optical fiber of the catheter. A silica core optical fiber may be used to deliver the excitation light to a sample or samples, such as tissue(s). However, the silica core fiber generates Raman signals when exciting the fiber (*e.g.,* as discussed in Stolen, et al., "Raman response function of silica-core fibers", J. Opt. Soc. Am. B, Vol. 6, page 1159, 1166 (June 1989), which is incorporated by reference herein in its entirety). The Raman scattering is an origin of the catheter background noise.

Some systems have used a spectral separation between a catheter background noise and the fluorescence from the tissues that use an optimized long pass filter. However, an intensity of the fluorescence from the tissues are also high where a catheter background noise is high. As such, if the background noise is spectrally removed by using a long pass filter, the fluorescence signal(s) is/are also removed, which will worsen the signal to noise ratio (SNR) of the fluorescence system.

Accordingly, it would be desirable to provide at least one imaging or optical apparatus/device, system, method, and storage medium that is able to reduce and stabilize background noise (*e.g*., the catheter background noise) without suffering from losing fluorescence signal(s) and that is able to evaluate and characterize a target, sample, or object (*e.g.,* a tissue, an organ, a part of a patient, a vessel, etc.). It also would be desirable to provide one or more probe/catheter/robot device techniques and/or structure for characterizing the target, sample, or object (*e.g*., a tissue, an organ, a part of a patient, a vessel, etc.) for use in at least one optical device, assembly, or system to achieve consistent, reliable detection, and/or characterization/imaging results at high efficiency and a reasonable cost of manufacture and maintenance.

### SUMMARY

Accordingly, it is a broad object of the present disclosure to provide imaging (*e.g*., OCT, NIRF, NIRAF, white light back-reflection, near-infrared spectroscopy (NIRS), robots, continuum robots, etc.) apparatuses, systems, methods and storage mediums for using and/or controlling multiple imaging modalities, that are able to reduce and stabilize background noise (*e.g.,* the catheter background noise) without suffering from losing fluorescence signal(s) and that are able to evaluate and characterize tissue in one or more images (*e.g*., intravascular images) with greater or maximum success and/or efficiency. It is also a broad object of the present disclosure to provide OCT devices, systems, methods, and storage mediums using an interference optical system, such as an interferometer (*e.g*., spectral-domain OCT (SD-OCT), swept-source OCT (SS-OCT), multimodal OCT (MM-OCT), Intravascular Ultrasound (IVUS), Near-Infrared Autofluorescence (NIRAF), Near-Infrared Spectroscopy (NIRS), Near-Infrared Fluorescence (NIRF), therapy modality using light, sound, or other source of radiation, etc.).

Further, it is a broad object of the present disclosure to provide one or more methods or techniques that operate to one or more of the following: (i) detect one or more tissue types (*e.g*., calcium, lipids, fibrous tissue, mixed tissue, other tissue, etc.) automatically in an entire or whole pullback of catheter or probe for one or more intravascular images (such as, but not limited to, OCT images); (ii) reduce computational time to characterize the pullback by processing one image (*e.g.,* one image only may be processed instead of a plurality (*e.g.,* 400) of images, a carpet view image may be constructed and processed, an intravascular image may be constructed and processed, etc.) in one or more embodiments; (iii) provide accurate fluorescence measurement results, and prevent or avoid incorrect measurement results due to any degradation of one or more detectors; (iv) reduce and stabilize background noise (*e.g*., the catheter background noise) without suffering from losing fluorescence signal(s); (v) increase a signal to noise ratio and/or become more sensitive to detect weak fluorescence (*e.g*., NIRF, NIRAF, etc.) signals; (vi) using a photo-bleached optical probe for a catheter and/or one or more methods for the photo-bleaching optical probe; and/or (vii) perform a more detailed tissue detection or characterization and/or imaging in one or more embodiments.

To overcome the aforementioned issue of having catheter background noise, several methodologies of the present disclosure have been developed which use an apparatus, a system, a method, a storage medium, etc. that operate to one or more of the following: reduce and stabilize the catheter background noise without suffering from losing fluorescence signal(s), increase signal to noise ratio, become more sensitive to detect weak fluorescence (*e.g*., NIRF, NIRAF, etc.) signals, and/or use a photo-bleached optical probe for a catheter. In one or more embodiments, to increase the SNR, the background noise may be reduced while the fluorescence signal may not be reduced.

As aforementioned, the fiber optic catheters and endoscopes of the present disclosure have been developed to access internal organs, tissues, or other targets, samples, or objects. For example in the cardiology, OCT (optical coherence tomography), white light back-reflection, NIRS (near infrared spectroscopy), and fluorescence technology have been developed to see structural and/or molecular images of vessels with a catheter. The catheter, which comprises a sheath and an optical probe in one or more embodiments, may be navigated to a target, sample, or object, such as, but not limited to, a coronary artery.

In order to acquire cross-sectional images of tubes and cavities, such as, but not limited to, vessels, an esophagus, and at least one nasal cavity, the optical probe may be rotated with a fiber optic rotary joint (FORJ). In addition, the optical probe may be simultaneously translated longitudinally during the rotation so that helical scanning pattern images are obtained. This translation may be performed by pulling the tip of the probe back towards a proximal end, and this translation is, therefore, referred to as a pullback. While particular tubes, cavities, or other targets, samples, or objects (*e.g*., coronary arteries) may be discussed herein, the targets, samples, or objects for which the features of the present disclosure may be used are not limited thereto. Additionally, while particular imaging modalities that may be used in combination are discussed herein (*e.g.*, an intravascular OCT and fluorescence system), the imaging modalities that may be used with one or more features of the present disclosure are not limited thereto.

In one or more embodiments, a photo-bleached imaging apparatus may include: a catheter having an optical probe having one or more optical fibers that operate to deliver and receive light, wherein the optical probe or one or more components of the optical probe is/are photo-bleached. In one or more embodiments, an optical system may include: an interference optical system that operates to: (i) receive and divide light from a light source into a first light with which an object or sample is to be irradiated and which travels along a sample arm of the interference optical system and a second reference light, (ii) send the second reference light along a reference arm of the interference optical system for reflection off of a reference reflection of the interference optical system, and (iii) generate interference light by causing reflected or scattered light of the first light with which the object or sample has been irradiated and the reflected second reference light to combine or recombine, and to interfere, with each other, the interference light generating one or more interference patterns; one or more detectors that operate to continuously acquire the interference light and/or the one or more interference patterns to measure the interference or the one or more interference patterns between the combined or recombined light to obtain data for one or more imaging modalities, wherein a wavelength of the first light is shorter than a wavelength of the reflected or scattered light and/or the generated interference light, and wherein the interference optical system or a probe of the interference optical system is photo-bleached. In one or more embodiments, the interference optical system or a probe of the interference optical system may include a double clad fiber. In one or more embodiments, an emission intensity of the photo-bleached interference optical system or the photo-bleached probe may stabilize within 10% of an averaged intensity over a predetermined or set period of time (*e.g*., a period of two minutes, a period of about two minutes, a period of time in a range of one minute to two minutes, a period of time in a range of about one minute to about two minutes, etc.).

In one or more embodiments, the one or more imaging modalities may include one or more of the following: Optical Coherence Tomography (OCT), single modality OCT, multi-modality OCT, swept source OCT, optical frequency domain imaging (OFDI), intravascular ultrasound (IVUS), another lumen image(s) modality, near-infrared spectroscopy (NIRS), near-infrared fluorescence (NIRF), near-infrared auto-fluorescence (NIRAF), near-infrared, fluorescence, and an intravascular imaging modality.

In one or more embodiments, a method for photo-bleaching an interference optical system and/or one or more optical probes may include: using or providing an excitation laser having a wavelength of 400 nm - 900 nm; coupling the excitation laser into the interference optical system, the one or more optical probes, and/or one or more components of the one or more optical probes; and exciting the interference optical system, the one or more optical probes, and/or one or more components of the one or more optical probes with the excitation laser for more than or equal to a set or predetermined amount of time. In one or more embodiments, the set or predetermined amount of time is thirty (30) minutes or is about thirty (30) minutes. In one or more embodiments, a user may set the amount of time for performing the photo-bleaching. In one or more embodiments, the one or more optical probes and/or the one or more components of the one or more optical probes may include or comprise of (or may each include or comprise of) a double clad fiber. In one or more embodiments, the set or predetermined amount of time for performing the excitation may be for 24 hours or more than 24 hours. In one or more embodiments, the excitation laser or light may have a 635 nm wavelength.

In one or more embodiments, one or more processors may perform or control the method for photo-bleaching. The one or more processors may receive the set or predetermined amount of time to perform the photo-bleaching or may automatically calculate and set the set or predetermined amount of time (*e.g*., based on a number of components or structure to be photo-bleached, based on a size and shape of the structure or the number of components to be photo-bleached, etc.).

In one or more embodiments, a photo-bleached imaging apparatus may include: an interference optical system that operates to: (i) receive and divide light from a light source into a first light with which an object or sample is to be irradiated and which travels along a sample arm of the interference optical system and a second reference light, (ii) send the second reference light along a reference arm of the interference optical system for reflection off of a reference reflection of the interference optical system, and (iii) generate interference light by causing reflected or scattered light of the first light with which the object or sample has been irradiated and the reflected second reference light to combine or recombine, and to interfere, with each other, the interference light generating one or more interference patterns; and one or more detectors that operate to continuously acquire the interference light and/or the one or more interference patterns to measure the interference or the one or more interference patterns between the combined or recombined light to obtain data for one or more imaging modalities, wherein a wavelength of the first light is shorter than a wavelength of the reflected or scattered light and/or the generated interference light, and the interference optical system or one or more components of an optical probe or catheter of the interference optical system is/are photo-bleached. In one or more embodiments, one or more of the following may occur: (i) the one or more detectors operate to continuously acquire the interference light and/or the one or more interference patterns in the photo-bleached interference optical system, optical probe, or catheter such that an emission intensity of the photo-bleached interference optical system, optical probe, or catheter stabilizes within 10% or about 10% of an averaged intensity over a predetermined or set period of time and/or such that the interference optical system has a higher signal to noise ratio as compared to an interference optical system, optical probe, or catheter without being photo-bleached; and/or (ii) the interference optical system or the optical probe or the catheter of the interference optical system includes a double clad fiber. One or more of the following may occur: (i) an emission intensity of the photo-bleached interference optical system, optical probe, or catheter stabilizes within 10% or about 10% of an averaged intensity over a predetermined or set period of time; and/or (ii) the predetermined or set period of time is one of the following: two minutes, about two minutes, a period of time in a range of one minute to two minutes, and/or a period of time in a range of about one minute to about two minutes.

An imaging apparatus may include one or more processors that operate to perform a pullback of the optical probe or the catheter and/or obtain one or more images or frames of one or more imaging modalities from the pullback of the optical probe or the catheter. In one or more embodiments, the one or more imaging modalities may include one or more of the following: Optical Coherence Tomography (OCT), single modality OCT, multi-modality OCT, swept source OCT, optical frequency domain imaging (OFDI), intravascular ultrasound (IVUS), another lumen image(s) modality, near-infrared spectroscopy (NIRS), near-infrared fluorescence (NIRF), near-infrared auto-fluorescence (NIRAF), near-infrared, fluorescence, and/or an intravascular imaging modality. The one or more processors may further operate to display the one or more images on a display, store the one or more images in a memory, or use the one or more images to train one or more models or AI-networks to auto-detect or to perform photo-bleaching and/or to automatically obtain one or more images of the one or more imaging modalities. One or more of the following may occur: (i) the trained model may be one or a combination of the following: a neural net model or neural network model, a deep convolutional neural network model, a recurrent neural network model with long short-term memory that can take temporal relationships across images or frames into account, a generative adversarial network (GAN) model, a consistent generative adversarial network (cGAN) model, a three cycle-consistent generative adversarial network (3cGAN) model, a model that can take temporal relationships across images or frames into account, a model that can take temporal relationships into account including tissue location(s) and/or photo-bleach location(s) during pullback in a vessel and/or including tissue and/or photo-bleach characterization data during pullback in a vessel, a model that can use prior knowledge about a procedure and incorporate the prior knowledge into the machine learning algorithm or a loss function, a model using feature pyramid(s) that can take different image resolutions into account, and/or a model using residual learning technique(s), a segmentation model, a segmentation model with post-processing, a model with pre-processing, a model with post-processing, a segmentation model with pre-processing, a deep learning or machine learning model, a semantic segmentation model or classification model, an object detection or regression model, an object detection or regression model with pre-processing or post-processing, a combination of a semantic segmentation model and an object detection or regression model, a model using repeated segmentation model technique(s), a model using feature pyramid(s), a genetic algorithm that operates to breed multiple models for improved performance, and/or a model using repeated object detection or regression model technique(s); and/or (ii) the one or more processors may further operate to use one or more neural networks or convolutional neural networks to one or more of: load a trained model of images including photo-bleached area(s); perform photo-bleaching on the optical probe and/or the catheter; determine whether the photo-bleached area(s) is/are accurate or correct; determine one or more of the characteristics of one or more objects, targets, or samples in the one or more images; identify or detect the one or more objects, targets, or samples; overlay data on at least one of the one or more images to show location(s) of intravascular image(s), the photo-bleached area(s), or the objects, targets, or samples; incorporate image processing and machine learning (ML) or deep learning to automatically identify and locate photo-bleached portions or components of the interference optical system, the optical probe, or the catheter; incorporate image processing and machine learning (ML) or deep learning to automatically identify and locate the one or more objects, targets, or samples; display the results for the photo-bleach identification/detection or characterization on a display; and/or acquire or receive image data during the pullback operation of the catheter or the optical probe.

In one or more embodiments, an imaging apparatus may further include one or more of the following: (i) the light source that operates to produce the light; (ii) the light source that operates to produce the light, the light source producing the light to operate as an excitation laser or light having a wavelength of 400 nm - 900 nm or 635 nm; (iii) the light source that operates to produce the light, the light source producing the light as an excitation laser or light and coupling the excitation laser or light into the interference optical system, the optical probe, and/or one or more components of the optical probe and/or of the catheter; (iv) the light source that operates to produce the light, the light source producing the light as an excitation laser or light and exciting the interference optical system, the optical probe, and/or one or more components of the optical probe and/or of the catheter with the excitation laser or light for more than or equal to a set or predetermined amount of time; and/or (v) the light source that operates to produce the light, the light source producing the light as an excitation laser or light and exciting the interference optical system, the optical probe, and/or one or more components of the optical probe and/or of the catheter with the excitation laser or light for more than or equal to a set or predetermined amount of time, where the set or predetermined amount of time is one or more of the following: thirty (30) minutes, thirty (30) minutes or more, in a range of thirty (30) minutes to twenty-four (24) hours, twenty-four (24) hours, twenty-four (24) hours or more, an amount of time calculated or set/received by one or more processors of the imaging apparatus or by a user of the imaging apparatus, and/or an amount of time calculated or set by the one or more processors of the imaging apparatus or by the user of the imaging apparatus based on a size and shape to be photo-bleached or based a number of components or structure to be photo-bleached.

In a case where the interference optical system, the optical probe or catheter, or one or more components of the optical probe or catheter include or are attached to a double clad fiber, one or more of the following may exist: (i) the imaging apparatus further comprises one or more processors that operate to perform a pullback of the optical probe or the catheter and/or obtain one or more images or frames of one or more imaging modalities from the pullback of the optical probe or the catheter; (ii) the imaging apparatus further comprises: one or more processors that operate to perform a pullback of the optical probe or the catheter and/or obtain one or more images or frames of one or more imaging modalities from the pullback of the optical probe or the catheter, and the one or more processors further include or operate to be used with a core/clad ratio adjustment processor or unit that operates to control a ratio of excitation laser or light between a core and a clad of the double clad fiber; (iii) the imaging apparatus further comprises: one or more processors that operate to perform a pullback of the optical probe or the catheter and/or obtain one or more images or frames of one or more imaging modalities from the pullback of the optical probe or the catheter, and the one or more processors further include or operate to be used with a core/clad ratio adjustment processor or unit that operates to control a ratio of an excitation laser or light between a core and a clad of the double clad fiber, wherein the ratio is one or more of the following: 10% or more than 10% of the excitation laser or light being sent to the clad so that a ratio value for the amount of the excitation laser or light being sent to the core is 90% or less than 90%; 50% or about 50% of the excitation laser or light sent to the clad and 50% or about 50% or more of the excitation laser or light sent to the core; 47% to the clad and 53% to the core; and/or 50%-x% to the clad and 50%+x% to the core where x% is a value equal to a difference between 50% and the percentage value going to the clad; and/or (iv) the interference optical system further comprises a fluorescence sub-system and a sub-system for another imaging modality.

An imaging apparatus may further include a lens unit or one or more lens components that operate to filter an excitation laser or light of the light source and pass through the emission to and/or from the interference optical system, the optical probe, and/or one or more components of the optical probe and/or of the catheter. One or more of the following may occur: (i) the one or more components of the optical probe and/or the catheter include or comprise a double clad fiber; and/or (ii) an optical power of the excitation laser or light into the interference optical system, the optical probe, and/or one or more components of the optical probe and/or of the catheter in total is one of the following: same as a nominal intensity as compared to a case where the excitation laser or light is used as part of a system, the interference optical system, the optical probe, and/or one or more components of the optical probe and/or of the catheter, which is at least 0.1 mW; two (2) times or more higher than the nominal intensity, which is at least 0.2 mW or at least 0.5 mW; ten (10) times or more higher than the nominal intensity, which is at least 1 mW; and/or a hundred (100) times or more higher than the nominal intensity, which is at least 10 mW.

In one or more embodiments, a method for photo-bleaching an optical probe and/or one or more components of the optical probe of an imaging apparatus may include: using an excitation laser or light with a wavelength of a predetermined range or value on or in the optical probe, the optical probe for use in a catheter and/or in one or more components of the optical probe for a predetermined or set amount of time or more to perform the photo-bleaching such that lower and stabilized background emission noise and/or a high signal to noise ratio is/are achieved for the optical probe and/or for the one or more components of the optical probe. In one or more embodiments, a method for photo-bleaching an interference optical system, an optical probe, and/or one or more components of the optical probe and/or of a catheter of an imaging apparatus may include: using an excitation laser or light with a wavelength of a predetermined range or value on or in the interference optical system, the optical probe, and/or one or more components of the optical probe and/or of a catheter for a predetermined or set amount of time or more to perform the photo-bleaching such that lower and stabilized background emission noise and/or a high signal to noise ratio is/are achieved for the interference optical system, the optical probe, and/or the one or more components of the optical probe and/or of the catheter. In one or more embodiments, the predetermined range or value is one or more of the following: 400 nm - 900 nm and/or 635 nm; and the predetermined or set amount of time is one or more of the following: thirty (30) minutes, thirty (30) minutes or more, in a range of thirty (30) minutes to twenty-four (24) hours, twenty-four (24) hours, twenty-four (24) hours or more, an amount of time calculated or set/received by one or more processors of the imaging apparatus or by a user of the imaging apparatus, and/or an amount of time calculated or set by the one or more processors of the imaging apparatus or by the user of the imaging apparatus based on a size and shape to be photo-bleached or based a number of components or structure to be photo-bleached. The method may further include one or more of the following: (i) using one or more detectors of the imaging apparatus to continuously acquire the interference light and/or the one or more interference patterns in the photo-bleached optical interference system, optical probe, or catheter such that an emission intensity of the photo-bleached optical interference system, optical probe, or catheter stabilizes within 10% or about 10% of an averaged intensity over a predetermined or set period of time and/or such that the interference optical system has a higher signal to noise ratio as compared to an interference optical system without being photo-bleached; and/or (ii) using the interference optical system or the optical probe or the catheter of the interference optical system while including a double clad fiber. Any method of the present disclosure may use any feature as discussed for an apparatus, system, other method, a storage medium, Artificial Intelligence (AI) system or method, etc. of the present disclosure. For example, the interference optical system may operate to: (i) receive and divide light from a light source into a first light with which an object or sample is to be irradiated and which travels along a sample arm of the interference optical system and a second reference light, (ii) send the second reference light along a reference arm of the interference optical system for reflection off of a reference reflection of the interference optical system, and (iii) generate interference light by causing reflected or scattered light of the first light with which the object or sample has been irradiated and the reflected second reference light to combine or recombine, and to interfere, with each other, the interference light generating one or more interference patterns; the imaging apparatus includes one or more detectors that operate to continuously acquire the interference light and/or the one or more interference patterns to measure the interference or the one or more interference patterns between the combined or recombined light to obtain data for one or more imaging modalities; a wavelength of the first light is shorter than a wavelength of the reflected or scattered light and/or the generated interference light, and the interference optical system or one or more components of an optical probe or catheter of the interference optical system is/are photo-bleached.

In one or more embodiments, a computer-readable storage medium storing at least one program that operates to cause one or more processors to execute a method for photo-bleaching an interference optical system, an optical probe, and/or one or more components of the optical probe and/or of a catheter of an imaging apparatus may be used where the method may include any feature discussed herein, including, but not limited to: using an excitation laser or light with a wavelength of a predetermined range or value on or in the interference optical system, the optical probe, and/or one or more components of the optical probe and/or of a catheter for a predetermined or set amount of time or more to perform the photo-bleaching such that lower and stabilized background emission noise and/or a high signal to noise ratio is/are achieved for the interference optical system, the optical probe, and/or the one or more components of the optical probe and/or of the catheter.

In one or more embodiments, the object, target, or sample may include one or more of the following: a vessel; a target, a specimen, or object; a tissue or tissues; a patient; an interference optical system; one or more optical probes; and/or one or more components of the one or more optical probes.

The one or more processors may further operate to perform the coregistration by co-registering an acquired or received angiography image or the constructed image (e.g., a carpet view) and an obtained one or more intravascular images, such as, but not limited to, OCT or IVUS images or frames.

In one or more embodiments, a loaded, trained model may be one or a combination of the following: a segmentation (classification) model, a segmentation model with pre-processing, a segmentation model with post-processing, an object detection (regression) model, an object detection model with pre-processing, an object detection model with post-processing, a combination of a segmentation (classification) model and an object detection (regression) model, a deep convolutional neural network model, a recurrent neural network model with long short-term memory that can take temporal relationships across images or frames into account, a model using feature pyramid(s) that can take different image resolutions into account, a genetic algorithm that operates to breed multiple models for improved performance (as compared with a case where the genetic algorithm is not used), a model using residual learning technique(s), and/or any other model discussed herein or known to those skilled in the art.

In one or more embodiments, the one or more processors may further operate to one or more of the following: (i) display an image for each of one or more imaging modalities on a display, wherein the one or more imaging modalities include one or more of the following: a tomography image; an Optical Coherence Tomography (OCT) image; a fluorescence image; a near-infrared auto-fluorescence (NIRAF) image; a near-infrared auto-fluorescence (NIRAF) image in a predetermined view, a carpet view, and/or an indicator view; a near-infrared fluorescence (NIRF) image, a near-infrared fluorescence (NIRF) image in a predetermined view, a carpet view, and/or an indicator view; a near-infrared spectroscopy (NIRS) image; a three-dimensional (3D) rendering; a 3D rendering of a vessel; a 3D rendering of a vessel in a half-pipe view or display; a 3D rendering of the object; a lumen profile; a lumen diameter display; a longitudinal view; computer tomography (CT); Magnetic Resonance Imaging (MRI); Intravascular Ultrasound (IVUS); an X-ray image or view; and an angiography view; and (ii) change or update the displays based on the tissue(s) or tissue characteristic(s) evaluation results, based on the photo-bleach evaluation results, and/or based on an updated location of the probe or catheter.

One or more embodiments of a non-transitory computer-readable storage medium storing at least one program for causing a computer to execute a method for training a model using artificial intelligence may be used with any method(s) discussed in the present disclosure, including but not limited to, one or more tissue(s) or tissue characteristic(s) evaluation/determination method(s), one or more photo-bleach characteristic(s) evaluation/determination and/or performance method(s).

One or more embodiments of any method discussed herein (e.g., training method(s), detecting method(s), imaging or visualization method(s), photo-bleach method(s), artificial intelligence method(s), etc.) may be used with any feature or features of the apparatuses, systems, other methods, storage mediums, or other structures discussed herein.

One or more of the artificial intelligence features discussed herein that may be used in one or more embodiments of the present disclosure, includes but is not limited to, using one or more of deep learning, a computer vision task, keypoint detection, a unique architecture of a model or models, a unique training process or algorithm, a unique optimization process or algorithm, input data preparation techniques, input mapping to the model, pre-processing, post-processing, and/or interpretation of the output data as substantially described herein or as shown in any one of the accompanying drawings.

In one or more embodiments, tissue(s) and or characteristic(s) of one or more tissues and/or photo-bleaching may be evaluated and determined using an algorithm, such as, but not limited to, the Viterbi algorithm.

One or more embodiments of the present disclosure may track and/or calculate a tissue(s) or tissue characteristic(s) evaluation success rate and/or a photo-bleach or photo-bleach characteristic(s) evaluation success rate.

The following paragraphs describe certain explanatory embodiments. Other embodiments may include alternatives, equivalents, and modifications. Additionally, the explanatory embodiments may include several novel features, and a particular feature may not be essential to some embodiments of the devices, systems, and methods that are described herein.

According to other aspects of the present disclosure, one or more additional devices, one or more systems, one or more methods and one or more storage mediums using OCT and/or other imaging modality technique(s) to perform tissue characterization, to perform photo-bleaching and/or photo-bleach characterization, and to perform coregistration using artificial intelligence, including, but not limited to, deep or machine learning, using results of the tissue detection and/or tissue characterization and/or using results of the photo-bleaching and/or photo-bleach characterization for performing coregistration, etc., are discussed herein. Further features of the present disclosure will in part be understandable and will in part be apparent from the following description and with reference to the attached drawings.

In accordance with one or more embodiments of the present disclosure, apparatuses and systems, and methods and storage mediums for tissue detection and/or tissue characterization and/or for photo-bleaching and/or photo-bleach characterization in one or more images may operate to characterize biological objects, such as, but not limited to, blood, mucus, tissue (including different types of tissue), etc.

It should be noted that one or more embodiments of the tissue detection and/or characterization method(s) or feature(s) and/or one or more embodiments of the photo-bleaching and/or photo-bleach characterization method(s) or feature(s) of the present disclosure may be used in other imaging systems, apparatuses or devices, where images are formed from signal reflection and scattering within tissue sample(s) using a scanning probe. For example, IVUS images may be processed in addition to or instead of OCT images.

One or more embodiments of the present disclosure may be used in clinical application(s), such as, but not limited to, intervascular imaging, atherosclerotic plaque assessment, cardiac stent evaluation, balloon sinuplasty, sinus stenting, arthroscopy, ophthalmology, ear research, veterinary use and research, etc.

In accordance with at least another aspect of the present disclosure, one or more technique(s) discussed herein may be employed to reduce the cost of at least one of manufacture and maintenance of the one or more apparatuses, devices, systems and storage mediums by reducing or minimizing a number of optical components and by virtue of the efficient techniques to cut down cost of use/manufacture of such apparatuses, devices, systems and storage mediums.

According to other aspects of the present disclosure, one or more additional devices, one or more systems, one or more methods and one or more storage mediums using, or for use with, one or more tissue detection and/or tissue characterization techniques and/or one or more photo-bleaching and/or photo-bleach characterization techniques are discussed herein. Further features of the present disclosure will in part be understandable and will in part be apparent from the following description and with reference to the attached drawings.

### BRIEF DESCRIPTION OF THE DRAWINGS

For the purposes of illustrating various aspects of the disclosure, wherein like numerals indicate like elements, there are shown in the drawings simplified forms that may be employed, it being understood, however, that the disclosure is not limited by or to the precise arrangements and instrumentalities shown. To assist those of ordinary skill in the relevant art in making and using the subject matter hereof, reference is made to the appended drawings and figures, wherein:
FIG. 1A is a schematic diagram showing at least one embodiment of a system that may be used for performing one or multiple imaging modality viewing and control and/or for at least photo-bleaching and/or tracking/evaluating photo-bleach characteristic(s) in accordance with one or more aspects of the present disclosure;
FIG. 1B is a schematic diagram illustrating an imaging system for executing one or more steps to process image data and/or for at least detecting or identifying photo-bleaching and/or tracking/evaluating photo-bleach characteristic(s) in accordance with one or more aspects of the present disclosure;
FIG. 2 is a diagram of at least one embodiment of a catheter that may be used with one or more embodiments for at least photo-bleaching and/or tracking/evaluating photo-bleach characteristic(s) in accordance with one or more aspects of the present disclosure;
FIGS. 3A-3B are diagrams showing embodiments of one or more multi-modality systems (such as, but not limited to, OCT and fluorescence) which can utilize one or more detectors, one or more processors, and/or one or more photo-bleaching feature(s) or technique(s) in accordance with one or more aspects of the present invention;
FIG. 4 is a schematic diagram of at least one free space beam combiner in a Patient Interface Unit (PIU) (that may be used with a FORJ) that may be used in accordance with one or more aspects of the present disclosure;
FIG. 5 shows a graph for at least one embodiment example how emission noise from at least one optical probe varies when exciting the at least one optical probe that may be used in accordance with one or more aspects of the present disclosure;
FIG. 6 shows a graph for at least one embodiment of an application where an intensity change of an emission is within 10% over two minutes in accordance with one or more aspects of the present disclosure;
FIG. 7 shows a schematic diagram of at least one apparatus or system that may be used for photo-bleaching in accordance with one or more aspects of the present disclosure;
FIG. 8A shows a graph for at least one test after performing photo-bleaching in accordance with one or more aspects of the present disclosure;
FIG. 8B shows at least one embodiment of a method for performing photo-bleaching in accordance with one or more aspects of the present disclosure;
FIG. 9A shows at least one embodiment of an apparatus or system for utilizing one or more imaging modalities and/or artificial intelligence for performing imaging, photo-bleaching, and/or co-registration in accordance with one or more aspects of the present disclosure;
FIG. 9B shows at least another embodiment of an imaging apparatus or system for utilizing one or more imaging modalities and artificial intelligence for performing imaging, photo-bleaching, and/or co-registration in accordance with one or more aspects of the present disclosure;
FIG. 9C shows at least a further embodiment of an imaging (such as, but not limited to, OCT and NIRF/NIRAF, OCT and fluorescence, etc.) apparatus or system for utilizing one or more imaging modalities and artificial intelligence for performing imaging, photo-bleaching, and/or co-registration in accordance with one or more aspects of the present disclosure;
FIG. 10 is a flow diagram showing a method of performing an imaging feature, function, or technique in accordance with one or more aspects of the present disclosure;
FIG. 11 shows a schematic diagram of an embodiment of a computer that may be used with one or more embodiments of an apparatus or system or one or more methods discussed herein in accordance with one or more aspects of the present disclosure;
FIG. 12 shows a schematic diagram of another embodiment of a computer that may be used with one or more embodiments of an imaging apparatus or system or methods discussed herein in accordance with one or more aspects of the present disclosure;
FIG. 13 shows a schematic diagram of at least an embodiment of a system using a computer or processor, a memory, a database, and input and output devices in accordance with one or more aspects of the present disclosure;
FIG. 14 shows a created architecture of or for a regression model(s) that may be used for imaging, tissue characterization, tissue identification, photo-bleaching, performing co-registration, and/or any other technique discussed herein in accordance with one or more aspects of the present disclosure;
FIG. 15 shows a convolutional neural network architecture that may be used for imaging, photo-bleaching, photo-bleach characterization, tissue characterization, tissue detection, and/or any other technique discussed herein in accordance with one or more aspects of the present disclosure;
FIG. 16 shows a created architecture of or for a regression model(s) that may be used for imaging, photo-bleaching, photo-bleach characterization, tissue characterization, tissue detection, and/or any other technique discussed herein in accordance with one or more aspects of the present disclosure; and
FIG. 17 is a schematic diagram of or for a segmentation model(s) that may be used for imaging, photo-bleaching, photo-bleach characterization, tissue characterization, tissue detection, and/or any other technique discussed herein in accordance with one or more aspects of the present disclosure.

### DETAILED DESCRIPTION

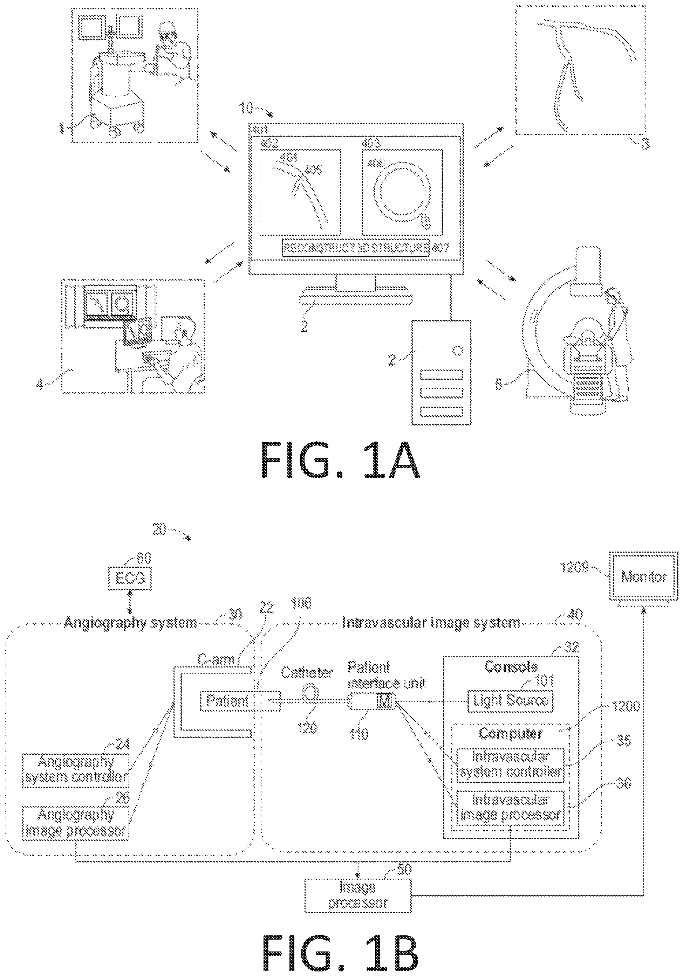

One or more devices, systems, methods and storage mediums for characterizing tissue, or an object, using one or more imaging techniques or modalities (such as, but not limited to, OCT, fluorescence, IVUS, MRI, CT, NIRF, NIRAF, NIRS, etc.), and using artificial intelligence for evaluating and detecting photo-bleaching and/or photo-bleaching characteristics, detecting tissue types and/or characteristics, and/or performing coregistration are disclosed herein. Several embodiments of the present disclosure, which may be carried out by the one or more embodiments of an apparatus, system, method and/or computer-readable storage medium of the present disclosure are described diagrammatically and visually in at least FIGS. 1A through 17 and further discussed below.

One or more embodiments of the present disclosure provide at least one imaging or optical apparatus/device, system, method, and storage medium that may perform photo-bleaching and/or evaluate/determine photo-bleach characteristics.

One or more embodiments of the present disclosure provide at least one imaging or optical apparatus/device, system, method, and storage medium that may evaluate and characterize a target, sample, or object (*e*.*g*., a tissue, an organ, a part of a patient, a vessel, etc.). One or more embodiments of the present disclosure may also provide or use one or more probe/catheter/robot device techniques and/or structure for characterizing the target, sample, or object (*e*.*g*., a tissue, an organ, a part of a patient, a vessel, etc.) for use in at least one optical device, assembly, or system to achieve consistent, reliable detection, and/or characterization results at high efficiency and a reasonable cost of manufacture and maintenance.

One or more embodiments of the present disclosure provide imaging (*e.g*., OCT, NIRF, NIRAF, robots, continuum robots, etc.) apparatuses, systems, methods and storage mediums for using and/or controlling multiple imaging modalities, that may apply machine learning, especially deep learning, to perform photo-bleaching and/or evaluate and characterize tissue in one or more images (*e.g*., intravascular images) with greater or maximum success. One or more embodiments of the present disclosure may operate to provide OCT devices, systems, methods, and storage mediums using an interference optical system, such as an interferometer (*e.g*., spectral-domain OCT (SD-OCT), swept-source OCT (SS-OCT), multimodal OCT (MM-OCT), Intravascular Ultrasound (IVUS), Near-Infrared Autofluorescence (NIRAF), Near-Infrared Spectroscopy (NIRS), Near-Infrared Fluorescence (NIRF), therapy modality using light, sound, or other source of radiation, etc.).

Accordingly, it is a broad object of the present disclosure to provide imaging (*e.g*., OCT, NIRF, NIRAF, white light back-reflection, near-infrared spectroscopy (NIRS), robots, continuum robots, etc.) apparatuses, systems, methods and storage mediums for using and/or controlling multiple imaging modalities, that are able to reduce and stabilize background noise (*e.g.,* the catheter background noise) without suffering from losing fluorescence signal(s) and that are able to evaluate and characterize tissue in one or more images (*e.g*., intravascular images) with greater or maximum success and/or efficiency. It is also a broad object of the present disclosure to provide OCT devices, systems, methods, and storage mediums using an interference optical system, such as an interferometer (*e.g*., spectral-domain OCT (SD-OCT), swept-source OCT (SS-OCT), multimodal OCT (MM-OCT), Intravascular Ultrasound (IVUS), Near-Infrared Autofluorescence (NIRAF), Near-Infrared Spectroscopy (NIRS), Near-Infrared Fluorescence (NIRF), therapy modality using light, sound, or other source of radiation, etc.).

Further, it is a broad object of the present disclosure to provide one or more methods or techniques that operate to one or more of the following: (i) detect one or more tissue types (*e.g*., calcium, lipids, fibrous tissue, mixed tissue, other tissue, etc.) automatically in an entire or whole pullback of catheter or probe for one or more intravascular images (such as, but not limited to, OCT images); (ii) reduce computational time to characterize the pullback by processing one image (*e.g.,* one image only may be processed instead of a plurality (*e.g.,* 400) of images, a carpet view image may be constructed and processed, an intravascular image may be constructed and processed, etc.) in one or more embodiments; (iii) provide accurate fluorescence measurement results, and prevent or avoid incorrect measurement results due to any degradation of one or more detectors; (iv) reduce and stabilize background noise (*e.g*., the catheter background noise) without suffering from losing fluorescence signal(s); (v) increase a signal to noise ratio and/or become more sensitive to detect weak fluorescence (*e.g*., NIRF, NIRAF, etc.) signals; (vi) using a photo-bleached optical probe for a catheter and/or one or more methods for the photo-bleaching optical probe; and/or (vii) perform a more detailed tissue detection or characterization and/or imaging in one or more embodiments.

To overcome the aforementioned issue of having catheter background noise, several methodologies of the present disclosure have been developed which use an apparatus, a system, a method, a storage medium, etc. that operate to one or more of the following: reduce and stabilize the catheter background noise without suffering from losing fluorescence signal(s), increase signal to noise ratio, become more sensitive to detect weak fluorescence (*e.g*., NIRF, NIRAF, etc.) signals, and/or use a photo-bleached optical probe for a catheter. In one or more embodiments, to increase the SNR, the background noise may be reduced while the fluorescence signal may not be reduced.

As aforementioned, the fiber optic catheters and endoscopes of the present disclosure have been developed to access internal organs, tissues, or other targets, samples, or objects. For example in the cardiology, OCT (optical coherence tomography), white light back-reflection, NIRS (near infrared spectroscopy), and fluorescence technology have been developed to see structural and/or molecular images of vessels with a catheter. The catheter, which comprises a sheath and an optical probe in one or more embodiments, may be navigated to a target, sample, or object, such as, but not limited to, a coronary artery.

In order to acquire cross-sectional images of tubes and cavities, such as, but not limited to, vessels, an esophagus, and at least one nasal cavity, the optical probe may be rotated with a fiber optic rotary joint (FORJ). In addition, the optical probe may be simultaneously translated longitudinally during the rotation so that helical scanning pattern images are obtained. This translation may be performed by pulling the tip of the probe back towards a proximal end, and this translation is, therefore, referred to as a pullback. While particular tubes, cavities, or other targets, samples, or objects (*e.g*., coronary arteries) may be discussed herein, the targets, samples, or objects for which the features of the present disclosure may be used are not limited thereto. Additionally, while particular imaging modalities that may be used in combination are discussed herein (*e.g*., an intravascular OCT and fluorescence system), the imaging modalities that may be used with one or more features of the present disclosure are not limited thereto.

In one or more embodiments, an optical system may include: an interference optical system that operates to: (i) receive and divide light from a light source into a first light with which an object or sample is to be irradiated and which travels along a sample arm of the interference optical system and a second reference light, (ii) send the second reference light along a reference arm of the interference optical system for reflection off of a reference reflection of the interference optical system, and (iii) generate interference light by causing reflected or scattered light of the first light with which the object or sample has been irradiated and the reflected second reference light to combine or recombine, and to interfere, with each other, the interference light generating one or more interference patterns; one or more detectors that operate to continuously acquire the interference light and/or the one or more interference patterns to measure the interference or the one or more interference patterns between the combined or recombined light to obtain data for one or more imaging modalities, wherein a wavelength of the first light is shorter than a wavelength of the reflected or scattered light and/or the generated interference light, and wherein the interference optical system or a probe of the interference optical system is photo-bleached. In one or more embodiments, the interference optical system or a probe of the interference optical system may include a double clad fiber. In one or more embodiments, an emission intensity of the photo-bleached interference optical system or the photo-bleached probe may stabilize within 10% of an averaged intensity over a predetermined or set period of time (*e*.*g*., a period of two minutes, a period of about two minutes, a period of time in a range of one minute to two minutes, a period of time in a range of about one minute to about two minutes, etc.).

In one or more embodiments, the one or more imaging modalities may include one or more of the following: Optical Coherence Tomography (OCT), single modality OCT, multi-modality OCT, swept source OCT, optical frequency domain imaging (OFDI), intravascular ultrasound (IVUS), another lumen image(s) modality, near-infrared spectroscopy (NIRS), near-infrared fluorescence (NIRF), near-infrared auto-fluorescence (NIRAF), near-infrared, fluorescence, and an intravascular imaging modality.

In one or more embodiments, a method for photo-bleaching an interference optical system and/or one or more optical probes may include: using or providing an excitation laser having a wavelength of 400 nm - 900 nm; coupling the excitation laser into the interference optical system, the one or more optical probes, and/or one or more components of the one or more optical probes; and exciting the interference optical system, the one or more optical probes, and/or one or more components of the one or more optical probes with the excitation laser for more than or equal to a set or predetermined amount of time. In one or more embodiments, the set or predetermined amount of time is thirty (30) minutes or is about thirty (30) minutes. In one or more embodiments, a user may set the amount of time for performing the photo-bleaching. In one or more embodiments, the one or more optical probes and/or the one or more components of the one or more optical probes may include or comprise of (or may each include or comprise of) a double clad fiber. In one or more embodiments, the set or predetermined amount of time for performing the excitation may be for 24 hours or more than 24 hours.

In one or more embodiments, one or more processors may perform or control the method for photo-bleaching. The one or more processors may receive the set or predetermined amount of time to perform the photo-bleaching or may automatically calculate and set the set or predetermined amount of time (*e.g*., based on a number of components or structure to be photo-bleached, based on a size and shape of the structure or the number of components to be photo-bleached, etc.).

In one or more embodiments using or involving a double clad fiber, the one or more processors may include or may be used with a core/clad ratio adjustment processor or unit that operates to control a ratio of excitation light between a core and a clad of the double clad fiber. In one or more embodiments, the one or more processors operate to set or use, or the method includes setting or using, the ratio of the clad (*e.g.,* the ratio of the amount of excitation light being sent to the clad) as being 10% or more than 10% (so that the ratio value for the amount of excitation light sent to the core is 90% or less than 90%). In one or more embodiments, the one or more processors operate to set or use, or the method includes setting or using, the ratio of the clad (*e.g.,* the ratio of the amount of excitation light being sent to the clad) as being 50% or being nearly or about 50% (so that the ratio value for the amount of excitation light sent to the core is 50% or a little over or about 50% (*e.g.,* the ratio value for the amount of excitation light may be a little over or about 50% by a difference that the ratio of the amount of excitation light being sent to the clad is nearly or about 50% (*e.g.,* in a case where the ratio value for the clad may be 47% (a 3% difference under 50%), then the ratio value for the core may be 53% (the 3% difference over 50%)).

In one or more embodiments, the photo-bleach method(s) may include using a detector and a lens or lens unit, the lens or lens unit operating to filter the excitation light and to pass an emission of the lens or lens unit through to and/or from the interference optical system, the one or more optical probes, and/or one or more components of the one or more optical probes.

In one or more embodiments, an optical power of the excitation laser into the interference optical system, the one or more optical probes, and/or one or more components of the one or more optical probes in total is one of the following: same as a nominal intensity as compared to a case where the excitation laser is used as part of a system, the interference optical system, the one or more optical probes, and/or one or more components of the one or more optical probes, which is at least 0.1 mW; two (2) times or more higher than the nominal intensity, which is at least 0.2 mW or at least 0.5 mW; ten (10) times or more higher than the nominal intensity, which is at least 1mW; and/or a hundred (100) times or more higher than the nominal intensity, which is at least 10mW.

In one or more embodiments, the method for photo-bleaching may be performed using artificial intelligence structure, such as, but not limited, convolutional neural networks, generative adversarial networks (GANs), neural networks, any other AI structure or feature(s) discussed herein, any other AI network structure(s) known to those skilled in the art, etc. For example, a generator of a generative adversarial network may operate to generate an image(s) that is/are so similar to ground truth image(s) that a discriminator of the generative adversarial network is not able to distinguish between the generated image(s) (*e.g*., of the photo-bleached interference optical system, the photo-bleached one or more optical probes, and/or one or more photo-bleached components of the one or more optical probes; of the estimated tissue type(s) or characteristic(s); etc.) and the ground truth image(s) (*e.g*., image(s) that may represent a set, predetermined, or target/desired amount of photo-bleaching for the interference optical system, the one or more optical probes, and/or one or more components of the one or more optical probes; image(s) that may represent the actual tissue type(s) or characteristic(s); etc.). The generative adversarial network may include one or more generators and one or more discriminators. Each generator of the generative adversarial network may operate to estimate the photo-bleach characteristic(s) of the interference optical system, the one or more optical probes, and/or one or more components of the one or more optical probes (*e.g*., using one or more images (*e.g*., a CT image, an OCT image, an IVUS image, a bronchoscopic image, etc.) of the interference optical system, the one or more optical probes, and/or one or more components of the one or more optical probes), and each discriminator of the generative adversarial network may operate to determine whether the estimated photo-bleach characteristic(s) of the interference optical system, the one or more optical probes, and/or one or more components of the one or more optical probes (*e.g.,* using one or more images (*e.g.,* a CT image, an OCT image, an IVUS image, a bronchoscopic image, etc.) of the interference optical system, the one or more optical probes, and/or one or more components of the one or more optical probes) is estimated (or fake) or ground truth (or real). In one or more embodiments, each generator of the generative adversarial network may operate to estimate the tissue type(s) or characteristic(s) (*e*.*g*., using one or more images (*e*.*g*., a CT image, an OCT image, an IVUS image, a bronchoscopic image, etc.), and each discriminator of the generative adversarial network may operate to determine whether each of the estimated tissue type(s) or characteristic(s) (*e*.*g*., using one or more images (*e.g.,* a CT image, an OCT image, an IVUS image, a bronchoscopic image, etc.) is estimated (or fake) or ground truth (or real). In one or more embodiments, an AI network, such as, but not limited to, a GAN or a consistent GAN (cGAN), may receive an image or images as an input and may obtain or create a photo-bleach map (*e*.*g*., annotated area(s) representing one or more photo-bleach characteristics and/or amounts/levels) for each image or images of the interference optical system, the one or more optical probes, and/or one or more components of the one or more optical probes. In one or more embodiments, an AI network may evaluate obtained one or more images (*e*.*g*., a CT image, an OCT image, an IVUS image, a bronchoscopic image, etc.), one or more virtual images, and one or more ground truth photo-bleach maps to generate photo-bleach map(s) for the one or more images and/or evaluate the generated photo-bleach map(s). A Three Cycle-Consistent Generative Adversarial Network (3cGAN) may be used to obtain or construct the photo-bleach map(s) and/or to evaluate the quality of the photo-bleach map(s).

In one or more embodiments, an artificial intelligence training apparatus using a neural network or other AI-ready network may include: a memory; one or more processors in communication with the memory, the one or more processors operating to: train one or more models for photo-bleaching an interference optical system, one or more optical probes, and/or one or more components of the one or more optical probes. In one or more embodiments of the present disclosure, an apparatus, a system, or a storage medium may use an AI network, a neural network, or other AI-ready network to perform any of the method step(s) or feature(s) discussed herein.

The one or more processors may further operate to use one or more neural networks, convolutional neural networks, recurrent neural networks, a generative adversarial network (GAN), a consistent generative adversarial network (cGAN), a three cycle-consistent generative adversarial network (3cGAN), and/or any other AI architecture discussed herein or known to those skilled in the art to one or more of: load a trained model (*e.g*., a model for tissue detection and/or characterization, a model for photo-bleaching, etc.), select a set of angiography frames or other type of image frames or select one or more intravascular images, identify one or more targets, objects, or samples (or one or more tissues in the targets, objects, or samples) and/or one or more photo-bleached interference optical systems, one or more photo-bleached optical probes, and/or one or more photo-bleached components of the one or more optical probes, evaluate the tissue(s) and/or tissue characteristic(s) and/or evaluate the photo-bleached characteristic(s), determine whether the tissue(s) or tissue characteristic(s) evaluation and/or whether the photo-bleached characteristic(s) evaluation is appropriate with respect to given prior knowledge (for example, vessel location and pullback direction, prior tissue location or characteristic information, target/desired/set/predetermined photo-bleach information or settings, etc.), modify the evaluation results or the location of the tissue(s) or characteristic(s) of the tissue(s) for each frame and/or modify the photo-bleach evaluation results or the location of the photo-bleach or photo-bleach characteristics for each frame, construct an image based on the tissue(s) or characteristic(s) of the tissue(s) and/or based on the photo-bleach or photo-bleach characteristic(s), perform coregistration of the constructed image with one or more intravascular images (*e.g*., OCT images, two-dimensional (2D) OCT images, etc.), insert or overlay intravascular image data (*e.g*., indicators or lines used to represent one or more tissue types, indicators or lines used to represent calcified (*e.g*., with a solid line) areas, indicators or lines used to represent lipid (*e.g.,* with a dashed or dotted line)) over the constructed image (*e.g.,* a line may represent a border in each of the intravascular images, a line or an outlined area may represent areas corresponding to different tissue(s) (*e.g*., calcified areas, lipid areas, etc.), indicators or lines used to represent photo-bleached areas of the interference optical system, one or more optical probes, and/or one or more components of the one or more optical probes, etc.), and/or acquire or receive the image data during the pullback operation.

Turning now to the details of the figures, imaging modalities may be displayed in one or more ways as discussed herein. One or more displays discussed herein may allow a user of the one or more displays to use, control and/or emphasize multiple imaging techniques or modalities, such as, but not limited to, OCT, CT, IVUS, NIRF, NIRAF, fluorescence, NIRS, etc., and may allow the user to use, control, and/or emphasize the multiple imaging techniques or modalities synchronously.

As shown diagrammatically in FIG. 1A, one or more embodiments for visualizing, emphasizing and/or controlling one or more imaging modalities and for performing photo-bleaching, evaluating and detecting or identifying one or more tissue types and/or tissue characteristics, and/or performing coregistration of the present disclosure may be involved with one or more predetermined or desired procedures, such as, but not limited to, performing photo-bleaching, medical procedure planning and performance (*e*.*g*., Percutaneous Coronary Intervention (PCI)), etc. For example, the system 2 may communicate with the image scanner 5 (*e.g.,* a CT scanner, an X-ray machine, etc.) to request information for use in the medical procedure (*e*.*g*., PCI) planning and/or performance, such as, but not limited to, bed positions, and the image scanner 5 may send the requested information along with the images to the system 2 once a clinician uses the image scanner 5 to obtain the information via scans of the patient. In some embodiments, one or more angiograms 3 taken concurrently or from an earlier session are provided for further planning and visualization. The system 2 may further communicate with a workstation such as a Picture Archiving and Communication System (PACS) 4 to send and receive images of a patient to facilitate and aid in the medical procedure planning and/or performance. Once the plan is formed, a clinician may use the system 2 along with a medical procedure/imaging device 1 (*e.g.,* an imaging device, an OCT device, an IVUS device, a PCI device, an ablation device, a 3D structure construction or reconstruction device, etc.) to consult a medical procedure chart or plan to understand the shape and/or size of the targeted biological object to undergo the imaging and/or medical procedure. Each of the medical procedure/imaging device 1, the system 2, the locator device 3, the PACS 4 and the scanning device 5 may communicate in any way known to those skilled in the art, including, but not limited to, directly (via a communication network) or indirectly (via one or more of the other devices such as 1 or 5, or additional flush and/or contrast delivery devices; via one or more of the PACS 4 and the system 2; via clinician interaction; etc.).

In medical procedures, improvement or optimization of physiological assessment is preferable to decide a course of treatment for a particular patient. By way of at least one example, physiological assessment is very useful for deciding treatment for cardiovascular disease patients. In a catheterization lab, for example, physiological assessment may be used as a decision-making tool - *e*.*g*., whether a patient should undergo a PCI procedure, whether a PCI procedure is successful, etc. While the concept of using physiological assessment is theoretically sound, physiological assessment still waits for more adaption and improvement for use in the clinical setting(s). This situation may be because physiological assessment may involve adding another device and medication to be prepared, and/or because a measurement result may vary between physicians due to technical difficulties. Such approaches add complexities and lack consistency. Therefore, one or more embodiments of the present disclosure may employ computational fluid dynamics based (CFD-based) physiological assessment that may be performed from imaging data to eliminate or minimize technical difficulties, complexities and inconsistencies during the measurement procedure. To obtain accurate physiological assessment, an accurate 3D structure of the vessel may be reconstructed from the imaging data as disclosed in U.S. Pat. Pub. No. 2021/0077037 A1, published on March 18, 2021, the disclosure of which is incorporated by reference herein in its entirety. Additionally or alternatively, the determination or identification of one or more tissue types and/or tissue characteristics operates to provide additional information for physiological assessment.

In at least one embodiment of the present disclosure, a method may be used to provide more accurate 3D structure(s) compared to using only one imaging modality. In one or more embodiments, a combination of multiple imaging modalities may be used, photo-bleaching may be performed, one or more characteristics of photo-bleaching may be detected, one or more tissue types and/or tissue characteristics may be detected, and coregistration may be processed/performed using artificial intelligence.

One or more embodiments of the present disclosure may apply machine learning, especially deep learning, to perform photo-bleaching, detect photo-bleach characteristic(s), detect one or more tissue types and/or tissue characteristics, etc. in an image frame without user input(s) that define an area where intravascular imaging pullback occurs. Using artificial intelligence, for example, deep learning, one or more embodiments of the present disclosure may achieve a better or maximum success rate of photo-bleaching, photo-bleach characteristic(s) detection, tissue type(s) and/or tissue characteristic(s) detection from image data without (or with less) user interactions, and may reduce processing and/or prediction time to display coregistration result(s) based on the improved image quality obtained, including, but not limited to, when using photo-bleaching, when detecting photo-bleach characteristic(s), and/or when detecting tissue type(s) and/or tissue characteristic(s).

One or more embodiments of the present disclosure may achieve the efficient catheter (or other imaging device) photo-bleaching, detection of photo-bleach characteristics(s), detection of tissue type(s) and/or tissue characteristic(s), and/or efficient coregistration result(s) from image(s). In one or more embodiments, the image data may be acquired during intravascular imaging pullback using a catheter (or other imaging device) that may be visualized in an image. In one or more embodiments, a ground truth identifies a location or locations of the catheter or a portion of the catheter (or of another imaging device or a portion of the another imaging device). For example, while not limited hereto, the ground truth may identify a photo-bleached portion of the catheter. In one or more embodiments, a model has enough resolution to predict the photo-bleach and/or tissue type(s) and/or tissue characteristic(s) (*e.g*., location, size, etc.) in a given image with sufficient accuracy depending on the application or procedure being performed. The performance of the model may be further improved by adding more training data. For example, additional training data may include image annotations, where a user labels or corrects the tissue type(s), tissue characterization(s), and/or catheter detection(s) in each image.

In one or more embodiments, one or more photo-bleach characteristic(s) and/or tissue type(s) or characteristic(s) may be detected and/or monitored using an algorithm, such as, but not limited to, the Viterbi algorithm.

One or more embodiments may automate characterization of tissue(s) and/or identification of tissue type(s) in images using convolutional neural networks (or other AI structure discussed herein or known to those skilled in the art), and may fully automate frame detection on angiographies, intravascular pullbacks, etc. using training (*e.g*., offline training) and using applications (*e.g*., online application(s)) to extract and process frames via deep learning.

One or more embodiments of the present disclosure may track and/or calculate photo-bleaching, photo-bleach characteristic(s), and/or tissue type(s) and/or tissue characteristic(s) detection or identification success rate(s).

In at least one further embodiment example, a method of 3D reconstruction without adding any imaging requirements or conditions may be employed. One or more methods of the present disclosure may use intravascular imaging, *e.g.,* IVUS, OCT, etc., and one (1) view of angiography. One or more embodiments may use one image only (*e*.*g*., carpet view, a frame of carpet views, another frame or image type discussed herein or known to those skilled in the art, etc.) In the description below, while intravascular imaging of the present disclosure is not limited to OCT, OCT is used as a representative of intravascular imaging for describing one or more features herein.

Referring now to FIG. 1B, shown is a schematic diagram of at least one embodiment of an imaging system 20 for detecting photo-bleaching or photo-bleaching characteristic(s) in a catheter and/or generating an imaging catheter path based on a detected location of an imaging catheter, based on tissue type(s) and/or tissue characteristic(s) detection or identification, and/or a regression line representing the imaging catheter path by using an image frame that is simultaneously acquired during intravascular imaging pullback. The embodiment of FIG. 1B may be used with one or more of the artificial intelligence feature(s) discussed herein. The imaging system 20 may include an angiography system 30, an intravascular imaging system 40, an image processor 50, a display or monitor 1209, and an electrocardiography (ECG) device 60. The angiography system 30 may include an X-ray imaging device such as a C-arm 22 that is connected to an angiography system controller 24 and an angiography image processor 26 for acquiring angiography image frames of an object (*e.g*., any object that may be imaged using the size and shape of the imaging device, a sample, a vessel, a target specimen or object, etc.) or patient 106.

The intravascular imaging system 40 of the imaging system 20 may include a console 32, a catheter 120, and a patient interface unit or PIU 110 that connects between the catheter 120 and the console 32 for acquiring intravascular image frames. The catheter 120 may be inserted into a blood vessel of the patient 106 (or inside a specimen or other target object, inside tissue, etc.). The catheter 120 may function as a light irradiator and a data collection probe that is disposed in a lumen of a particular blood vessel, such as, for example, a coronary artery, or in another type of tissue or specimen. The catheter 120 may include a probe tip, one or more markers or radiopaque markers, an optical fiber, and a torque wire. The probe tip may include one or more data collection systems. The catheter 120 may be threaded in an artery of the patient 106 to obtain images of the coronary artery. The patient interface unit 110 may include a motor M inside to enable pullback of imaging optics during the acquisition of intravascular image frames. The imaging pullback procedure may obtain images of the blood vessel. The imaging pullback path may represent the co-registration path, which may be a region of interest or a targeted region of the vessel.

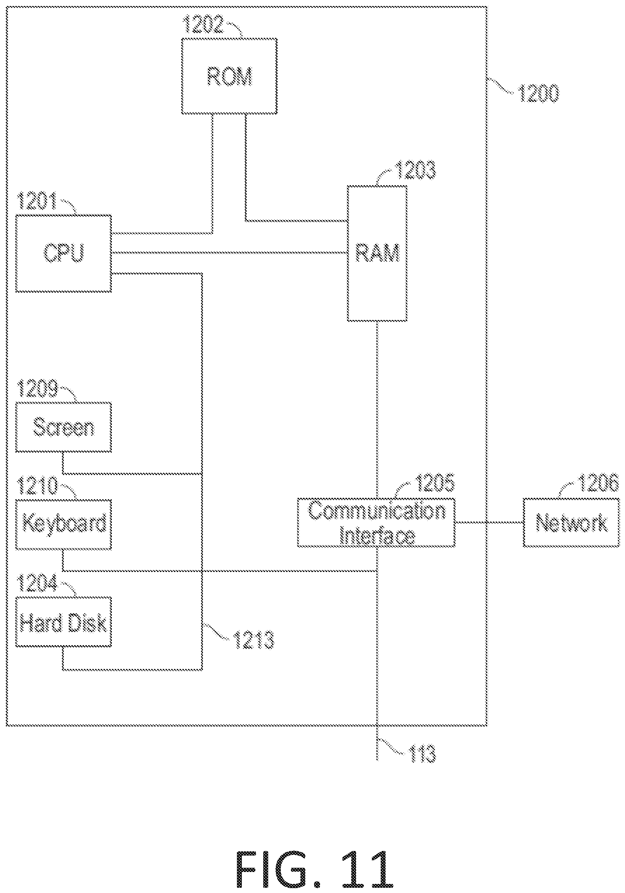

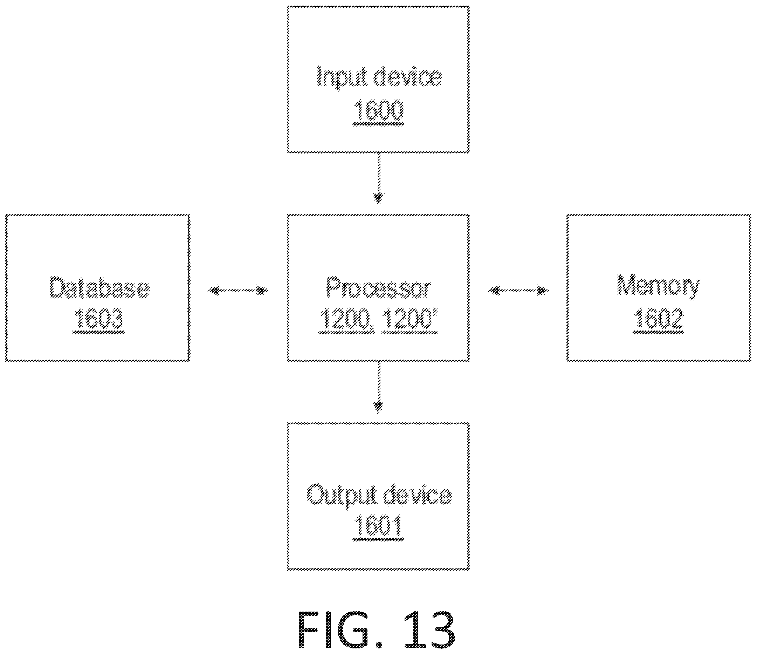

The console 32 may include a light source(s) 101 and a computer 1200. The computer 1200 may include features as discussed herein and below (*see e.g.,* FIG. 11, FIG. 13, etc.), or alternatively may be a computer 1200' (*see e.g.,* FIG. 11, FIG. 13, etc.) or any other computer or processor discussed herein. In one or more embodiments, the computer 1200 may include an intravascular system controller 35 and an intravascular image processor 36. The intravascular system controller 35 and/or the intravascular image processor 36 may operate to control the motor M in the patient interface unit 110. The intravascular image processor 36 may also perform various steps for image processing and control the information to be displayed.

Various types of intravascular imaging systems may be used within the imaging system 20. The intravascular imaging system 40 is merely one example of an intravascular imaging system that may be used within the imaging system 20. Various types of intravascular imaging systems may be used, including, but not limited to, an OCT system, a multi-modality OCT system or an IVUS system, by way of example.

The imaging system 20 may also connect to an electrocardiography (ECG) device (or other monitoring device) 60 for recording the electrical activity of the heart (or other organ being monitored, tissue being monitored, specimen being monitored, etc.) over a period of time using electrodes placed on the skin of the patient 106. The imaging system 20 may also include an image processor 50 for receiving angiography data, intravascular imaging data, and data from the ECG device 60 to execute various image-processing steps to transmit to a display 1209 for displaying an angiography image frame with a co-registration path. Although the image processor 50 associated with the imaging system 20 appears external to both the angiography system 20 and the intravascular imaging system 30 in FIG. 1B, the image processor 50 may be included within the angiography system 30, the intravascular imaging system 40, the display 1209, or a stand-alone device. Alternatively, the image processor 50 may not be required if the various image processing steps are executed using one or more of the angiography image processor 26, the intravascular image processor 36 of the imaging system 20, or any other processor discussed herein (*e.g*., computer 1200, computer 1200', computer or processor 2, etc.).

To collect data that may be used to train one or more neural nets, one or more features of an OCT device or system (*e.g*., an MM-OCT device or system, a SS-OCT device or system, etc.) may be used. Collecting a series of OCT images with or without tissue(s) being shown, with one or more tissue types, with one or more tissue characteristics, etc. may result in a plurality (*e.g*., several thousand) of training images. In one or more embodiments, the data may be labeled based on whether photo-bleaching characteristic(s) were identified or detected and/or whether a tissue type was identified or detected, a tissue characteristic was identified or detected, a tissue location was identified or detected, a plurality of tissue types are detected, a plurality of tissue characteristics are detected, etc. (as confirmed by a trained operator or user of the device or system). In one or more embodiments, after at least 30,000 OCT images are captured and labeled, the data may be split into a training population and a test population. In one or more embodiments, data collection may be performed in the same environment or in different environments. For example, during data collection, a flashlight (or any light source) may be used to shine the light down a barrel of an imaging device with no catheter imaging core to confirm that a false positive would not occur in a case where a physician pointed the imaging device at external lights (*e.g*., operating room lights, a computer screen, etc.). After training is complete, the testing data may be fed through the neural net or neural networks, and the accuracy of the model(s) may be evaluated based on the result(s) of the test data.

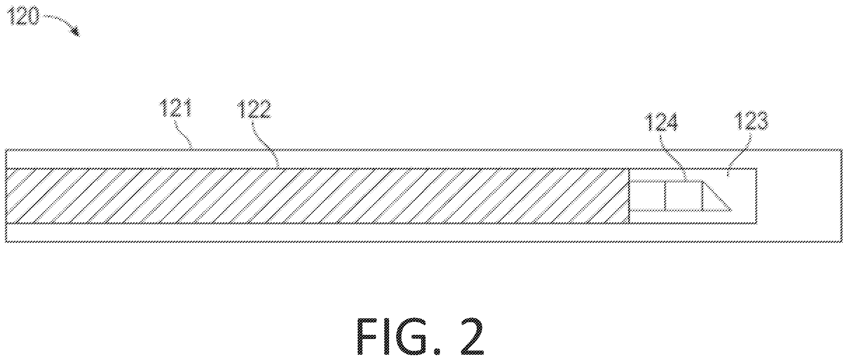

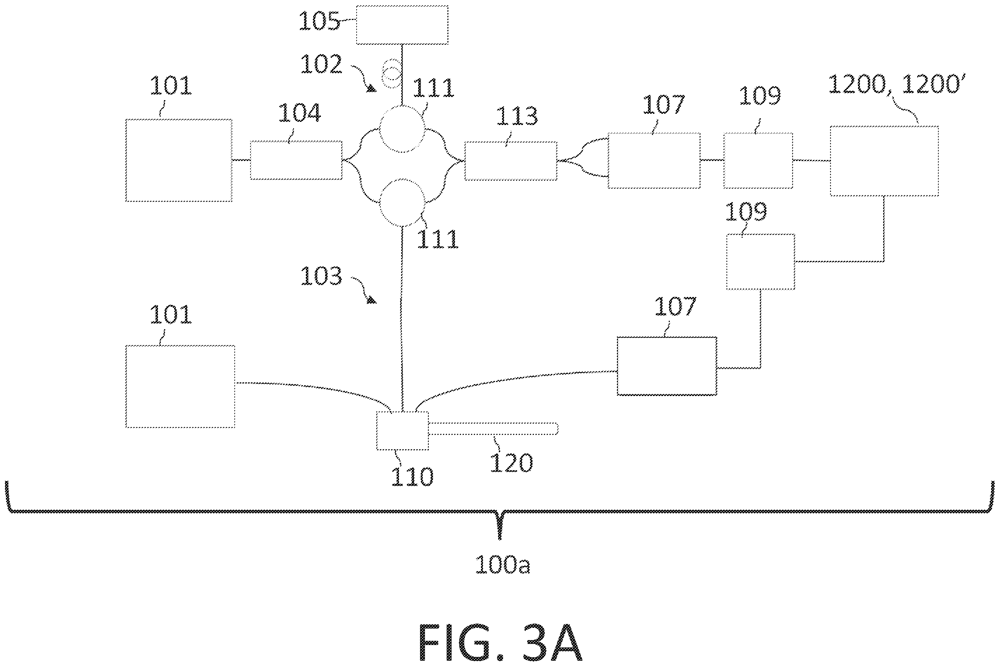

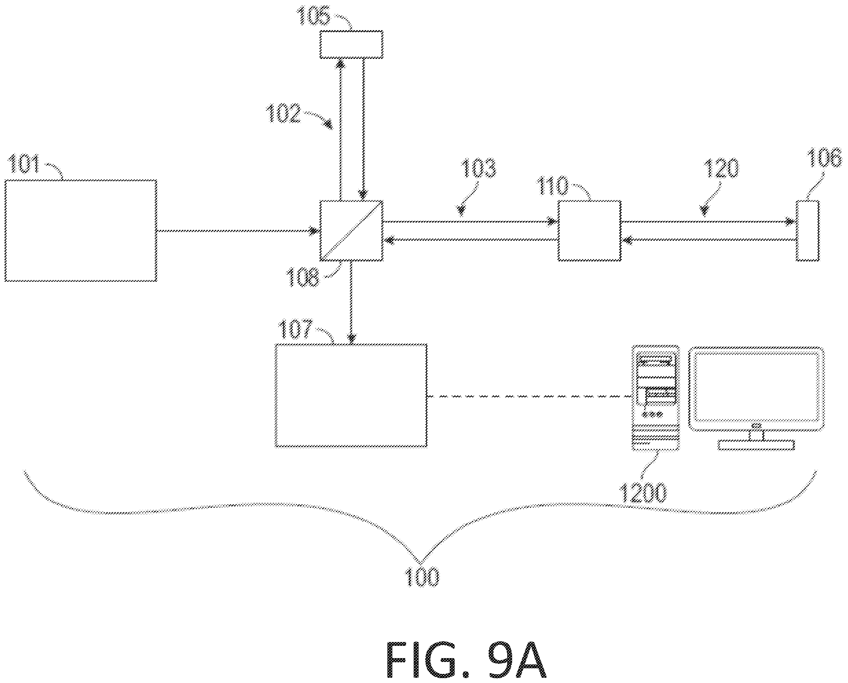

FIG. 2 shows at least one embodiment of a catheter 120 that may be used in one or more embodiments of the present disclosure for obtaining images; for using and/or controlling multiple imaging modalities, to perform photo-bleaching, to identify or detect photo-bleach characteristic(s), and/or to identify one or more tissue type(s) and/or one or more tissue characteristic(s) in an image or frame with greater or maximum success; and for using the results to perform coregistration more efficiently or with maximum efficiency. FIG. 2 shows an embodiment of the catheter 120 including a sheath 121, a coil 122, a protector 123, and an optical probe 124. As shown schematically in FIGS. 3A-3B and 9A-9C (discussed further below), the catheter 120 may be connected to a patient interface unit (PIU) 110 to spin the coil 122 with pullback (*e.g*., at least one embodiment of the PIU 110 operates to spin the coil 122 with pullback). The coil 122 delivers torque from a proximal end to a distal end thereof (*e.g*., via or by a rotational motor in the PIU 110). In one or more embodiments, the coil 122 is fixed with/to the optical probe 124 so that a distal tip of the optical probe 124 also spins to see an omnidirectional view of the object (*e.g*., a biological organ, sample or material being evaluated, such as, but not limited to, hollow organs such as vessels, a heart, a coronary artery, etc.). For example, fiber optic catheters and endoscopes may reside in the sample arm (such as the sample arm 103 as shown in one or more of FIGS. 9A-9C discussed below) of an OCT interferometer in order to provide access to internal organs, such as intravascular images, gastro-intestinal tract or any other narrow area, that are difficult to access. As the beam of light through the optical probe 124 inside of the catheter 120 or endoscope is rotated across the surface of interest, cross-sectional images of one or more objects are obtained. In order to acquire imaging data or three-dimensional data, the optical probe 124 is simultaneously translated longitudinally during the rotational spin resulting in a helical scanning pattern. This translation is most commonly performed by pulling the tip of the probe 124 back towards the proximal end and therefore referred to as a pullback.

The catheter 120, which, in one or more embodiments, comprises the sheath 121, the coil 122, the protector 123 and the optical probe 124 as aforementioned (and as shown in FIG. 2), may be connected to the PIU 110. In one or more embodiments, the optical probe 124, which may be a photo-bleached optical probe 124 using one or more of the photo-bleach features of the present disclosure, may comprise an optical fiber connector, an optical fiber and a distal lens. The optical fiber connector may be used to engage with the PIU 110. The optical fiber may operate to deliver light to the distal lens. The distal lens may operate to shape the optical beam and to illuminate light to the object (*e.g.,* the object 106 (*e.g.,* a vessel) discussed herein), and to collect light from the sample (*e.g.,* the object 106 (*e.g.,* a vessel) discussed herein) efficiently. While the target, sample, or object 106 may be a vessel in one or more embodiments, the target, sample, or object 106 may be different from a vessel (and not limited thereto) depending on the particular use(s) or application(s) being employed with the catheter 120. A photo-bleached optical probe may be fabricated/processed by performing one or more photo-bleaching processes to an optical probe. The optical probe may emit background emission noise (or catheter background noise) in a case where excitation light couples into an optical fiber of the catheter (*e.g*., such as the catheter 120). An intensity of the emission noise (or background noise) varies depending on how long an excitation light may go through an optical fiber of the catheter (*e.g*., the catheter 120).

As aforementioned, in one or more embodiments, the coil 122 delivers torque from a proximal end to a distal end thereof (*e.g*., via or by a rotational motor in the PIU 110). There may be a mirror at the distal end so that the light beam is deflected outward. In one or more embodiments, the coil 122 is fixed with/to the optical probe 124 so that a distal tip of the optical probe 124 also spins to see an omnidirectional view of an object (*e.g.*, a biological organ, sample or material being evaluated, such as, but not limited to, hollow organs such as vessels, a heart, a coronary artery, etc.). In one or more embodiments, the optical probe 124 may include a fiber connector at a proximal end, a double clad fiber and a lens at distal end. The fiber connector operates to be connected with the PIU 110. The double clad fiber may operate to transmit & collect OCT light through the core and, in one or more embodiments, to collect Raman and/or fluorescence from an object (*e.g.,* the object 106 (*e.g.,* a vessel) discussed herein, an object and/or a patient (*e.g*., a vessel in the patient), etc.) through the clad. The lens may be used for focusing and collecting light to and/or from the object (*e.g.,* the object 106 (*e.g.,* a vessel) discussed herein). In one or more embodiments, the scattered light through the clad is relatively higher than that through the core because the size of the core is much smaller than the size of the clad.

FIG. 3A shows at least one embodiment of a system 100a which includes OCT and fluorescence sub-systems. In one or more embodiments, the OCT sub-system includes a light source, such as the light source 101, a splitter (such as the splitter 104; another type of deflecting or deflection device discussed below may be used in place of the splitter 104), one or more circulators 111, a reference reflection (such as the reference reflection 105), a combiner (such as the combiner 113), and at least one detector (such as the at least one detector 107). The OCT sub-system may be connected to, and include, a patient interface unit, such as the PIU 110, and the catheter 120 to expose a sample, such as the sample 106, to light and receive information in response thereto. In one or more embodiments, the fluorescence sub-system may include a light source for fluorescence (such as the second light source 101 shown in FIG. 3A) and at least one detector (such as the second at least one detector 107 shown in FIG. 3A). The fluorescence sub-system, including, but not limited to, the second light source 101 and the second at least one detector 107, may also be connected to (*see* FIG. 3A), and/or include, a patient interface unit, such as the PIU 110, and the catheter 120 to expose a sample, such as the sample 106, to fluorescent light and receive information in response thereto. For example, in at least one embodiment, an OCT light with a wavelength of around 1.3um from a light source (such as the light source 101 of the OCT sub-system) may be delivered and split into a reference arm (*e.g*., the reference arm 102) and a sample arm (*e.g.,* the sample arm 103) with a splitter (*e.g.,* the splitter 104). A reference beam is reflected from a reference mirror (*e*.*g*., the reference reflection 105) in the reference arm (*e*.*g*., the reference arm 102) while a sample beam is reflected or scattered from a sample through a PIU (patient interface unit) (such as the PIU 110) and a catheter (*e*.*g*., the catheter 120) in the sample arm (*e*.*g*., the sample arm 103). Both beams combine at a combiner (*e.g.,* the splitter 104 in FIG. 3A, the combiner 113 in FIG. 3A, etc.) and generate interference patterns. The output of the interferometer is detected with detectors (*e*.*g*., the at least one detector 107 shown in FIG. 3A, the at least one detector 107 of the OCT sub-system shown in FIG. 3A, etc.) such as photodiodes, multi-array cameras, PMTs, etc. Then signals are transferred to a computer (*e*.*g*., the computer(s) 2, 1200 as shown in FIGS. 1A-3B, 9A-9C, 11, and 13, the computer 1200' of FIGS. 3A and 12-13, etc.) to perform signal processing. The interference patterns are generated only when the path length of the sample arm (*e*.*g*., the sample arm 103) matches that of the reference arm (*e*.*g*., the reference arm 102) to within the coherence length of the light source (*e*.*g*., the light source 101 of FIG. 1B, the light source 101 of the OCT sub-system of FIG. 3A, etc.). In one or more embodiments, the one or more detectors 107 of the OCT sub-system may send the signal(s) to a first data acquisition unit or processor 109, and the first data acquisition unit or processor 109 may send the signal(s) to the computer (*e*.*g*., the computer 1200, the computer 1200', any other computer discussed herein, etc.). In one or more embodiments, the one or more detectors 107 of the fluorescence sub-system may send the signal(s) to a second data acquisition unit or processor 109, and the second data acquisition unit or processor 109 may send the signal(s) to the computer (*e*.*g*., the computer 1200, the computer 1200', any other computer discussed herein, etc.).

In one or more embodiments, although not limited to double clad fiber(s), fiber(s) of the PIU (*e.g.,* the PIU 110) and the catheter (*e.g.,* the probe of the catheter 120) may be made of a double clad fiber (DCF). OCT light may illuminate a sample (*e*.*g*., the sample 106) through the DCF (*e.g.,* through the core of the DCF), and scattered light from the sample (*e.g.,* the sample 106) may be collected and delivered back to the circulator (*e*.*g*., the circulator 111, the lower or second circulator 111 in FIG. 3A, a circulator of an OCT interferometer, the circulator 111 of the OCT sub-system, etc.) via the PIU (*e.g.,* the PIU 110) and may be combined with the reference beam at a combiner (*e*.*g*., the combiner 113) to generate one or more interference patterns. The output of an OCT interferometer and/or the combiner 113 may be detected with the one or more OCT detectors (*e*.*g*., the one or more detectors 107), such as, but not limited to, photodiodes, multi-array cameras, PMTs, etc. Signals may be transferred to a computer (*e*.*g*., the computer 1200, the computer 1200', any other computer discussed herein, etc.) to perform signal processing to generate OCT image(s). The interference patterns may be generated when the path length of the sample arm (*e.g.,* the sample arm 103) matches that of the reference arm (*e.g.,* the reference arm 102) to within the coherence length of the light source (*e*.*g*., the light source 101, the light source 101 of the OCT sub-system, etc.).

An excitation light with a wavelength (*e*.*g*., any predetermined wavelength visible to infrared (IR)), for example, 0.633 um from a light source (*e*.*g*., the light source 101 of the fluorescence sub-system of FIG. 3A) or 0.635 um from the light source (*e*.*g*., the light source 101 of the fluorescence sub-system of FIG. 3A) may be delivered to the sample (*e*.*g*., the sample 106) through the PIU (*e.g.,* the PIU 110) and the catheter (*e.g.,* the catheter 120). One or more embodiments of the PIU may comprise or include a free space beam combiner so that the excitation light couples into a common DCF or other possible/useful fiber with OCT, and a laser or light may be used for the excitation laser or light while an excitation laser or light may be illuminated to a sample (*e*.*g*., the sample 106) from a distal end of the optical probe in a catheter (*e.g.,* the catheter 120). The sample (*e.g.,* the sample 106) may emit auto-fluorescence light with broadband wavelengths of, for example, 0.633 um - 0.80 um by the excitation light. By way of another example, the sample (*e*.*g*., the sample 106) may emit auto-fluorescence light with broadband wavelengths of 0.65 um - 0.90 um. In one or more embodiments, the catheter (*e*.*g*., the catheter 120) may emit a Raman scattering around 0.65 - 0.75 um. The auto-fluorescence light and the Raman light/signal may be collected with the catheter (*e*.*g*., the catheter 120 of FIG. 3A) and delivered to detectors (*e.g*., the detector(s) 107 of the fluorescence sub-system of FIG. 3A, a fluorescence detector(s) such as a photo-multiplier tube (PMT) or tubes (PMTs), etc.) via the PIU *(e.g.,* the PIU 110). An electrical signal *(e.g.,* an analog electric signal) at the fluorescence detector 107 may be acquired by the second data acquisition unit or processor 109, and the electrical signal may be acquired in a case where a trigger is detected in one or more embodiments. Other wavelengths, in the visible and NIR are also contemplated. In one or more embodiments, the patient interface unit (PIU; *e.g.,* the PIU 110 as further discussed below) may include or comprise a free space beam combiner so that the excitation light couples into a common DCF or other possible/useful fiber with OCT. The excitation light may be illuminated to the sample (*e.g.,* the sample 106) from a distal end of the optical probe in the catheter (*e.g.,* the catheter or the probe 120).

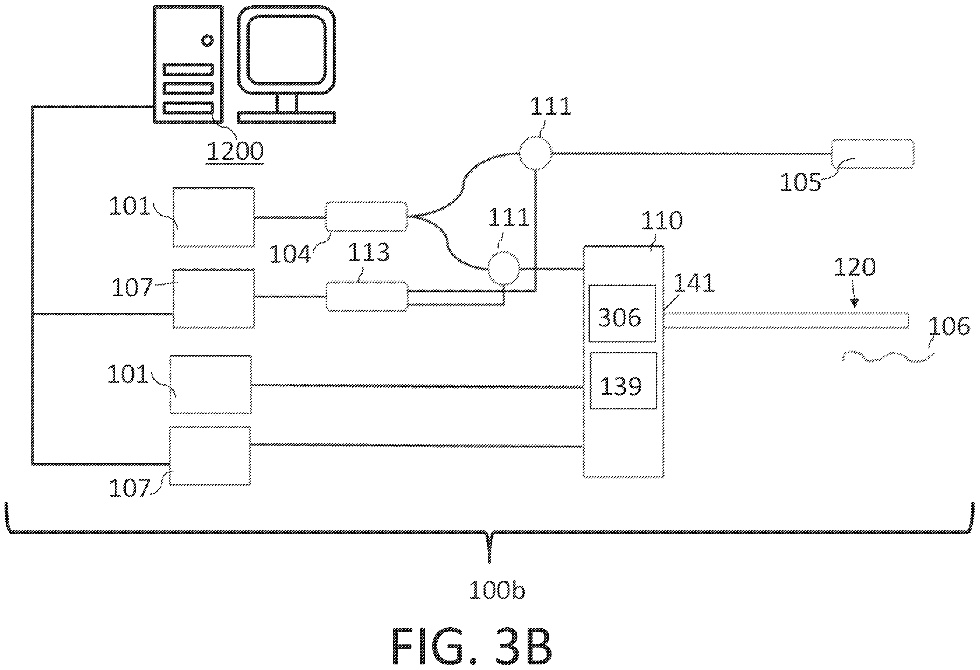

FIG. 3B shows at least one embodiment of a system 100b which includes OCT and fluorescence sub-systems. In one or more embodiments, the OCT sub-system includes a light source, such as the light source 101, a splitter (such as the splitter 104; another type of deflecting or deflection device discussed below may be used in place of the splitter 104), one or more circulators 111, a reference reflection (such as the reference reflection 105), a combiner (such as the combiner 113), and at least one detector (such as the at least one detector 107). The OCT sub-system may be connected to, and include, a patient interface unit, such as the PIU 110, and the catheter 120 to expose a sample, such as the sample 106, to light and receive information in response thereto. In one or more embodiments, the fluorescence sub-system may include a light source for fluorescence (such as the second light source 101 shown in FIG. 3B) and at least one detector (such as the second at least one detector 107 shown in FIG. 3B). The fluorescence sub-system, including, but not limited to, the second light source 101 and the second at least one detector 107, may also be connected to (*see* FIG. 3B), and/or include, a patient interface unit, such as the PIU 110, and the catheter 120 to expose a sample, such as the sample 106, to fluorescent light and receive information in response thereto. For example, in at least one embodiment, an OCT light with a wavelength of around 1.3um from a light source (such as the light source 101 of the OCT sub-system) is delivered and split into a reference arm (*e.g*., the reference arm 102) and a sample arm (*e.g.,* the sample arm 103) with a splitter (*e.g.,* the splitter 104). A reference beam is reflected from a reference mirror (*e*.*g*., the reference reflection 105) in the reference arm (*e*.*g*., the reference arm 102) while a sample beam is reflected or scattered from a sample through a PIU (patient interface unit) (such as the PIU 110) and a catheter (*e*.*g*., the catheter 120) in the sample arm (*e*.*g*., the sample arm 103). Both beams combine at a combiner (*e.g.,* the splitter 104 in FIG. 3B, the combiner 113 in FIG. 3B, etc.) and generate interference patterns. The output of the interferometer is detected with detectors (*e*.*g*., the at least one detector 107 shown in FIG. 3B, the at least one detector 107 of the OCT sub-system shown in FIG. 3B, etc.) such as photodiodes or multi-array cameras. Then signals are transferred to a computer (*e.g.,* the computer(s) 2, 1200 as shown in FIGS. 1A-3B, 9A-9C, 11 and 13, the computer 1200' of FIGS. 3A and 12-13, etc.) to perform signal processing. The interference patterns are generated only when the path length of the sample arm (*e*.*g*., the sample arm 103) matches that of the reference arm (*e*.*g*., the reference arm 102) to within the coherence length of the light source (*e.g.,* the light source 101 of FIG. 1B, the light source 101 of the OCT sub-system of FIG. 3B, etc.).

An excitation light with a wavelength (*e*.*g*., any predetermined wavelength visible to infrared (IR)), for example, 0.633 um from a light source (*e*.*g*., the light source 101 of the fluorescence sub-system of FIG. 3B) is delivered to the sample (*e*.*g*., the sample 106) through the PIU (*e.g.,* the PIU 110) and the catheter (*e.g.,* the catheter 120). The sample (*e.g.,* the sample 106) emits auto-fluorescence light with broadband wavelengths of, for example, 0.633 um - 0.80 um by the excitation light. The auto-fluorescence light is collected with the catheter (*e*.*g*., the catheter 120 of FIG. 3B) and delivered to detectors (*e*.*g*., the detector(s) 107 of the fluorescence sub-system of FIG. 3B) via the PIU (*e.g.,* the PIU 110). By way of another example, the sample (*e.g.,* the sample 106) may emit auto-fluorescence light with broadband wavelengths of 0.65 um - 0.90 um. In one or more embodiments, the catheter (*e*.*g*., the catheter 120) may emit a Raman scattering around 0.65 - 0.75 um. The auto-fluorescence light may be collected with the catheter (*e.g.,* the catheter 120 of FIG. 3B) and delivered to detectors (*e.g.,* the detector(s) 107 of the fluorescence sub-system of FIG. 3B, a fluorescence detector(s) such as a photo-multiplier tube (PMT) or tubes (PMTs), etc.) via the PIU (*e.g.,* the PIU 110). Other wavelengths, in the visible and NIR are also contemplated. The embodiment of FIG. 3B may include one or more of the aforementioned features of FIG. 3A, and such features are not further discussed herein as a result.

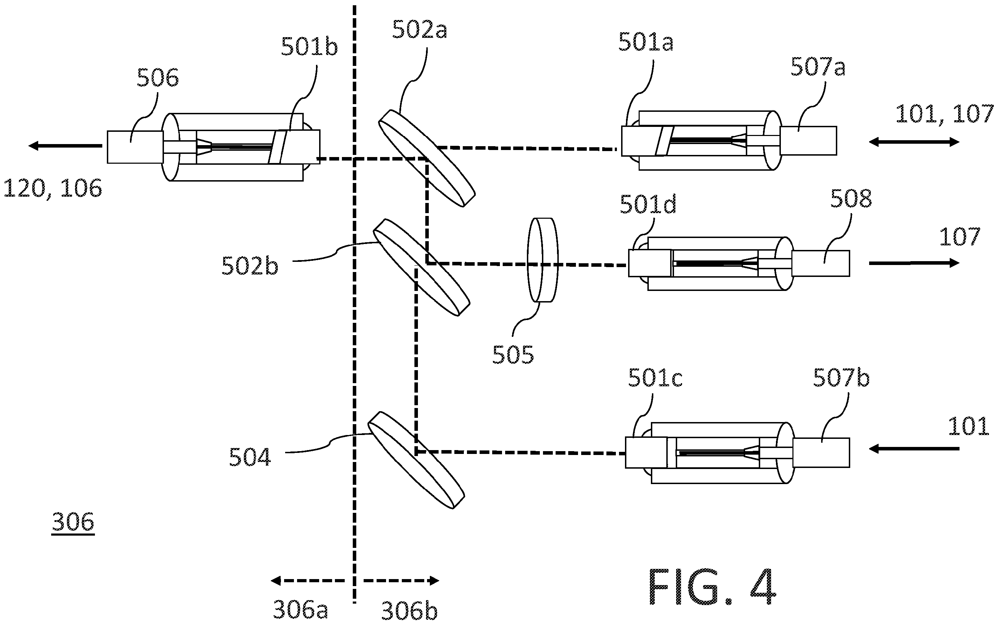

FIG. 4 shows a configuration of a free space beam combiner and FORJ in accordance with at least one embodiment of the present disclosure. In one or more embodiments, a FORJ (*e.g.,* the FORJ 306) may include a rotor 306a and a stator 306b. In an OCT and fluorescence system (such as the system 100a of FIG. 3A and/or the system 100b as shown in FIG. 3B), the stator (*e*.*g*., the stator 306b of FIG. 4) comprises at least two (2) optical fibers for OCT and excitation (*see e.g.,* single mode fiber 507a of FIG. 4 that operates for OCT light source delivery and light detection; single mode fiber 507b of FIG. 4 that operates to work with the excitation light source 107 (*e.g.,* light source 101 of the fluorescence sub-system or portion of system 100a of FIG. 3A and/or of system 100b of FIG. 3B); etc.). Each fiber has a lens at the beam combiner side of each fiber (*e*.*g*., the single mode fiber 507a is connected to a GRIN lens 501a as shown in FIG. 4; the multi-mode fiber 508 is connected to a GRIN lens 501d as shown in FIG. 4; the single mode fiber 507b is connected to a GRIN lens 501c as shown in FIG. 4; etc.). In one or more embodiments, the PIU (*e.g.,* the PIU 110) may comprise, include, or be connected to a free space beam combiner, a FORJ, a rotational motor and a translation motorized stage, and a catheter connector. One or more embodiments of a FORJ (such as, but not limited to, the FORJ 306) of the present disclosure may allow uninterrupted transmission of an optical signal while rotating the fiber (*e.g.,* a DCF or other fiber discussed herein or known to those skilled in the art) on the left side (e.g., on a side having the rotor 306a) along a fiber axis in FIG. 4. The FORJ 306 may have a free space optical beam coupler to separate the rotor 306a and the stator 306b. The rotor (*e.g.,* the rotor 306a of FIG. 4) may be made of a double clad fiber (*e.g.,* the double clad fiber 506) with a fiber connection at the catheter (*e.g*., the catheter 120) side and a lens (*e.g.,* a GRIN lens 501b as shown in FIG. 4) at the beam combiner side. In one or more embodiments, the rotor or rotator may include a DCF or other fiber with a lens configuration to make a collimated beam. Then, the fiber connector of the rotor (*e.g*., the rotor 306a) is connected to the optical probe (*e.g*., the optical probe 124 via the catheter 120 as shown in FIGS. 2 and 4), and the stator (*e.g*., the stator 306b) is connected to the optical sub-systems (as shown schematically in FIG. 4). For example, in at least one embodiment as best seen schematically in FIG. 4, the single mode fiber 507a is connected to the OCT light source (*e.g*., the light source 101) and the detection elements (*e.g*., the at least one detector 107) of the OCT sub-system, the multi-mode fiber 508 is connected to the fluorescence detection elements (*e.g*., the at least one detector 107) of the fluorescence sub-system), and the single mode fiber 507b is connected to the excitation light source (*e.g*., the light source 101) of the fluorescence sub-system. The rotational motor (*e.g.,* the rotational motor 139 in FIG. 3B) delivers the torque to the rotor (*e.g.,* the rotor 306a). Also, the translation motorized stage is used for a pullback such that the beam is scanned inside the lumen sample in a helical manner. The catheter connector (*e.g*., the catheter connector 141 as shown schematically in FIG. 3B) is connected to the catheter (*e.g*., the catheter 120). In one or more embodiments, the rotator or rotor may include or may comprise a double clad fiber with a lens to make a collimated beam. The rotor may be connected to the optical probe, and the stator may be connected to the optical sub-systems. The rotational motor delivers the torque to the rotor. Also, the translation motorized stage may be used for a pullback as aforementioned.

In one or more embodiments, the free space beam combiner may have dichroic filters (*e.g.,* the dichroic filter(s) 502a discussed herein) to separate different wavelength lights (OCT, excitation light, and Raman and auto-fluorescence lights in one or more embodiments). The beam combiner also may include or may comprise low-pass filters or band-pass filters in front of the Raman and auto-fluorescence channel to eliminate excitation light because of minimized excitation light noises at the fluorescence detector (*e.g*., the one or more detectors 107 of the fluorescence sub-system). The cut-off wavelength of the filter (low-pass or band-pass) may be selected around from 645 to 700 nm (but is not limited to this range in one or more embodiments).

As best seen in FIG. 4, OCT light is collimated with a GRIN lens 501a from single mode fiber 507a. The collimated OCT light couples into the core of the double clad fiber 506 (of rotor 306a) via a dichroic filter 502a and a GRIN lens 501b. Also, the back scattered OCT light from the sample (e.g., the sample 106) goes back to the rotor 306a (via the catheter 120). The light is collimated with the GRIN lens 501b and couples into the single mode fiber 507a. In one or more embodiments (and while not limited hereto), the magnification may be approximately or about 1, or is 1, in order to couple fiber efficiently because OCT light is delivered with reversible paths (for example, from stator 306b to rotor 306a and from rotor 306a to stator 306b). In one or more embodiments, coupling efficiency may be improved or maximized when having the magnification be approximately or about 1, or be 1.

One or more embodiments of how to couple OCT and excitation channels into a single core of a double clad fiber in a rotary junction may be used with one or more embodiments of the present disclosure. For example, one or more embodiments of how to couple OCT and excitation channels into a single core of a double clad fiber in a rotary junction may be used as discussed in U.S. Pat. Pub. No. 2018/0348439, published on December 6, 2018, the disclosure of which is incorporated by reference herein in its entirety. One or more features of a rotary joint, a rotary junction, a FORJ, etc. may be used as discussed in U.S. Pat. Pub. No. 2018/0348439, published on December 6, 2018, the disclosure of which is incorporated by reference herein in its entirety.

In one or more embodiments, as best seen in FIG. 4, dichroic filter 502a may be used for separating OCT light from the rest of excitation and fluorescence light. Dichroic filter 502b may be used for a separation of the excitation and fluorescence light. The mirror 504 is used to reflect the excitation light. The long-pass filter 505 may be used to filter out back-reflection and/or stray light of excitation light.

In one or more alternative embodiments, a free space beam combiner, which is located inside an FORJ, may be provided the same as the embodiment shown in FIG. 4, with the following exceptions: the stator 306b of the rotary junction 306 in FIG. 4 may include two optical fibers (and not three) because the multi-mode fiber 508 and the GRIN lens 501d may be removed, and the stator 306b of the rotary junction 306 may include a double clad fiber 506 being used with GRIN lens 501a (instead of the single mode fiber 507a as shown in FIG. 4). OCT light goes through the core of the double clad fiber 506 in the alternative subject stator 306, and may be then collimated with the GRIN lens 501a. The collimated light is coupled into the core of the double clad fiber 506 in the rotor 306a in the subject alternative embodiment. Excitation light, with wavelength shorter than that of OCT light, is converged and focused with the GRIN lens 501c at the middle (or at a predetermined position) of the optical path to GRIN lens 501b. Then, the light is coupled into mostly the core of the double clad fiber 506 in the rotor 306a of the rotary junction 306. In one or more embodiments, fluorescence light from the sample (e.g., the sample 106) may be delivered through mostly the clad of the double clad fiber 506 in rotor 306a. Then, the light may be coupled into the clad of the double clad fiber 506 in the stator 306b. To separate OCT light and fluorescence light, a double clad fiber coupler may be used either inside the PIU 110 or in the imaging subsystem. For example, a dichroic filter 502a may be used to separate excitation light and the rest of the fluorescence and OCT lights. The double clad fiber 506 of the stator 306b may be connected to a core/clad beam splitter to separate OCT and fluorescence light. As such, a simple and more compact FORJ may be achieved with this configuration as an alternative where desired because of a lack of the free-space optical fluorescence channel. Also, it may be easier to fabricate the beam combiner because OCT and fluorescence lights may be coupled using a common double clad fiber (*e*.*g*., the fiber 506). In one or more embodiments, fibers other than a DCF may be used. A FORJ (such as the FORJ 306') and/or other features for same may be used as discussed in U.S. Pat. Pub. No. 2022/0042781 A1, published on February 10, 2022, the disclosure of which is incorporated by reference herein in its entirety. For example, in one or more embodiments, the optical probe may be removably attachable to the optical system and/or FORJ (*e.g*., via a catheter connector), and a catheter disconnect mode or a catheter connect mode may be performed to determine a disconnection/connection or to disconnect/connect the catheter or probe to/from the optical system and/or FORJ. In one or more embodiments, a mirror, a ferrule, a sleeve and/or epoxy as discussed in the present disclosure may be optional, and the fibers, lenses and a dichroic filter may be used without one or more of the mirror, the ferrule, the sleeve and/or the epoxy.

Descriptions of like-numbered elements present in the system(s) 100, 100', 100", and/or the rotary junction 306' are the same for features/elements already described above, such as for the system 100a, the system 100b, the rotary junction 306, any other system discussed herein, etc., shall not be repeated, and are incorporated by reference herein in their entireties.

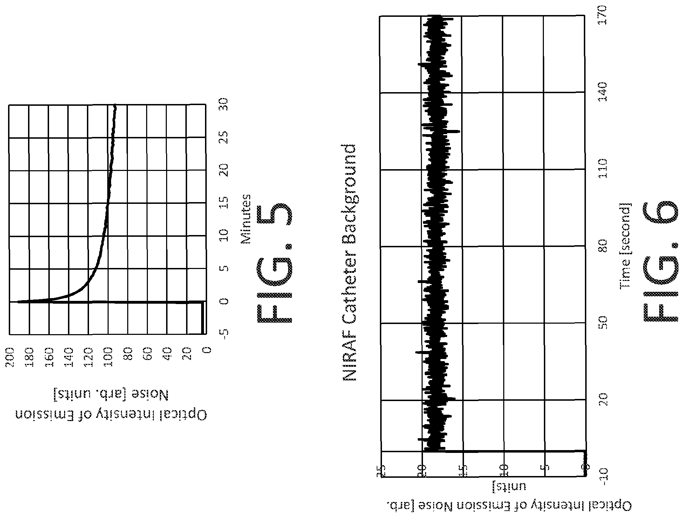

FIG. 5 shows an example of how the emission noise from the optical probe varies when exciting the optical probe (such as the catheter or probe 120). The intensity drops the first 5 minutes by about 40%. Because the noise intensity is depending on an excitation time, it is difficult to estimate the emission noise level.

After processing the photo-bleach optical probe, the emission noise level becomes lower and stable over time, as shown in FIG. 6. The intensity change of the emission noise is within 10% over 2 minutes.

To improve the signal to noise ratio of the fluorescence detection, the catheter background noise are reduced and stabilized.

In one or more embodiments, the coil 122 delivers the torque from the proximal end to the distal end of the catheter 120 or the optical probe 124 by using or via the rotational motor 139 in the PIU 110. There may be a mirror at the distal end of the catheter 120 or the optical probe 124 in one or more embodiments so that the light beam may be deflected outward. The coil 122 may be fixed with the optical probe 124 so that a distal tip of the optical probe 124 and/or the catheter 120 also spins to see omnidirectional view of the inner surface of hollow organ(s), such as vessels, or any other target, object, or sample 106 being viewed/imaged. The optical probe 124 of the catheter 120 may comprise a fiber connector (*e.g*., fiber or catheter connector 141) at a proximal end, a double clad fiber (*e.g.,* DCF 506), and a lens at a distal end. The fiber/catheter connector 141 operates to be connected with the PIU 110 (as best seen in FIG. 2B). The double clad fiber (*e.g.,* DCF 506) may be used to transmit & collect OCT light through the core and to collect auto-fluorescence from a sample (*e*.*g*., the target, object, or sample 106) through the clad. The lens may be used for focusing and collecting light to and/or from the sample (*e.g*., the target, object, or sample 106). In one or more embodiments, the scattered light through the clad may be relatively higher than scattered light through the core because the size of the core is much smaller than the clad.

The catheter background emission noise may be minimized and may become stable by using a photo-bleached optical fiber for a catheter in accordance with one or more features of the present disclosure without suffering from losing fluorescence signal. The intensity change of the emission noise over 2 minutes becomes within 10% and stabilizes. In one or more embodiments, a system using one or more photo-bleach features and/or photo-bleach methods of the present disclosure is able to increase signal to noise ratio, and such a system is able to become a more sensitive system to detect weak NIRF/NIRAF signals.

One or more photo-bleach features and/or methods of the present disclosure operate to reduce and stabilize optical probe emission noise (and/or catheter background noise) for optic catheters and endoscopes. The photo-bleach process(es) of the present disclosure may be performed to an optical probe or probes and/or to components for optical probe(s).

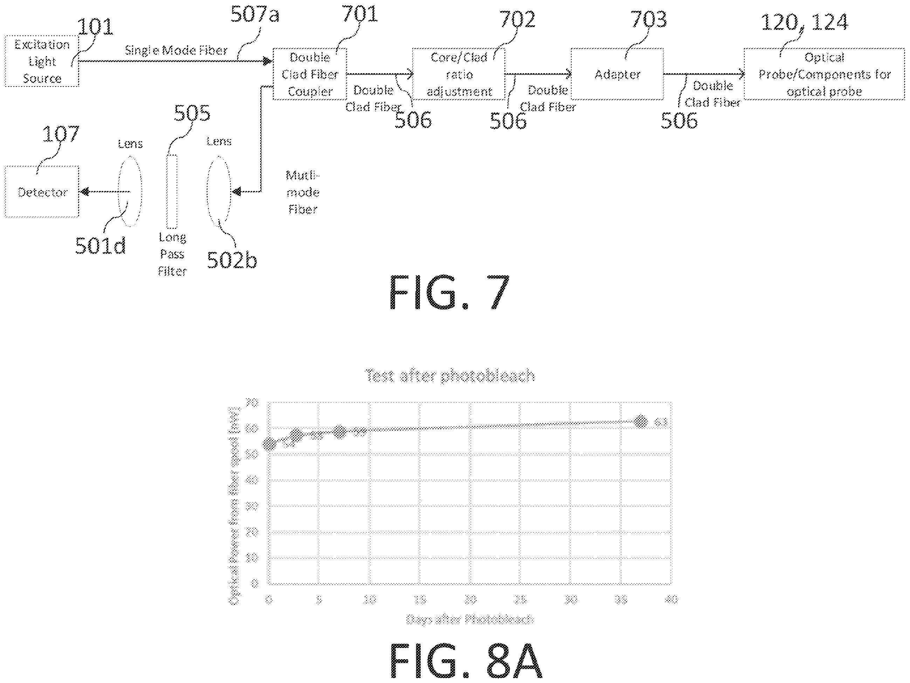

FIG. 7 shows at least one embodiment of a photo-bleached process or example of the present disclosure. An excitation light with a wavelength of about 0.635 um may be delivered or transmitted to a coupler, such as a double clad fiber coupler 701, through a single mode fiber (*e.g.,* a single mode fiber 507a). The excitation light couples into the core of a double clad fiber (*e.g.,* the DCF 506), then the light is delivered to a core/clad ratio adjustment unit or processor 702. The core/clad ration adjustment unit or processor 702 operates to control how much of the excitation light goes to the core and the clad of the double clad fiber (*e.g*., the DCF 506), and then the DCF 506 operates to bring the light to optical probes/components for optical probes 124 of the catheter 120. The adapter 703 may be used for easy connections with the catheter 120 (which may be replaceable (*e.g.,* with another catheter 120) or disposable (*e.g.,* after use, by detaching from the adapter 703, etc.)). In one or more embodiments, the core/clad ratio adjustment unit or processor 702 may include, comprise, or be connected to any of the processors discussed herein, including, but not limited to, the computer or processor 1200, the computer or processor 1200', etc. The optical probe/components of optical probes 124 of the catheter 120 emit auto-fluorescence with broadband wavelengths of 0.65-0.90 um, and the catheter 120 also may emit the Raman scattering around 0.65-0.75um. The auto-fluorescence and the Raman signals are delivered to a detector (*e*.*g.*, the detector 107), such as a photo-multi plier tube (PMT) or photodiode (or other detector(s) discussed herein), via the double clad fiber coupler 701. After the double clad fiber coupler 701, the emission signals are delivered by a multi-mode fiber (*e.g.,* the multi-mode fiber 508) to lens section (*e.g*., such as one or more of the following: a dichroic filter 502b, a long pass filter 505, a lens 501d, etc.) to filter out the excitation light. The detected signals may be monitored during one or more photo-bleach processes, and a DCF (such as the DCF 506) may be used for an optical fiber for the optical probe 124 of the catheter 120.

In one or more embodiments, the core/clad ratio adjustment unit or processor 701 operates to control the excitation light to excite the core and the clad of the double clad fiber (*e.g.,* DCF 506) to perform photo-bleaching efficiently. In one or more embodiments, 10% or more than 10% of the excitation light may go to the clad of the double clad fiber (*e.g.,* DCF 506) for efficient photo-bleaching, and nearly or approximately 50% or 50% of the core and clad ratio is more efficient and minimizes the photo-bleach time. In one or more embodiments, the photo-bleaching duration may be more than 30 minutes to stabilize the emission noise, and may be more than 24 hours to reduce more emission noises moreover. 30 minutes after photo-bleaching, the photo-bleached optical probe may become stable, as shown in FIG. 6. Additionally, after such a photo-bleaching process, the emission noise will not come back to an original noise level, shown in FIG. 8A, for example. Tests were conducted as follow-up photo-bleach tests to measure the effectiveness of a photo-bleached fiber, optical probe, and/or catheter (or one or more components thereof) over time. The results of the tests clearly indicated that the validity of the photo-bleaching features of the present disclosure is/are sustained over forty (40) days after a photo-bleaching process. For example, the optical intensity of emission noise was reduced by half at or within a first thirty (30) minutes of a photo-bleaching process(es) of the present disclosure, as shown in FIG. 6, while the optical power intensity was increased by just (i) 5.9% increased after two (2) days; (ii) 8.1% increased after one (1) week; and (iii) 13% increased after 1 month. The components of an optical probe (e.g., the optical probe 124 and/or the catheter 120) may be an optical fiber spool so that the long optical fiber may be photo-bleached at the same time. With such process feature(s), the photo-bleach does not have to be performed for each optical probe (*e*.*g*., the optical probe 124 and/or the catheter 120), and such feature(s) will save processing time.

The one or more photo-bleach method features discussed above may be used for optical probes/components of optical probes (*e.g*., the optical probe 124, the catheter 120, any component of the catheter 120, etc.). With these photo-bleached process(es)/feature(s), obtaining optical probes/catheters with lowered and stabilized background emission noise may be achieved. By using the photo-bleached optical probe 124 and/or catheter 120, a fluorescence system with a high signal to noise ratio may be achieved.

Once trained, a neural network or networks may use a single image (*e.g*., a single OCT image, an image of a different imaging modality, etc.) to determine or identify one or more tissue types and/or tissue characteristics (or otherwise characterize tissue) and/or to determine or identify whether an optical probe 124 and/or catheter 120 (or one or more other portions of the catheter 120) have been photo-bleached.

In one or more embodiments, a method for photo-bleaching an interference optical system and/or one or more optical probes may include: using or providing an excitation laser having a wavelength of 400 nm - 900 nm; coupling the excitation laser into the interference optical system, the one or more optical probes, and/or one or more components of the one or more optical probes; and exciting the interference optical system, the one or more optical probes, and/or one or more components of the one or more optical probes with the excitation laser for more than or equal to a set or predetermined amount of time. In one or more embodiments, the set or predetermined amount of time is thirty (30) minutes or is about thirty (30) minutes. In one or more embodiments, a user may set the amount of time for performing the photo-bleaching. In one or more embodiments, the one or more optical probes and/or the one or more components of the one or more optical probes may include or comprise of (or may each include or comprise of) a double clad fiber. In one or more embodiments, the set or predetermined amount of time for performing the excitation may be for 24 hours or more than 24 hours. In one or more embodiments, one or more processors may perform or control the method for photo-bleaching. The one or more processors may receive the set or predetermined amount of time to perform the photo-bleaching or may automatically calculate and set the set or predetermined amount of time (*e.g*., based on a number of components or structure to be photo-bleached, based on a size and shape of the structure or the number of components to be photo-bleached, etc.).

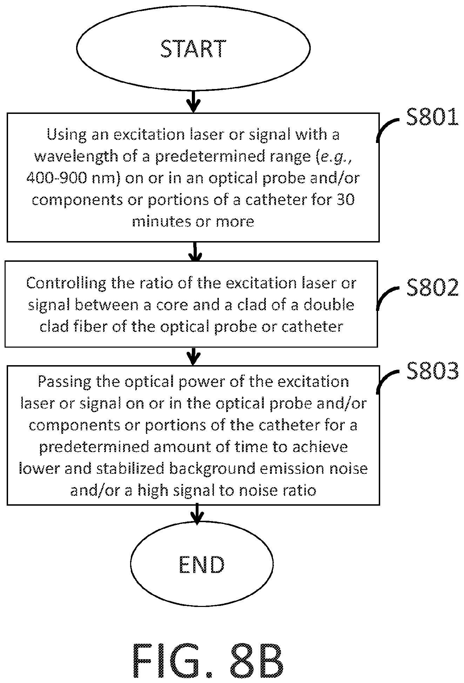

FIG. 8B shows at least one embodiment of a method for a photo-bleaching method for an optical probe and/or catheter (or one or more components or portions of the optical probe and/or catheter) that may be used in accordance with one or more aspects of the present disclosure. In one or more embodiments, a photo-bleaching method or methods may include one or more of the following: (i) using an excitation laser or signal with a wavelength of a predetermined range (*e.g*., 400-900 nm) on or in an optical probe and/or components or portions of a catheter for 30 minutes or more (*see e.g.,* step S801 in FIG. 8B); (ii) controlling the ratio of the excitation laser or signal between a core and a clad of a double clad fiber of the optical probe or catheter (*see e.g.,* step s802 in FIG. 8B); and (iii) passing the optical power of the excitation laser or signal on or in the optical probe and/or components or portions of the catheter for a predetermined amount of time to achieve lower and stabilized background emission noise and/or a high signal to noise ratio (*see e.g.,* step s803 in FIG. 8B). In one or more embodiments, the data for or from performing photo-bleaching may be saved in one or more memories or may be sent to one or more processors and/or a neural net (or other AI compatible network or AI-ready network) for use in the AI evaluations/determinations or for use with any other technique(s) or process(es) discussed herein. For example, one or more embodiments of the present disclosure may incorporate image processing and machine learning (ML) to automatically identify and locate sufficiently photo-bleached portions or components of the optical probe and/or catheter and/or may use AI-related features to control any aspect of photo-bleaching, including, but not limited to, controlling the intensity of the excitation laser or light during photo-bleaching, based on innovative feature(s) of the present disclosure. As aforementioned, in one or more embodiments, 10% or more than 10% of the excitation light may go or be transmitted to the clad of the double clad fiber (*e.g.,* DCF 506) for efficient photo-bleaching, and nearly or approximately 50% or 50% of the core and clad ratio may be achieved for improved efficiency and to minimize the photo-bleach time. In one or more embodiments, the photo-bleaching duration may be about 30 minutes or more than 30 minutes to stabilize the emission noise, and may be more than 24 hours to reduce more emission noises moreover. The components of an optical probe (*e.g.*, the optical probe 124 and/or the catheter 120) may be an optical fiber spool so that the long optical fiber may be photo-bleached at the same time in one or more embodiments. With such process feature(s), the photo-bleach does not have to be performed for each optical probe (*e.g*., the optical probe 124 and/or the catheter 120), and such feature(s) will save processing time. In one or more embodiments, the method may include use of a detector and a lens unit or component(s). The lens unit or component(s) may operate to filter the excitation light and pass through the emission from the optical probe 124 and/or components of the optical probe 124 or of the catheter 120. The components of the optical probe 124 and/or the catheter 120 may include or comprise a double clad fiber (*e.g.,* DCF 506). An optical power of the excitation laser or light that is transmitted in or through the optical probe 124 and/or the components of the optical probe 124 and/or of the catheter 120 may be one or more of the following: (i) an amount that is same or similar to a nominal or predetermined intensity as when the power and excitation laser or light is used in a probe or in the system, where the amount is at least 0.1 milliWatts (mW); (ii) two (2) times higher than the nominal intensity, which is at least 0.2-0.5 mW; (iii) ten (10) times higher than the nominal intensity, which is at least 1 mW; and/or (iv) one-hundred (100) times higher than the nominal intensity, which is at least 10 mW. In one or more embodiments, the optical power of the excitation laser or light may be an amount in the range of at least 0.1 mW - at least 10 mW. In one or more embodiments, the optical power may be over 10 mW. In one or more embodiments using or involving a double clad fiber, the one or more processors may include or may be used with a core/clad ratio adjustment processor or unit that operates to control a ratio of excitation light between a core and a clad of the double clad fiber. In one or more embodiments, the one or more processors operate to set or use, or the method includes setting or using, the ratio of the clad (*e.g.,* the ratio of the amount of excitation light being sent to the clad) as being 10% or more than 10% (so that the ratio value for the amount of excitation light sent to the core is 90% or less than 90%). In one or more embodiments, the one or more processors operate to set or use, or the method includes setting or using, the ratio of the clad (*e.g.,* the ratio of the amount of excitation light being sent to the clad) as being 50% or being nearly or about 50% (so that the ratio value for the amount of excitation light sent to the core is 50% or a little over or about 50% (*e.g.,* the ratio value for the amount of excitation light may be a little over or about 50% by a difference that the ratio of the amount of excitation light being sent to the clad is nearly or about 50% (*e.g.,* in a case where the ratio value for the clad may be 47% (a 3% difference under 50%), then the ratio value for the core may be 53% (the 3% difference over 50%)).

Embodiments of a method or methods for detecting or identifying one or more tissue types and/or tissue characteristics and/or for imaging may be used independently or in combination, including, but not limited to, independently from or in combination with photo-bleaching features of the present disclosure.

In one or more embodiments, a model (which, in one or more embodiments, may be software, software/hardware combination, or a procedure that utilizes one or more machine or deep learning algorithms/procedures/processes that has/have been trained on data to make one or more predictions for future, unseen data) has enough resolution to predict and/or evaluate the tissue characterization and/or the photo-bleaching estimate(s) and/or photo-bleaching result(s) with sufficient accuracy depending on the application or procedure being performed. The performance of the model may be further improved by subsequently adding more training data and retraining the model to create a new instance of the model with better or optimized performance. For example, additional training data may include data based on user input, where the user may identify or correct the location of a tissue or tissues in an image and/or may identify or correct the location and/or amount of photo-bleaching for the optical probe 124 and/or components of the optical probe 124 and/or of the catheter 120.

One or more methods, medical imaging devices, Intravascular Ultrasound (IVUS) or Optical Coherence Tomography (OCT) devices, imaging systems, and/or computer-readable storage mediums for evaluating tissue characterization(s) and/or for performing photo-bleaching using artificial intelligence may be employed in one or more embodiments of the present disclosure.

In one or more embodiments, an artificial intelligence training apparatus using a neural network or other AI-ready network may include: a memory; one or more processors in communication with the memory, the one or more processors operating to: training a classifier or patch feature extraction and training an AI-classifier (*e.g*., a ML classifier, a DL classifier, etc.). In one or more embodiments of the present disclosure, an apparatus, a system, or a storage medium may use an AI network, a neural network, or other AI-ready network to perform any of the aforementioned method step(s), including, but not limited to, the steps of FIG. 8B and/or any other step(s) discussed herein.

The one or more processors may further operate to use one or more neural networks, convolutional neural networks, and/or recurrent neural networks (or other AI-ready or AI compatible network(s)) to one or more of: load the trained model, select a set of image frames, evaluate the tissue characterization and/or the photo-bleaching, construct the image (*e.g.,* the carpet view image (CVI)), perform the coregistration and/or the photo-bleaching, overlay data on the image and/or the intravascular image(s) (*e.g*., the CVI, the OCT image(s), etc.) and acquire or receive the image data during the pullback operation.

In one or more embodiments, the object, target, or sample may include one or more of the following: a vessel, a target specimen or object, one or more tissues, a patient (or a target or tissue(s) in the patient), one or more optical probe(s) and/or catheter(s) and/or one or more components of the optical probe(s) and/or catheter(s).

The one or more processors may further operate to perform the coregistration by co-registering an acquired or received angiography image and an obtained one or more intravascular images, such as, but not limited to. Optical Coherence Tomography (OCT) or Intravascular Ultrasound (IVUS) images or frames, and/or by co-registering the carpet view (CVI) with the one or more intravascular images, such as, but not limited to, Optical Coherence Tomography (OCT) or Intravascular Ultrasound (IVUS) images or frames.

In one or more embodiments, a loaded, trained model may be one or a combination of the following: a random forest(s) model, a Support Vector Machine (SVM) model, a segmentation (classification) model, a segmentation model with pre-processing, a segmentation model with post-processing, an object detection (regression) model, an object detection model with pre-processing, an object detection model with post-processing, a combination of a segmentation (classification) model and an object detection (regression) model, a deep convolutional neural network model, a recurrent neural network model with long short-term memory that can take temporal relationships across images or frames into account, a model using feature pyramid(s) that can take different image resolutions into account, a genetic algorithm that operates to breed multiple models for improved performance, and/or a model using residual learning technique(s).

In one or more embodiments, the one or more processors may further operate to one or more of the following: perform any of the steps of FIG. 8B, perform any of the steps or feature(s) related to FIGS. 1A-8A, 9A-10, 11-17, and/or any combination of the steps or features discussed in the present disclosure.

One or more embodiments of the present disclosure may use other artificial intelligence technique(s) or method(s) for performing training, for splitting data into different groups (e.g., training group, validation group, test group, etc.), or other artificial intelligence technique(s) or method(s), such as, but not limited to, embodiment(s) as discussed in PCT/US2020/051615, filed on September 18, 2020 and published as WO 2021/055837 A9 on March 25, 2021, and as discussed in U.S. Pat. App. No. 17/761,561, filed on March 17, 2022, the applications and publications of which are incorporated by reference herein in their entireties. For example, angiography data and/or intravascular data may be used for training, validation, and/or testing as desired. One or more embodiments of a non-transitory computer-readable storage medium storing at least one program for causing a computer to execute a method for training a model using artificial intelligence may be used with any method(s) discussed in the present disclosure, including but not limited to, one or more tissue type and/or characteristic evaluation/determination method(s).

In one or more embodiments of the present disclosure, an OCT image may be formed in a polar coordinate system from A-lines. Each A-line includes much information about the imaged object, such as, but not limited to: clear indications of artifacts from metal objects (e.g., stents, stent struts, guide wires, etc.) like narrow signal width and/or sharp rising and falling edges; significant difference in signal intensity and shape for unobstructed soft tissue compared to the sheath reflection and other artifacts like wide signal width and a gentle falling edge, tissue type and/or tissue characterization, etc.. Each A-line represents a cross-sectional 1D sampling of a target, sample, object, etc., such as, but not limited to, a vessel, along a certain view angle. As an imaging probe or device rotates (*e.g*., rotates about 0 to about 360 degrees, about 180 degrees to about 360 degrees, about 360 degrees, etc.), the corresponding A-lines form the complete two-dimensional (2D) cross-section of the target, sample, object, etc. (*e.g.,* the vessel) in polar coordinates, which is then converted into Cartesian coordinates to form a tomographical-view (tomo-view) image of the cross-section of the target, sample, object, etc. (*e.g.,* the vessel).

In accordance with at least one aspect of the present disclosure and as aforementioned, one or more additional methods for target or object detection of OCT images are provided herein and are discussed in U.S. Pat. App. No. 16/414,222, filed on May 16, 2019 and published on December 12, 2019 as U.S. Pat. Pub. No. 2019/0374109, the entire disclosure of which is incorporated by reference herein in its entirety. By way of a few examples, pre-processing may include, but is not limited to, one or more of the following steps: (1) smoothening the 2D image in the Polar coordinate (*e.g*., using a Gaussian filter, as discussed below, etc.), (2) computing vertical and/or horizontal gradients using a Sobel operator (as otherwise discussed below, etc.), and/or (3) computing binary image using an Otsu method. For example, Otsu's method is an automatic image thresholding technique to separate pixels into two classes, foreground and background, and the method minimizes the intraclass variances between two classes and is equivalent to a globally optimal k-means (*see e.g.,* https://en.wikipedia.org/wiki/Otsu%27s_method). One skilled in the art would appreciate that pre-processing methods other than Otsu's method (such as, but not limited to, Jenks optimization method) may be used in addition to or alternatively to Otsu's method in one or more embodiments.

By way of at least one embodiment example of sheath, a Polar coordinate image (*e.g.,* an OCT Polar coordinate image) may include (from the top side to the bottom side, from the top side to the bottom side of an OCT Polar coordinate image, etc.) a sheath area, and a normal field of view (FOV). In one or more embodiments, the lumen area and edge are within the FOV. Because one or more shapes of the sheath may not be a circle (as may be typically assumed) and because the sheath (and, therefore, the sheath shape) may be attached to or overlap with the lumen or guide wire, it may be useful to separate the sheath from the other shapes (*e.g*., the lumen, the guide wire, tissue(s), etc.) ahead of time.

By way of at least one embodiment example of computing/finding a peak and a major or maximum gradient edge (*e.g*., for each A-line), soft tissue and other artifacts may be presented on each A-line by one or more peaks with different characteristics, for example, in one or more embodiments of a lumen OCT image(s) (*e.g*., a normal lumen OCT image). For example, the soft tissue may have a wide bright region beyond the lumen edge, while the artifacts may produce an abrupt dark shadow area beyond the edge. Due to the high-resolution nature of one or more OCT images, transitions between neighbor A-lines may have signals for both peaks. Such signals may allow one or more method embodiments or processes to obtain more accurate locations of the artifact objects and/or the lumen edges. In one or more embodiments, detection of tissue, lumen edges, artifacts, etc. may be performed as discussed in U.S. Pat. Pub. No. 2021/0174125 A1, published on 6/10/2021, the disclosure of which is incorporated by reference herein in its entirety.

Additionally or alternatively, in one or more embodiments, a principal component analysis method and/or a regional covariance descriptor(s) may be used to detect objects, such as tissue(s), and/or to detect, evaluate, and/or perform photo-bleaching. Cross-correlation among neighboring images may be used to improve tissue characterization and/or detection result(s) and/or to improve photo-bleaching estimate(s) and/or result(s). One or more embodiments may employ segmentation based image processing and/or gradient based edge detection to improve result(s).

One or more methods or algorithms for performing co-registration and/or imaging may be used in one or more embodiments of the present disclosure, including, but not limited to, the methods or algorithms discussed in U.S. Pat. No. 12,076,177, issued on September 3, 2024, discussed in U.S. Pat. Pub. No. 2022/0104786 A1, published on April 7, 2022, and discussed in U.S. Pat. Pub. No. 2019/0029624, which patent(s) and publications are incorporated by reference herein in their entireties.

Such information and other features discussed herein may be applied to other applications, such as, but not limited to, controlling or modifying photo-bleaching region(s) of the component(s) of the optical probe 124 and/or of the catheter 120, co-registration, other modalities, etc. Indeed, the useful applications of the features of the present disclosure and of the aforementioned applications and patent publications are not limited to the discussed modalities, images, or medical procedures. Additionally, depending on the involved modalities, images, or medical procedures, one or more control bars may be contoured, curved, or have any other configuration desired or set by a user. For example, in an embodiment using a touch screen as discussed herein, a user may define or create the size and shape of a control bar based on a user moving a pointer, a finger, a stylus, another tool, etc. on the touch screen (or alternatively by moving a mouse or other input tool or device regardless of whether a touch screen is used or not). For example, in one or more embodiments, a control bar or a cursor may be used to target region(s) or component(s) of the optical probe 124 and/or of the catheter 120 to be photo-bleached.

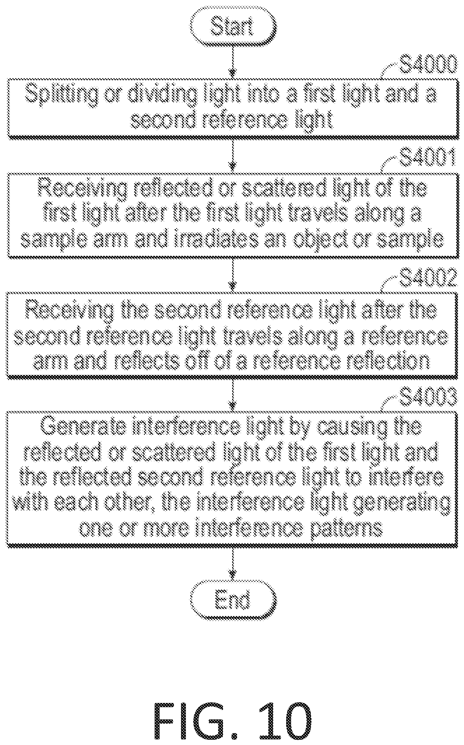

A computer, such as the console or computer 1200, 1200', may perform any of the steps (e.g., method step(s) related to FIGS. 1A-8A; steps S801-S803 of FIG. 8B; steps S4000-S4003 of FIG. 10 discussed further below; etc.) for any system being manufactured or used, including, but not limited to, system 20, system 100a, system 100b, system 100, system 100', system 100", etc.

In accordance with one or more further aspects of the present disclosure, bench top systems may be utilized for one or more features of the present disclosure, such as, but not limited to, for one or more imaging modalities (such as, but not limited to, angiography, Optical Coherence Tomography (OCT), Multi-modality OCT (MM-OCT), near-infrared auto-fluorescence (NIRAF), near-infrared fluorescence (NIRF), OCT-NIRAF, OCT-NIRF, etc.), and/or for employing one or more additional features discussed herein, including, but not limited to, artificial intelligence processes (*e.g*., machine or deep learning, residual learning, artificial intelligence ("AI") co-registration, tissue detection, tissue characterization, photo-bleaching, etc.) in accordance with one or more aspects of the present disclosure.

FIG. 9A shows an OCT system 100 (as referred to herein as "system 100" or "the system 100") which may be used for one or more imaging modalities, such as, but not limited to, angiography, Optical Coherence Tomography (OCT), Multi-modality OCT (MM-OCT), near-infrared autofluorescence (NIRAF), near-infrared fluorescence (NIRF), OCT-NIRAF, etc., and/or for employing one or more additional features discussed herein, including, but not limited to, artificial intelligence processes (*e.g*., machine or deep learning, residual learning, artificial intelligence ("AI") co-registration, tissue characterization, photo-bleaching, etc.) or other process(es) (*e.g*., co-registration, tissue detection, tissue characterization, photo-bleaching, etc.) in accordance with one or more aspects of the present disclosure. The system 100 comprises a light source 101, a reference arm 102, a sample arm 103, a deflected or deflecting section 108, a reference mirror (also referred to as a "reference reflection", "reference reflector", "partially reflecting mirror" and a "partial reflector") 105, and one or more detectors 107 (which may be connected to a computer 1200). In one or more embodiments, the system 100 may include a patient interface device or unit ("PIU") 110 and a catheter or probe 120 *(see e.g.,* embodiment examples of a PIU and a catheter as shown in FIGS. 1A-4, FIG. 7, and/or FIGS. 9A-9C), and the system 100 may interact with an object 106, a patient (e.g., a blood vessel of a patient) 106, a sample, one or more tissues, one or more portions or components of an optical probe 124 and/or of the catheter 120, etc. (*e.g.*, via the catheter 120 and/or the PIU 110). In one or more embodiments, the system 100 includes an interferometer or an interferometer is defined by one or more components of the system 100, such as, but not limited to, at least the light source 101, the reference arm 102, the sample arm 103, the deflecting section 108 and the reference mirror 105.

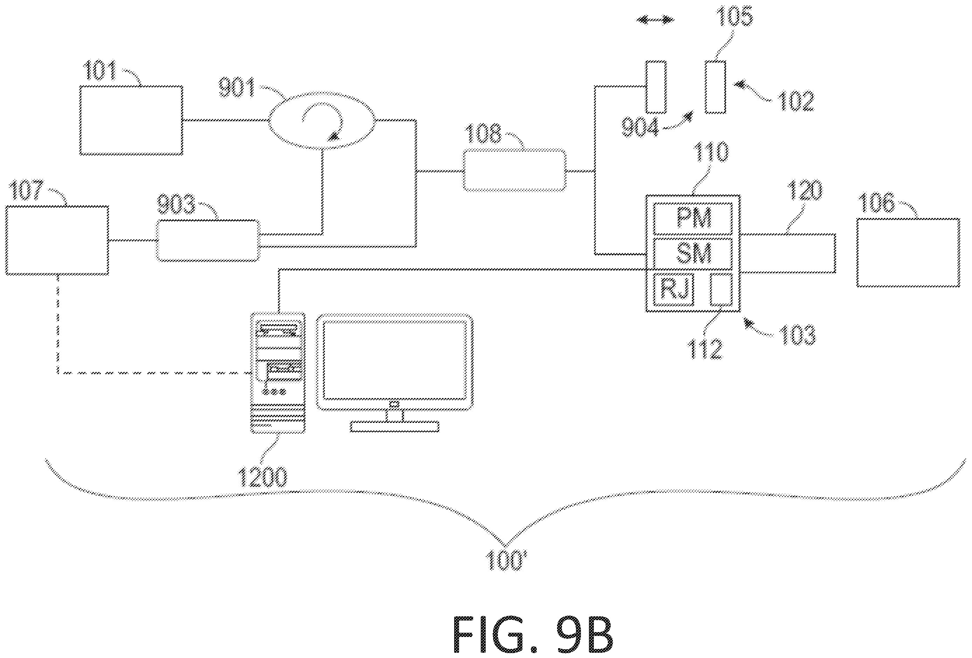

FIG. 9B shows an example of a system that can utilize the one or more imaging modalities, such as, but not limited to, angiography, Optical Coherence Tomography (OCT), Multi-modality OCT (MM-OCT), near-infrared auto-fluorescence (NIRAF), near-infrared fluorescence (NIRF), OCT-NIRAF, OCT-NIRF, etc., and/or can be used for employing one or more additional features discussed herein, including, but not limited to, artificial intelligence processes (*e.g*., machine or deep learning, residual learning, artificial intelligence ("AI"), or other AI features discussed herein) or other process(es) (*e.g*., co-registration, tissue detection, tissue characterization, photo-bleaching, etc.) in accordance with one or more aspects of the present disclosure discussed herein for a bench-top such as for ophthalmic applications. A light from a light source 101 delivers and splits into a reference arm 102 and a sample arm 103 with a deflecting section 108. A reference beam goes through a length adjustment section 904 and is reflected from a reference mirror (such as or similar to the reference mirror or reference reflection 105 shown in FIG. 9A) in the reference arm 102 while a sample beam is reflected or scattered from an object, a patient (*e.g*., blood vessel of a patient), etc. 106 in the sample arm 103 (*e.g*., via the PIU 110 and the catheter 120). In one embodiment, both beams combine at the deflecting section 108 and generate interference patterns. In one or more embodiments, the beams go to the combiner 903, and the combiner 903 combines both beams via the circulator 901 and the deflecting section 108, and the combined beams are delivered to one or more detectors (such as the one or more detectors 107). The output of the interferometer is continuously acquired with one or more detectors, such as the one or more detectors 107. The electrical analog signals are converted to the digital signals to analyze them with a computer, such as, but not limited to, the computer 1200 (*see* FIGS. 1B, 3A-3B, and 9A-9C; also shown in FIGS. 11 and 13 discussed further below), the computer 1200' (*see e.g.,* FIGS. 3A, 12 and 13 discussed further below), the computer 2 (*see* FIG. 1A), the processors 26, 36, 50 (*see* FIG. 1B), any other computer or processor discussed herein, etc. Additionally or alternatively, one or more of the computers, CPUs, processors, etc. discussed herein may be used to process, control, update, emphasize, and/or change one or more of imaging modalities, and/or process the related techniques, functions or methods, or may process the electrical signals as discussed above.

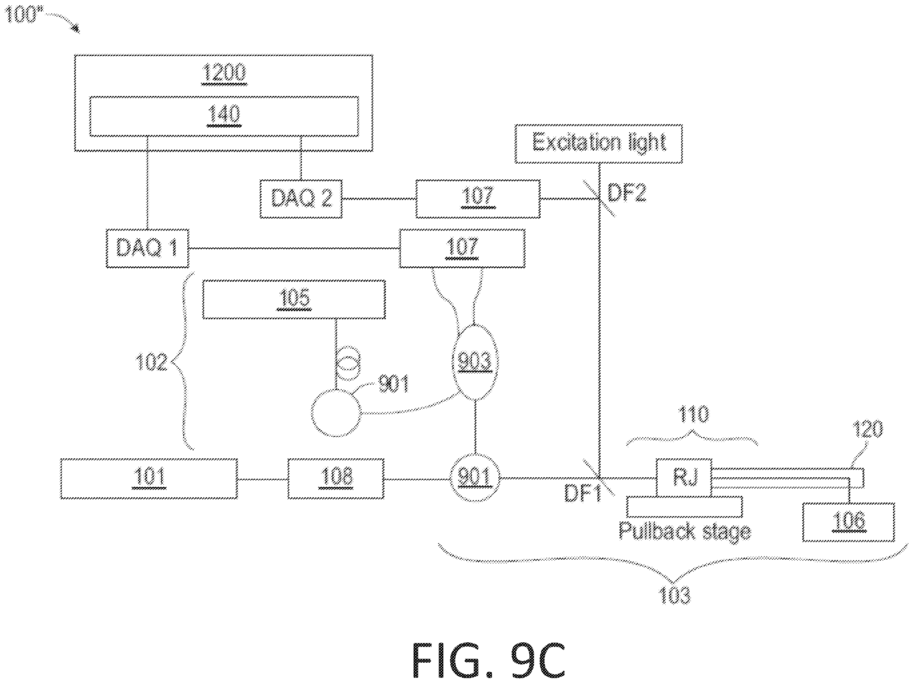

The electrical analog signals may be converted to the digital signals to analyze them with a computer, such as, but not limited to, the computer 1200 (*see* FIGS. 1B, 3A-3B, and 9A-9C; also shown in FIGS. 11 and 13 discussed further below), the computer 1200' (*see e.g.,* FIGS. 3A, 12, and 13 discussed further below), the computer 2 (*see* FIG. 1A), any other processor or computer discussed herein, etc. Additionally or alternatively, one or more of the computers, CPUs, processors, etc. discussed herein may be used to process, control, update, emphasize, and/or change one or more imaging modalities, and/or process the related techniques, functions or methods, or may process the electrical signals as discussed above. In one or more embodiments (*see e.g.,* FIG. 9B), the sample arm 103 includes the PIU 110 and the catheter 120 so that the sample beam is reflected or scattered from the object, patient (*e.g*., blood vessel of a patient), etc. 106 as discussed herein. In one or more embodiments, the PIU 110 may include one or more motors to control the pullback operation of the catheter 120 (or one or more components thereof) and/or to control the rotation or spin of the catheter 120 (or one or more components thereof) (*see e.g.,* the motor M of FIG. 1B). For example, as best seen in FIG. 9B, the PIU 110 may include a pullback motor (PM) and a spin motor (SM), and/or may include a motion control unit 112 that operates to perform the pullback and/or rotation features using the pullback motor PM and/or the spin motor SM. As discussed herein, the PIU 110 may include a rotary junction (*e.g*., rotary junction RJ as shown in FIGS. 9B and 9C). The rotary junction RJ may be connected to the spin motor SM so that the catheter 120 may obtain one or more views or images of the object, patient (*e.g*., blood vessel of a patient, tissue(s), etc.), portion(s) or component(s) of the optical probe 124 and/or of the catheter 120, etc. 106. The computer 1200 (or the computer 1200', computer 2, any other computer or processor discussed herein, etc.) may be used to control one or more of the pullback motor PM, the spin motor SM and/or the motion control unit 112. An OCT system may include one or more of a computer (*e.g*., the computer 1200, the computer 1200', computer 2, any other computer or processor discussed herein, etc.), the PIU 110, the catheter 120, a monitor (such as the display 1209), etc. One or more embodiments of an OCT system may interact with one or more external systems, such as, but not limited to, an angio system, external displays, one or more hospital networks, external storage media, a power supply, a bedside controller (e.g., which may be connected to the OCT system using Bluetooth technology or other methods known for wireless communication), etc.

In one or more embodiments including the deflecting or deflected section 108 (best seen in FIGS. 9A-9C), the deflected section 108 may operate to deflect the light from the light source 101 to the reference arm 102 and/or the sample arm 103, and then send light received from the reference arm 102 and/or the sample arm 103 towards the at least one detector 107 *(e.g.,* a spectrometer, one or more components of the spectrometer, another type of detector, etc.). In one or more embodiments, the deflected section (e.g., the deflected section 108 of the system 100, 100', 100", any other system discussed herein, etc.) may include or may comprise one or more interferometers or optical interference systems that operate as described herein, including, but not limited to, a circulator, a beam splitter, an isolator, a coupler (e.g., fusion fiber coupler), a partially severed mirror with holes therein, a partially severed mirror with a tap, etc. In one or more embodiments, the interferometer or the optical interference system may include one or more components of the system 100 (or any other system discussed herein) such as, but not limited to, one or more of the light source 101, the deflected section 108, the rotary junction RJ, a PIU 110, a catheter 120, etc. One or more features of the aforementioned configurations of at least FIGS. 1A-9B (and/or any other configurations discussed below) may be incorporated into one or more of the systems, including, but not limited to, the system 20, 100a, 100b, 100, 100', 100", etc. discussed herein.

In accordance with one or more further aspects of the present disclosure, one or more other systems may be utilized with one or more of the multiple imaging modalities and related method(s) as disclosed herein. FIG. 9C shows an example of a system 100" that may utilize the one or more multiple imaging modalities, such as, but not limited to, angiography, Optical Coherence Tomography (OCT), Multi-modality OCT (MM-OCT), near-infrared auto-fluorescence (NIRAF), near-infrared fluorescence (NIRF), OCT-NIRAF, OCT-NIRF, etc., and/or for employing one or more additional features discussed herein, including, but not limited to, artificial intelligence processes (*e.g*., machine or deep learning, residual learning, artificial intelligence ("AI") co-registration, tissue detection, tissue characterization, etc.) or other process(es) (*e.g*., co-registration, tissue detection, tissue characterization, photo-bleaching, etc.) and/or related technique(s) or method(s) such as for ophthalmic applications in accordance with one or more aspects of the present disclosure. FIG. 9C shows an exemplary schematic of an OCT-fluorescence imaging system 100", according to one or more embodiments of the present disclosure. An OCT light source 101 (*e.g*., with a 1.3µm) is delivered and split into a reference arm 102 and a sample arm 103 with a deflector or deflected section (*e.g*., a splitter) 108, creating a reference beam and sample beam, respectively. The reference beam from the OCT light source 101 is reflected by a reference mirror 105 while a sample beam is reflected or scattered from an object (*e.g*., an object to be examined, an object, a target, a patient, etc.) 106 through a circulator 901, a rotary junction 90 ("RJ") and a catheter 120. In one or more embodiments, the fiber between the circulator 901 and the reference mirror or reference reflection 105 may be coiled to adjust the length of the reference arm 102 (best seen in FIG. 9C). Optical fibers in the sample arm 103 may be made of double clad fiber ("DCF"). Excitation light for the fluorescence may be directed to the RJ 90 and the catheter 120, and illuminate the object (*e.g.*, an object to be examined, an object, a patient, etc.) 106. The light from the OCT light source 101 may be delivered through the core of DCF while the fluorescence light emitted from the object (*e.g.*, an object to be examined, an object, a target, a patient, etc.) 106 may be collected through the cladding of the DCF. For pullback imaging, the RJ 90 may be moved with a linear stage to achieve helical scanning of the object (*e.g*., an object to be examined, an object, a target, a patient, etc.) 106. In one or more embodiments, the RJ 90 may include any one or more features of an RJ as discussed herein. Dichroic filters DF1, DF2 may be used to separate excitation light and the rest of fluorescence and OCT lights. For example (and while not limited to this example), in one or more embodiments, DF1 may be a long pass dichroic filter with a cutoff wavelength of ~1000 nm, and the OCT light, which may be longer than a cutoff wavelength of DF1, may go through the DF1 while fluorescence excitation and emission, which are a shorter wavelength than the cut off, reflect at DF1. In one or more embodiments, for example (and while not limited to this example), DF2 may be a short pass dichroic filter; the excitation wavelength may be shorter than fluorescence emission light such that the excitation light, which has a wavelength shorter than a cutoff wavelength of DF2, may pass through the DF2, and the fluorescence emission light reflect with DF2. In one embodiment, both beams combine at the deflecting section 108 and generate interference patterns. In one or more embodiments, the beams go to the coupler or combiner 903, and the coupler or combiner 903 combines both beams via the circulator 901 and the deflecting section 108, and the combined beams are delivered to one or more detectors (such as the one or more detectors 107; *see e.g.,* the first detector 107 connected to the coupler or combiner 903 in FIG. 9C).

In one or more embodiments, the optical fiber in the catheter 120 operates to rotate inside the catheter 120, and the OCT light and excitation light may be emitted from a side angle of a tip of the catheter 120. After interacting with the object or patient 106, the OCT light may be delivered back to an OCT interferometer (*e.g*., via the circulator 901 of the sample arm 103), which may include the coupler or combiner 903, and combined with the reference beam (*e.g*., via the coupler or combiner 903) to generate interference patterns. The output of the interferometer is detected with a first detector 107, wherein the first detector 107 may be photodiodes or multi-array cameras, and then may be recorded to a computer (*e.g*., to the computer 2, the computer 1200 as shown in FIG. 9C, the computer 1200', or any other computer discussed herein) through a first data-acquisition unit or board ("DAQ1").

Simultaneously or at a different time, the fluorescence intensity may be recorded through a second detector 107 (*e.g.,* a photomultiplier) through a second data-acquisition unit or board ("DAQ2"). The OCT signal and fluorescence signal may be then processed by the computer (*e.g.,* to the computer 2, the computer 1200 as shown in FIG. 9C, the computer 1200', or any other computer discussed herein) to generate an OCT-fluorescence data set 140, which includes or is made of multiple frames of helically scanned data. Each set of frames includes or is made of multiple data elements of co-registered OCT and fluorescence data, which correspond to the rotational angle and pullback position.

In one or more embodiments, any of the systems 100a, 100b, 100, 100', 100", any other system discussed herein, etc. may be used to perform photo-bleaching feature(s) (*e.g*., estimating photo-bleaching, performing photo-bleaching, detecting photo-bleaching results, etc.) to one or more portions or components of the optical probe 124 and/or the catheter 120 of a respective system or of another system.

Detected fluorescence or auto-fluorescence signals may be processed or further processed as discussed in U.S. Pat. No. 11,707,186, issued on July 25, 2023, the disclosure of which is incorporated herein by reference in its entirety, and/or as discussed in U.S. Pat. No. 10,952,616, issued on March 23, 2021, the disclosure of which is incorporated herein by reference herein in its entirety.

While not limited to such arrangements, configurations, devices or systems, one or more embodiments of the devices, apparatuses, systems, methods, storage mediums, GUI's, etc. discussed herein may be used with an apparatus or system as aforementioned, such as, but not limited to, for example, the system 20, the system 100a, the system 100b, the system 100, the system 100', the system 100", the devices, apparatuses, or systems of FIGS. 1A-4, 7, and 9A-17, any other device, apparatus or system discussed herein, etc. and/or may be used with any AI-ready network discussed herein or known to those skilled in the art. In one or more embodiments, one user may perform the method(s) discussed herein. In one or more embodiments, one or more users may perform the method(s) discussed herein. In one or more embodiments, one or more of the computers, CPUs, processors, etc. discussed herein may be used to process, control, update, emphasize, and/or change one or more of the imaging modalities, and/or process the related techniques, functions or methods, or may process the electrical signals as discussed above.

The light source 101 may include a plurality of light sources or may be a single light source. The light source 101 may be a broadband lightsource, and may include one or more of a laser, an organic light emitting diode (OLED), a light emitting diode (LED), a halogen lamp, an incandescent lamp, supercontinuum light source pumped by a laser, and/or a fluorescent lamp. The light source 101 may be any light source that provides light which may then be dispersed to provide light which is then used for imaging, performing control, viewing, changing, emphasizing methods for imaging modalities, constructing or reconstructing image(s) or structure(s), characterizing tissue, and/or any other method discussed herein. The light source 101 may be fiber coupled or may be free space coupled to the other components of the apparatus and/or system 100a, 100b, 100, 100', 100", the devices, apparatuses or systems of FIGS. 1A-4, 7, and 9A-17, or any other embodiment discussed herein. As aforementioned, the light source 101 may be a swept-source (SS) light source.

Additionally or alternatively, the one or more detectors 107 may be a linear array, a charge-coupled device (CCD), a plurality of photodiodes or some other method of converting the light into an electrical signal. The detector(s) 107 may include an analog to digital converter (ADC). The one or more detectors may be detectors having structure as shown in one or more of FIGS. 1A-4, 7, and 9A-17 and as discussed herein.

In accordance with one or more aspects of the present disclosure, one or more methods for performing imaging are provided herein. FIG. 10 illustrates a flow chart of at least one embodiment of a method for performing imaging. The method(s) may include one or more of the following: (i) splitting or dividing light into a first light and a second reference light (*see* step S4000 in FIG. 10); (ii) receiving reflected or scattered light of the first light after the first light travels along a sample arm and irradiates an object (*see* step S4001 in FIG. 10); (iii) receiving the second reference light after the second reference light travels along a reference arm and reflects off of a reference reflection (*see* step S4002 in FIG. 10); and (iv) generating interference light by causing the reflected or scattered light of the first light and the reflected second reference light to interfere with each other (for example, by combining or recombining and then interfering, by interfering, etc.), the interference light generating one or more interference patterns (*see* step S4003 in FIG. 10). One or more methods may further include using low frequency monitors to update or control high frequency content to improve image quality. For example, one or more embodiments may use multiple imaging modalities, related methods or techniques for same, etc. to achieve improved image quality. In one or more embodiments, an imaging probe may be connected to one or more systems (*e.g*., the system 100a, the system 100b, the system 100, the system 100', the system 100", the devices, apparatuses or systems of FIGS. 1A-4, 7, and 9A-17, any other system or apparatus discussed herein, etc.) with a connection member or interface module. For example, when the connection member or interface module is a rotary junction for an imaging probe, the rotary junction may be at least one of: a contact rotary junction, a lenseless rotary junction, a lens-based rotary junction, or other rotary junction known to those skilled in the art. The rotary junction may be a one channel rotary junction or a two channel rotary junction. The rotary junction may be or include any RJ feature(s) discussed herein, including, but not limited to, the features shown in at least FIG 4. In one or more embodiments, the illumination portion of the imaging probe may be separate from the detection portion of the imaging probe. For example, in one or more applications, a probe may refer to the illumination assembly, which includes an illumination fiber (*e.g*., single mode fiber, a GRIN lens, a spacer and the grating on the polished surface of the spacer, etc.). In one or more embodiments, a scope may refer to the illumination portion which, for example, may be enclosed and protected by a drive cable, a sheath, and detection fibers (*e.g*., multimode fibers (MMFs)) around the sheath. Grating coverage is optional on the detection fibers (e.g., MMFs) for one or more applications. The illumination portion may be connected to a rotary joint and may be rotating continuously at video rate. In one or more embodiments, the detection portion may include one or more of: a detection fiber, a detector (*e.g*., the one or more detectors 107, a spectrometer, etc.), the computer 1200, the computer 1200', the computer 2, any other computer or processor discussed herein, etc. The detection fibers may surround the illumination fiber, and the detection fibers may or may not be covered by a grating, a spacer, a lens, an end of a probe or catheter, etc.

The one or more detectors 107 may transmit the digital or analog signals to a processor or a computer such as, but not limited to, an image processor, a processor or computer 1200, 1200' (*see e.g.,* FIG. 1B and FIGS. 3A-3B, 9A-9C, and 11-13), a computer 2 (*see e.g.,* FIG. 1A), any other processor or computer discussed herein, a combination thereof, etc. The image processor may be a dedicated image processor or a general purpose processor that is configured to process images. In at least one embodiment, the computer 1200, 1200', 2 or any other processor or computer discussed herein may be used in place of, or in addition to, the image processor. In an alternative embodiment, the image processor may include an ADC and receive analog signals from the one or more detectors 107. The image processor may include one or more of a CPU, DSP, FPGA, ASIC, or some other processing circuitry. The image processor may include memory for storing image, data, and instructions. The image processor may generate one or more images based on the information provided by the one or more detectors 107. A computer or processor discussed herein, such as, but not limited to, a processor of the devices, apparatuses, or systems of FIGS. 1A-4, 7, and 9A-17, the computer 1200, the computer 1200', the computer 2, the image processor, and/or any other processor discussed herein or AI-ready network or neural network discussed herein or known to those skilled in the art, may also include one or more components further discussed herein below (*see e.g.,* FIGS. 11-13).

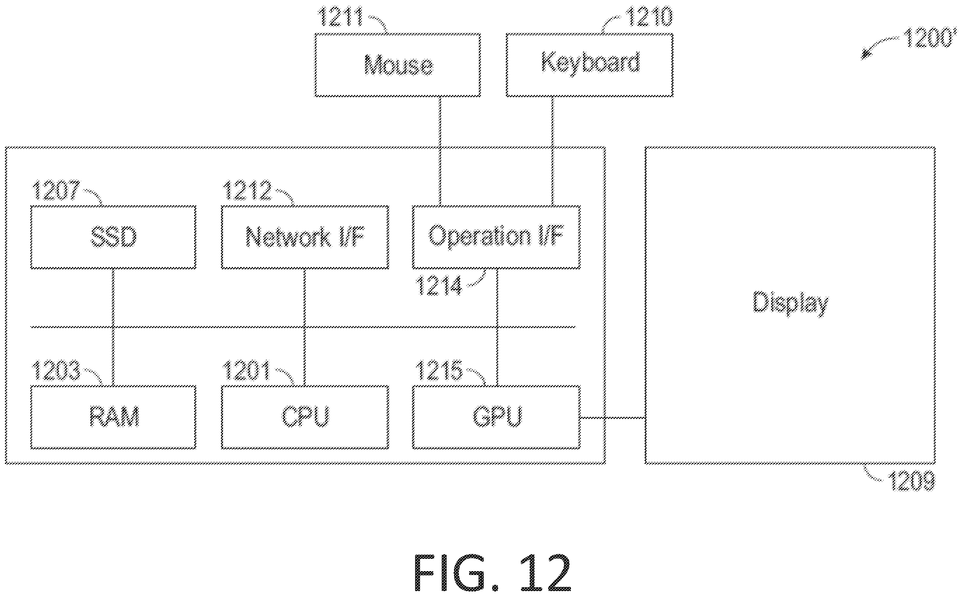

In at least one embodiment, a console or computer 1200, 1200', a computer 2, any other computer or processor discussed herein, etc. operates to control motions of the RJ via the motion control unit (MCU) 112 or a motor M, acquires intensity data from the detector(s) in the one or more detectors 107, and displays the scanned image (*e.g*., on a monitor or screen such as a display, screen or monitor 1209 as shown in the console or computer 1200 of any of FIGS. 1B, 3A-3B, 9A-9C, 11, and 13 and/or the console 1200' of FIGS. 12-13 as further discussed below; the computer 2 of FIG. 1A; any other computer or processor discussed herein; etc.). In one or more embodiments, the MCU 112 or the motor M operates to change a speed of a motor of the RJ and/or of the RJ. The motor may be a stepping or a DC servo motor to control the speed and increase position accuracy (*e.g*., compared to when not using a motor, compared to when not using an automated or controlled speed and/or position change device, compared to a manual control, etc.).

The output of the one or more components of any of the systems discussed herein may be acquired with the at least one detector 107, *e.g.,* such as, but not limited to, photodiodes, Photomultiplier tube(s) (PMTs), line scan camera(s), or multi-array camera(s). Electrical analog signals obtained from the output of the system 100a, 100b, 100, 100', 100", and/or the detector(s) 107 thereof, and/or from the devices, apparatuses, or systems of FIGS. 1A-4, 7, and 9A-17, are converted to digital signals to be analyzed with a computer, such as, but not limited to, the computer 1200, 1200'. In one or more embodiments, the light source 101 may be a radiation source or a broadband light source that radiates in a broad band of wavelengths. In one or more embodiments, a Fourier analyzer including software and electronics may be used to convert the electrical analog signals into an optical spectrum.

Unless otherwise discussed herein, like numerals indicate like elements. For example, while variations or differences exist between the systems, such as, but not limited to, the system 20, the system 100a, the system 100b, the system 100, the system 100', the system 100", or any other device, apparatus or system discussed herein, one or more features thereof may be the same or similar to each other, such as, but not limited to, the light source 101 or other component(s) thereof (e.g., the console 1200, the console 1200', etc.). Those skilled in the art will appreciate that the light source 101, the motor or MCU 112, the RJ, the at least one detector 107, and/or one or more other elements of the system 100 may operate in the same or similar fashion to those like-numbered elements of one or more other systems, such as, but not limited to, the devices, apparatuses or systems of FIGS. 1A-4, 7, and 9A-17, the system 100', the system 100", or any other system discussed herein. Those skilled in the art will appreciate that alternative embodiments of the devices, apparatuses or systems of FIGS. 1A-4, 7, and 9A-17, the system 100', the system 100", any other device, apparatus or system discussed herein, etc., and/or one or more like-numbered elements of one of such systems, while having other variations as discussed herein, may operate in the same or similar fashion to the like-numbered elements of any of the other systems (or components thereof) discussed herein. Indeed, while certain differences exist between the system 100 of FIG. 9A and one or more embodiments shown in any of FIGS. 1A-4, 7, and 9B-17, for example, as discussed herein, there are similarities. Likewise, while the console or computer 1200 may be used in one or more systems (e.g., the system 100a, the system 100b, the system 100, the system 100', the system 100", the devices, apparatuses or systems of any of FIGS. 1A-17, or any other system discussed herein, etc.), one or more other consoles or computers, such as the console or computer 1200', any other computer or processor discussed herein, etc., may be used additionally or alternatively.

One or more embodiments of the present disclosure may include taking multiple views (e.g., OCT image, ring view, tomo view, anatomical view, etc.), and one or more embodiments may highlight or emphasize NIRF and/or NIRAF. In one or more embodiments, two handles may operate as endpoints that may bound the color extremes of the NIRF and/or NIRAF data in or more embodiments. In addition to the standard tomographic view, the user may select to display multiple longitudinal views. When connected to an angiography system, the Graphical User Interface (GUI) may also display angiography images.

In accordance with one or more aspects of the present disclosure, the aforementioned features are not limited to being displayed or controlled using any particular GUI. In general, the aforementioned imaging modalities may be used in various ways, including with or without one or more features of aforementioned embodiments of a GUI or GUIs. For example, a GUI may show an OCT image with a tool or marker to change the image view as aforementioned even if not presented with a GUI (or with one or more other components of a GUI; in one or more embodiments, the display may be simplified for a user to display set or desired information).

The procedure to select the region of interest and the position of a marker, an angle, a plane, etc., for example, using a touch screen, a GUI (or one or more components of a GUI; in one or more embodiments, the display may be simplified for a user to display the set or desired information), a processor (*e.g*., processor or computer 2, 1200, 1200', or any other processor discussed herein) may involve, in one or more embodiments, a single press with a finger and dragging on the area to make the selection or modification. The new orientation and updates to the view may be calculated upon release of a finger, or a pointer. In one or more embodiments, a region of interest and/or a position of the marker may be set or selected automatically using AI features and/or processing features of the present disclosure.

For one or more embodiments using a touch screen, two simultaneous touch points may be used to make a selection or modification, and may update the view based on calculations upon release.

One or more functions may be controlled with one of the imaging modalities, such as the angiography image view or the intravascular image view (*e.g*., the OCT image view, the IVUS image view, another intravascular imaging modality image view, etc.), to centralize user attention, maintain focus, and allow the user to see all relevant information in a single moment in time.

In one or more embodiments, one imaging modality may be displayed or multiple imaging modalities may be displayed.

One or more procedures may be used in one or more embodiments to select a region of choice or a region of interest for a view. For example, after a single touch is made on a selected area (e.g., by using a touch screen, by using a mouse or other input device to make a selection, etc.), the semi-circle (or other geometric shape used for the designated area) may automatically adjust to the selected region of choice or interest. Two (2) single touch points may operate to connect/draw the region of choice or interest. For example, a user may desire to view photo-bleached portion(s) or component(s) of the optical probe 124 and/or of the catheter 120, and/or may desire to view the object, sample, or target 106.

There are many ways to compute intensity, viscosity, resolution (including increasing resolution of one or more images), etc., to use one or more imaging modalities, to construct or reconstruct images or structure(s), to detect tissue and/or characterize tissue, to perform photo-bleaching, and/or related methods for same, discussed herein, digital as well as analog. In at least one embodiment, a computer, such as the console or computer 1200, 1200', may be dedicated to control and monitor the imaging (e.g., OCT, single mode OCT, multimodal OCT, multiple imaging modalities, IVUS imaging modality, another intravascular imaging modality discussed herein or known to those skilled in the art, etc.) devices, systems, methods and/or storage mediums described herein.

The electric signals used for imaging may be sent to one or more processors, such as, but not limited to, a computer or processor 2 (*see e.g.,* FIG. 1A), a computer 1200 (*see e.g.,* FIGS. 3A-3B, 9A-9C, 11, and 13), a computer 1200' (*see e.g.,* FIGS. 3A, 12, and 13), etc. as discussed further below, via cable(s) or wire(s), such as, but not limited to, the cable(s) or wire(s) *113 (see* FIG. 11). Additionally or alternatively, the electric signals, as aforementioned, may be processed in one or more embodiments as discussed above by any other computer or processor or components thereof. The computer or processor 2 as shown in FIG. 1A may be used instead of any other computer or processor discussed herein (e.g., computer or processors 1200, 1200', etc.), and/or the computer or processor 1200, 1200' may be used instead of any other computer or processor discussed herein (*e.g*., computer or processor 2). In other words, the computers or processors discussed herein are interchangeable, and may operate to perform any of the multiple imaging modalities feature(s) and method(s) discussed herein, including using, controlling, and changing a GUI or multiple GUI's and/or performing tissue characterization, tissue detection, photo-bleaching, and coregistration.

Various components of a computer system 1200 are provided in FIG. 11. A computer system 1200 may include a central processing unit ("CPU") 1201, a ROM 1202, a RAM 1203, a communication interface 1205, a hard disk (and/or other storage device) 1204, a screen (or monitor interface) 1209, a keyboard (or input interface; may also include a mouse or other input device in addition to the keyboard) 1210 and a BUS (or "Bus") or other connection lines *(e.g.,* connection line 1213) between one or more of the aforementioned components (*e.g.,* including but not limited to, being connected to the console, the probe, the imaging apparatus or system, any motor discussed herein, a light source, etc.). In addition, the computer system 1200 may comprise one or more of the aforementioned components. For example, a computer system 1200 may include a CPU 1201, a RAM 1203, an input/output (I/O) interface (such as the communication interface 1205) and a bus (which may include one or more lines 1213 as a communication system between components of the computer system 1200; in one or more embodiments, the computer system 1200 and at least the CPU 1201 thereof may communicate with the one or more aforementioned components of a device or system, such as, but not limited to, an apparatus or system using one or more imaging modalities and related method(s) as discussed herein), and one or more other computer systems 1200 may include one or more combinations of the other aforementioned components (e.g., the one or more lines 1213 of the computer 1200 may connect to other components via line 113). The CPU 1201 is configured to read and perform computer-executable instructions stored in a storage medium. The computer-executable instructions may include those for the performance of the methods and/or calculations described herein. The system 1200 may include one or more additional processors in addition to CPU 1201, and such processors, including the CPU 1201, may be used for tissue or object characterization, diagnosis, evaluation, imaging, construction or reconstruction, photo-bleaching, and/or coregistration. The system 1200 may further include one or more processors connected via a network connection (*e.g*., via network 1206). The CPU 1201 and any additional processor being used by the system 1200 may be located in the same telecom network or in different telecom networks (*e.g.*, performing feature(s), function(s), technique(s), method(s), etc. discussed herein may be controlled remotely).

The I/O or communication interface 1205 provides communication interfaces to input and output devices, which may include a light source, a spectrometer, a microphone, a communication cable and a network (either wired or wireless), a keyboard 1210, a mouse (*see e.g.,* the mouse 1211 as shown in FIG. 12), a touch screen or screen 1209, a light pen and so on. The communication interface of the computer 1200 may connect to other components discussed herein via line 113 (as diagrammatically shown in FIG. 11). The Monitor interface or screen 1209 provides communication interfaces thereto.

Any methods and/or data of the present disclosure, such as the methods for performing tissue or object characterization, diagnosis, examination, imaging (including, but not limited to, increasing image resolution, performing imaging using one or more imaging modalities, viewing or changing one or more imaging modalities and related methods (and/or option(s) or feature(s)), etc.), tissue detection, photo-bleaching, and/or coregistration (using AI feature(s) with one or more of same), for example, as discussed herein, may be stored on a computer-readable storage medium. A computer-readable and/or writable storage medium used commonly, such as, but not limited to, one or more of a hard disk (*e.g*., the hard disk 1204, a magnetic disk, etc.), a flash memory, a CD, an optical disc *(e.g.,* a compact disc ("CD") a digital versatile disc ("DVD"), a Blu-ray^{™} disc, etc.), a magneto-optical disk, a random-access memory ("RAM") (such as the RAM 1203), a DRAM, a read only memory ("ROM"), a storage of distributed computing systems, a memory card, or the like (*e.g*., other semiconductor memory, such as, but not limited to, a non-volatile memory card, a solid state drive (SSD) (*see* SSD 1207 in FIG. 12), SRAM, etc.), an optional combination thereof, a server/database, etc. may be used to cause a processor, such as, the processor or CPU 1201 of the aforementioned computer system 1200 to perform the steps of the methods disclosed herein. The computer-readable storage medium may be a non-transitory computer-readable medium, and/or the computer-readable medium may comprise all computer-readable media, with the sole exception being a transitory, propagating signal in one or more embodiments. The computer-readable storage medium may include media that store information for predetermined, limited, or short period(s) of time and/or only in the presence of power, such as, but not limited to Random Access Memory (RAM), register memory, processor cache(s), etc. Embodiment(s) of the present disclosure may also be realized by a computer of a system or apparatus that reads out and executes computer executable instructions (e.g., one or more programs) recorded on a storage medium (which may also be referred to more fully as a "non-transitory computer-readable storage medium") to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s).

In accordance with at least one aspect of the present disclosure, the methods, systems, and computer-readable storage mediums related to the processors, such as, but not limited to, the processor of the aforementioned computer 1200, etc., as described above may be achieved utilizing suitable hardware, such as that illustrated in the figures. Functionality of one or more aspects of the present disclosure may be achieved utilizing suitable hardware, such as that illustrated in FIG. 11. Such hardware may be implemented utilizing any of the known technologies, such as standard digital circuitry, any of the known processors that are operable to execute software and/or firmware programs, one or more programmable digital devices or systems, such as programmable read only memories (PROMs), programmable array logic devices (PALs), etc. The CPU 1201 (as shown in FIG. 11), the processor or computer 2 (as shown in FIG. 1A) and/or the computer or processor 1200' (as shown in FIG. 12) may also include and/or be made of one or more microprocessors, nanoprocessors, one or more graphics processing units ("GPUs"; also called a visual processing unit ("VPU")), one or more Field Programmable Gate Arrays ("FPGAs"), or other types of processing components (e.g., application specific integrated circuit(s) (ASIC)). Still further, the various aspects of the present disclosure may be implemented by way of software and/or firmware program(s) that may be stored on suitable storage medium (e.g., computer-readable storage medium, hard drive, etc.) or media (such as floppy disk(s), memory chip(s), etc.) for transportability and/or distribution. The computer may include a network of separate computers or separate processors to read out and execute the computer executable instructions. The computer executable instructions may be provided to the computer, for example, from a network or the storage medium. The computers or processors (e.g., 2, 1200, 1200', etc.) may include the aforementioned CPU structure, or may be connected to such CPU structure for communication therewith.

As aforementioned, hardware structure of an alternative embodiment of a computer or console 1200' is shown in FIG. 12 (*see also,* FIG. 13). The computer 1200' includes a central processing unit (CPU) 1201, a graphical processing unit (GPU) 1215, a random access memory (RAM) 1203, a network interface device 1212, an operation interface 1214 such as a universal serial bus (USB) and a memory such as a hard disk drive or a solid state drive (SSD) 1207. The computer or console 1200' may include a display 1209. The computer 1200' may connect with a motor, a console, or any other component of the device(s) or system(s) discussed herein via the operation interface 1214 or the network interface 1212 *(e.g.,* via a cable or fiber, such as the cable or fiber 113 as similarly shown in FIG. 11). A computer, such as the computer 1200', may include a motor or motion control unit (MCU) in one or more embodiments. The operation interface 1214 is connected with an operation unit such as a mouse device 1211, a keyboard 1210 or a touch panel device. The computer 1200' may include two or more of each component.

At least one computer program is stored in the SSD 1207, and the CPU 1201 loads the at least one program onto the RAM 1203, and executes the instructions in the at least one program to perform one or more processes described herein, as well as the basic input, output, calculation, memory writing and memory reading processes.

The computer, such as the computer 2, the computer 1200, 1200', (or other component(s) such as, but not limited to, the CPU, etc.), etc. may communicate with a motion control unit (MCU), an interferometer, a spectrometer, a detector, etc. to perform imaging, and may reconstruct an image from the acquired intensity data. The monitor or display 1209 displays the reconstructed image, and may display other information about the imaging condition or about an object to be imaged. The monitor 1209 also provides a graphical user interface for a user to operate any system discussed herein. An operation signal is input from the operation unit *(e.g.,* such as, but not limited to, a mouse device 1211, a keyboard 1210, a touch panel device, etc.) into the operation interface 1214 in the computer 1200', and corresponding to the operation signal the computer 1200' instructs any system discussed herein to set or change the imaging condition (*e.g*., improving resolution of an image or images), and to start or end the imaging. A light or laser source and a spectrometer and/or detector may have interfaces to communicate with the computers 1200, 1200' to send and receive the status information and the control signals.

As shown in FIG. 13, one or more processors or computers 1200, 1200' (or any other processor discussed herein) may be part of a system in which the one or more processors or computers 1200, 1200' (or any other processor discussed herein) communicate with other devices (*e.g*., a database 1603, a memory 1602 (which may be used with or replaced by any other type of memory discussed herein or known to those skilled in the art), an input device 1600, an output device 1601, etc.). In one or more embodiments, one or more models may have been trained previously and stored in one or more locations, such as, but not limited to, the memory 1602, the database 1603, etc. In one or more embodiments, it is possible that one or more models and/or data discussed herein (*e.g.*, training data, testing data, validation data, imaging data, etc.) may be input or loaded via a device, such as the input device 1600. In one or more embodiments, a user may employ an input device 1600 (which may be a separate computer or processor, a keyboard such as the keyboard 1210, a mouse such as the mouse 1211, a microphone, a screen or display 1209 (*e.g.,* a touch screen or display), or any other input device known to those skilled in the art). In one or more system embodiments, an input device 1600 may not be used (*e.g*., where user interaction is eliminated by one or more artificial intelligence features discussed herein). In one or more system embodiments, the output device 1601 may receive one or more outputs discussed herein to perform the marker detection, the coregistration, the photo-bleaching, and/or any other process discussed herein. In one or more system embodiments, the database 1603 and/or the memory 1602 may have outputted information (*e.g*., trained model(s), detected marker information, image data, test data, validation data, training data, coregistration result(s), photo-bleaching result(s), segmentation model information, object detection/regression model information, combination model information, etc.) stored therein. That said, one or more embodiments may include several types of data stores, memory, storage media, etc. as discussed above, and such storage media, memory, data stores, etc. may be stored locally or remotely.

Additionally, unless otherwise specified, the term "subset" of a corresponding set does not necessarily represent a proper subset and may be equal to the corresponding set.

While one or more embodiments of the present disclosure include various details regarding a neural network model architecture and optimization approach, in one or more embodiments, any other model architecture, machine learning algorithm, or optimization approach may be employed. One or more embodiments may utilize hyper-parameter combination(s). One or more embodiments may employ data capture, selection, annotation as well as model evaluation (*e.g*., computation of loss and validation metrics) since data may be domain and application specific. In one or more embodiments, the model architecture may be modified and optimized to address a variety of computer visions issues (discussed below).

One or more embodiments of the present disclosure may automatically detect (predict a spatial location of) a radiodense OCT marker in a time series of X-ray images to co-register the X-ray images with the corresponding OCT images (at least one example of a reference point of two different coordinate systems). One or more embodiments may use deep (recurrent) convolutional neural network(s), which may improve marker detection, tissue detection, tissue characterization, photo-bleaching characterization/detection/performance, and image co-registration significantly. One or more embodiments may employ segmentation and/or object/keypoint detection architectures to solve one or more computer vision issues in other domain areas in one or more applications. One or more embodiments employ several novel materials and methods to solve one or more computer vision or other issues (*e.g*., radiodense OCT marker detection in time series of X-ray images, for instance; tissue detection; tissue characterization; photo-bleaching; etc.).

One or more embodiments employ data capture and selection. In one or more embodiments, the data is what makes such an application unique and distinguishes this application from other applications. For example, images may include a radiodense marker that is specifically used in one or more procedures (*e.g*., added to the OCT capsule, used in catheters/probes with a similar marker to that of an OCT marker, used in catheters/probes with a similar or same marker even in a case where the catheters/probes use an imaging modality different from OCT, etc.) to facilitate computational detection of a marker and/or tissue detection, characterization, validation, photo-bleaching, etc. in one or more images (*e.g*., X-ray images). One or more embodiments may couple a software device or features (model) to hardware (*e.g*., an OCT probe, a probe/catheter using an imaging modality different from OCT while using a marker that is the same as or similar to the marker of an OCT probe/catheter, etc.). One or more embodiments may utilize animal data in addition to patient data. Training deep learning may use a large amount of data, which may be difficult to obtain from clinical studies. Inclusion of image data from pre-clinical studies in animals into a training set may improve model performance. Training and evaluation of a model may be highly data dependent (*e.g.*, a way in which frames are selected (*e.g*., pullback only), split into training/validation/test sets, and grouped into batches as well as the order in which the frames, sets, and/or batches are presented to the model, any other data discussed herein, etc.). In one or more embodiments, such parameters may be more important or significant than some of the model hyper-parameters (*e.g.*, batch size, number of convolution layers, any other hyper-parameter discussed herein, etc.). One or more embodiments may use a collection or collections of user annotations after introduction of a device/apparatus, system, and/or method(s) into a market, and may use post market surveillance, retraining of a model or models with new data collected (*e.g*., in clinical use), and/or a continuously adaptive algorithm/method(s).

One or more embodiments may employ data annotation. For example, one or more embodiments may label pixel(s) representing a marker or a tissue detection, characterization, and/or validation as well as pixels representing a blood vessel(s) and/or photo-bleaching at different phase(s) of a procedure/method (*e.g*., different levels of contrast due to intravascular contrast agent) of frame(s) acquired during pullback.

One or more embodiments may employ incorporation of prior knowledge. For example, in one or more embodiments, a marker location may be known inside a vessel and/or inside a catheter or probe; a tissue location may be known inside a vessel or other type of target, object, or specimen; photo-bleached portion(s) and/or component(s) of the optical probe 124 and/or the catheter 120 may be known, etc. As such, simultaneous localization of the vessel and marker may be used to improve marker detection and/or tissue and/or photo-bleaching detection, characterization, and/or validation. For example, in a case where it is confirmed that the marker of the probe or catheter, or the catheter or probe, is by or near a target area for tissue detection and characterization, the integrity of the tissue identification/detection and/or characterization (e.g., photo-bleach detection) for that target area is improved or maximized (as compared to a false positive where a tissue may be detected in an area where the probe or catheter (or marker thereof) is not located). In one or more embodiments, a marker may move during a pullback inside a vessel, and such prior knowledge may be incorporated into the machine learning algorithm or the loss function.

One or more embodiments employ loss (cost) and evaluation function(s)/metric(s). For example, use of temporal information for model training and evaluation may be used in one or more embodiments. One or more embodiments may evaluate a distance between prediction and ground truth per frame as well as consider a trajectory of predictions across multiple frames of a time series. For example, the photo-bleaching process(es) of the portion(s) or component(s) of the optical probe 124 and/or of the catheter 120 may be evaluated over time using a distance between prediction and ground truth per frame.

### Application of machine learning

Application of machine learning may be used in one or more embodiment(s), as discussed in PCT/US2020/051615, filed on September 18, 2020 and published as WO 2021/055837 A9 on March 25, 2021, and as discussed in U.S. Pat. Pub. No. 2022/0346885 A1, published on November 3, 2022, the applications and publications of which are incorporated by reference herein in their entireties. For example, at least one embodiment of an overall process of machine learning is shown below:
i. Create a dataset that contains both images and corresponding ground truth labels;
ii. Split the dataset into a training set and a testing set;
iii. Select a model architecture and other hyper-parameters;
iv. Train the model with the training set;
v. Evaluate the trained model with the validation set; and
vi. Repeat iv and v with new dataset(s).

Based on the testing results, steps i and iii may be revisited in one or more embodiments.

One or more models may be used in one or more embodiment(s) to detect and/or characterize a tissue or tissues and/or to detect and/or characterize photo-bleaching, such as, but not limited to, the one or more models as discussed in PCT/US2020/051615, filed on September 18, 2020 and published as WO 2021/055837 A9 on March 25, 2021, and as discussed in U.S. Pat. Pub. No. 2022/0346885 A1, published on November 3, 2022, the applications and publications of which are incorporated by reference herein in their entireties. For example, one or more embodiments may use a segmentation model, a regression model, a combination thereof, etc.

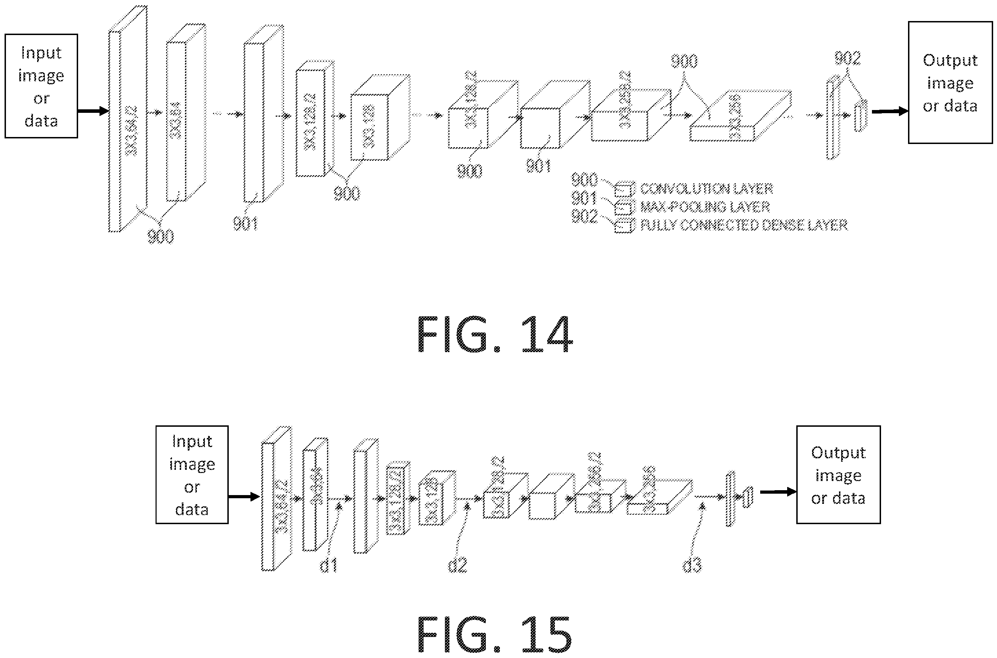

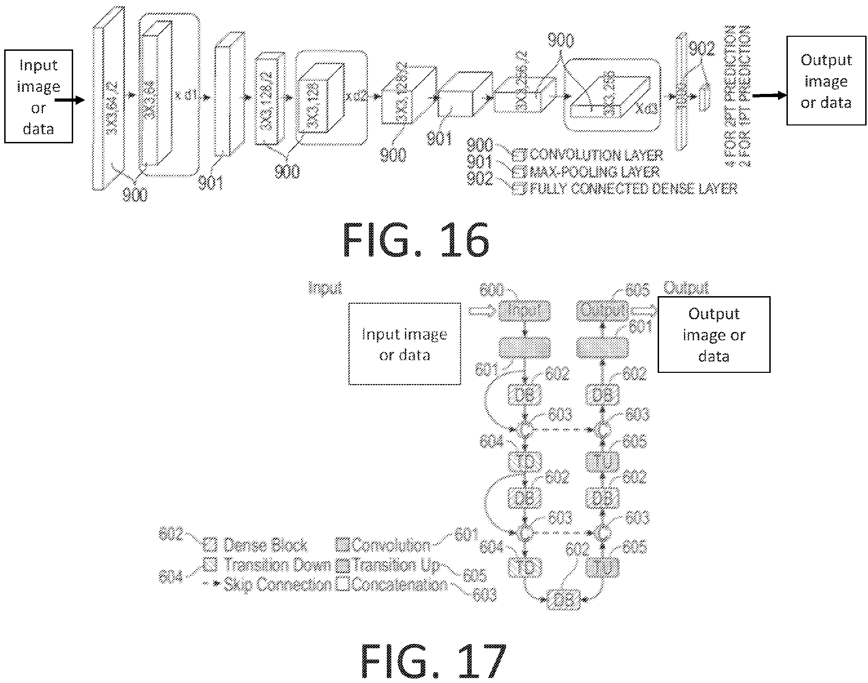

For regression model(s), the input may be the entire image frame or frames, and the output may be the centroid coordinates of radiopaque markers (target marker and stationary marker, if necessary/desired) and/or coordinates of a portion of a catheter or probe to be used in determining the tissue detection and/or characterization and/or used in determining photo-bleaching portion(s) or component(s) of the optical probe 124 and/or of the catheter 120. Additionally or alternatively, in one or more embodiments, input may comprise or include an entire image frame or frames (e.g., the aforementioned constructed CVI image or frame), and the output may be data regarding high textured areas formed due to the presence of sharp edges in an A-line or A-lines representing calcium in intravascular images as well as dark homogeneous areas representing lipids in the input image frame or frames (e.g., in the CVI image or frame). As shown diagrammatically in FIGS. 14-16, an example of an input image on the left side of FIGS. 14-16 and a corresponding output image on the right side of FIGS. 14-16 are illustrated for regression model(s). At least one architecture of a regression model is shown in FIG. 14. In at least the embodiment of FIG. 14, the regression model may use a combination of one or more convolution layers 900, one or more max-pooling layers 901, and one or more fully connected dense layers 902. While not limited to the Kernel size, Width/Number of filters (output size), and Stride sizes shown for each layer (e.g., in the left convolution layer of FIG. 14, the Kernel size is "3x3", the Width/# of filters (output size) is "64", and the Stride size is "2"). In one or more embodiments, another hyper-parameter search with a fixed optimizer and with a different width may be performed, and at least one embodiment example of a model architecture for a convolutional neural network for this scenario is shown in FIG. 15. One or more embodiments may use one or more features for a regression model as discussed in "Deep Residual Learning for Image Recognition" to Kaiming He, et al., Microsoft Research, December 10, 2015 (https://arxiv.org/pdf/1512.03385.pdf), which is incorporated by reference herein in its entirety. FIG. 16 shows at least a further embodiment example of a created architecture of or for a regression model(s).

Since the output from a segmentation model, in one or more embodiments, is a "probability" of each pixel that may be categorized as a tissue or photo-bleach characterization or tissue or photo-bleach identification/determination, post-processing after prediction via the trained segmentation model may be developed to better define, determine, or locate the final coordinate of tissue or photo-bleaching location (or a marker location where the marker is a part of the catheter) and/or determine the type and/or characteristics of the tissue or tissues or of the photo-bleaching. One or more embodiments of a semantic segmentation model may be performed using the One-Hundred Layers Tiramisu method discussed in "The One Hundred Layers Tiramisu: Fully Convolutional DenseNets for Semantic Segmentation" to Simon Jégou, et al., Montreal Institute for Learning Algorithms, published October 31, 2017 (https://arxiv.org/pdf/1611.09326.pdf), which is incorporated by reference herein in its entirety. A segmentation model may be used in one or more embodiment, for example, as shown in FIG. 17. At least one embodiment may utilize an input 600 as shown to obtain an output 605 of at least one embodiment of a segmentation model method. For example, by applying the One-Hundred Layers Tiramisu method(s), one or more features, such as, but not limited to, convolution 601, concatenation 603, transition up 605, transition down 604, dense block 602, etc., may be employed by slicing the training data set. While not limited to only or by only these embodiment examples, in one or more embodiments, a slicing size may be one or more of the following: 100 x 100, 224 x 224, 512 x 512, and, in one or more of the experiments performed, a slicing size of 224 x 224 performed the best. A batch size (of images in a batch) may be one or more of the following: 2, 4, 8, 16, and, from the one or more experiments performed, a bigger batch size typically performs better (*e.g*., with greater accuracy). In one or more embodiments, 16 images/batch may be used. The optimization of all of these hyper-parameters depends on the size of the available data set as well as the available computer/computing resources; thus, once more data is available, different hyper-parameter values may be chosen. Additionally, in one or more embodiments, steps/epoch may be 100, and the epochs may be greater than (>) 1000. In one or more embodiments, a convolutional autoencoder (CAE) may be used.

In one or more embodiments, hyper-parameters may include, but are not limited to, one or more of the following: Depth (*i.e.*, # of layers), Width (*i.e*., # of filters), Batch size (*i.e*., # of training images/step): May be >4 in one or more embodiments, Learning rate (*i.e*., a hyper-parameter that controls how fast the weights of a neural network (the coefficients of regression model) are adjusted with respect the loss gradient), Dropout (*i.e.*, % of neurons (filters) that are dropped at each layer), and/or Optimizer: for example, Adam optimizer or Stochastic gradient descent (SGD) optimizer. In one or more embodiments, other hyper-parameters may be fixed or constant values, such as, but not limited to, for example, one or more of the following: Input size *(e.g.,* 1024 pixel x 1024 pixel, 512 pixel x 512 pixel, another preset or predetermined number or value set, etc.), Epochs: 100, 200, 300, 400, 500, another preset or predetermined number, etc. (for additional training, iteration may be set as 3000 or higher), and/or Number of models trained with different hyper-parameter configurations (*e.g*., 10, 20, another preset or predetermined number, etc.).

One or more features discussed herein may be determined using a convolutional auto-encoder, Gaussian filters, Haralick features, and/or thickness or shape of the sample or object (*e.g*., the tissue or tissues, a specimen, a patient, a target in the patient, photo-bleached portion(s) or component(s) of the optical probe 124 and/or of the catheter 120, etc.).

One or more embodiments of the present disclosure may use machine learning to determine marker, tissue, or photo-bleach location; to determine, detect, or evaluate tissue type(s) and/or characteristic(s); to determine, detect, evaluate, or perform photobleaching or photo-bleach characteristic(s); to perform coregistration; and/or to perform any other feature discussed herein. Machine learning (ML) is a field of computer science that gives processors the ability to learn, via artificial intelligence. Machine learning may involve one or more algorithms that allow processors or computers to learn from examples and to make predictions for new unseen data points. In one or more embodiments, such one or more algorithms may be stored as software or one or more programs in at least one memory or storage medium, and the software or one or more programs allow a processor or computer to carry out operation(s) of the processes described in the present disclosure.

Similarly, the present disclosure and/or one or more components of devices, systems, and storage mediums, and/or methods, thereof also may be used in conjunction with optical coherence tomography probes. Such probes include, but are not limited to, the OCT imaging systems disclosed in U.S. Pat. Nos. 6,763,261; 7,366,376; 7,843,572; 7,872,759; 8,289,522; 8,676,013; 8,928,889; 9,087,368; 9,557,154; 10,912,462; 9,795,301; 9,332,942; and 10,939,825 to Tearney et al., and U.S. Pat. Pub. No. 2014/0276011; and WO 2016/015052 to Tearney et al., and arrangements and methods of facilitating photoluminescence imaging, such as those disclosed in U.S. Pat. No. 7,889,348 to Tearney et al., as well as the disclosures directed to multimodality imaging disclosed in U.S. Pat. 9,332,942; 9,557,154; 9,795,301; 10,578,422; 11,473,896; and 11,123,047 and U.S. Patent Publication Nos. 2010/0092389 and 2022/0241437 and WO 2016/144878, each of which patents and patent publications are incorporated by reference herein in their entireties. As aforementioned, any feature or aspect of the present disclosure may be used with OCT imaging systems, apparatuses, methods, storage mediums or other aspects or features as discussed in U.S. Pat. No. 11,382,516, the entire disclosure of which is incorporated by reference herein in its entirety.

The present disclosure and/or one or more components of devices, systems, and storage mediums, and/or methods, thereof also may be used with OCT imaging systems and/or catheters and catheter systems, such as, but not limited to, those in U.S. Pat. Nos. 9,869,828; 10,323,926; 10,558,001; 10,601,173; 10,606,064; 10,743,749; 10,884,199; 10,895,692; 11,175,126; 11,375,881; 11,406,327; and 11,796,741 as well as U.S. Patent Publication Nos. 2019/0254506; 2020/0390323; 2021/0121132; 2021/0149122; 2021/0174125; 2022/0040454; 2022/0043217; 2022/0044428; and 2023/0123224, and WO2021/055837, each of which patents and patent publications are incorporated by reference herein in their entireties.

Further, the present disclosure and/or one or more components of devices, systems, and storage mediums, and/or methods, thereof also may be used in conjunction with continuum robotic systems and catheters, such as, but not limited to, those described in U.S. Patent Publication Nos. 2019/0105468; 2021/0369085; 2020/0375682; 2021/0121162; 2021/0121051; and 2022/0040450, each of which patents and/or patent publications are incorporated by reference herein in their entireties.

Although the disclosure herein has been described with reference to particular embodiments, it is to be understood that these embodiments are merely illustrative of the principles and applications of the present disclosure (and are not limited thereto). It is therefore to be understood that numerous modifications may be made to the illustrative embodiments and that other arrangements may be devised without departing from the spirit and scope of the present disclosure. The scope of the following claims is to be accorded the broadest interpretation so as to encompass all such modifications and equivalent structures and functions.

## Claims

1. A photo-bleached imaging apparatus comprising:
a catheter having an optical probe having one or more optical fibers that operate to deliver and receive light, wherein the optical probe or one or more components of the optical probe is/are photo-bleached.

2. The imaging apparatus of claim 1, wherein one or more of the following:
(i) an emission intensity of the photo-bleached one or more components of the optical probe stabilizes within 10% or about 10% of an averaged intensity over a predetermined or set period of time;
(ii) an emission intensity of the photo-bleached one or more components of the optical probe stabilize within 10% or about 10% of an averaged intensity over a predetermined or set period of time and/or such that the optical probe has a higher signal to noise ratio as compared to an optical probe or catheter having an optical probe without being photo-bleached;
(iii) an emission intensity of the photo-bleached optical probe and/or of the photo-bleached one or more components of the optical probe stabilizes within 10% or about 10% of an averaged intensity over a predetermined or set period of time, and the predetermined or set period of time is one of the following: two minutes, about two minutes, a period of time in a range of one minute to two minutes, and/or a period of time in a range of about one minute to about two minutes; and/or
(iv) the optical probe includes a double clad fiber.

3. The imaging apparatus of claim 1, further comprising one or more processors that operate to perform a pullback of the optical probe within a catheter and/or obtain one or more images or frames of one or more imaging modalities from the pullback of the optical probe within the catheter.

4. The imaging apparatus of claim 3, wherein one or more of the following:
(i) the imaging apparatus further includes an interference optical system that operates to: (a) receive and divide light from a light source into a first light with which an object or sample is to be irradiated and which travels along a sample arm of the interference optical system and a second reference light, (b) send the second reference light along a reference arm of the interference optical system for reflection off of a reference reflection of the interference optical system, and (c) generate interference light by causing reflected or scattered light of the first light with which the object or sample has been irradiated and the reflected second reference light to combine or recombine, and to interfere, with each other, the interference light generating one or more interference patterns; and one or more detectors that operate to continuously acquire the interference light and/or the one or more interference patterns to measure the interference or the one or more interference patterns between the combined or recombined light to obtain data for the one or more imaging modalities;
(ii) the imaging apparatus further includes an interference optical system that operates to: (a) receive and divide light from a light source into a first light with which an object or sample is to be irradiated and which travels along a sample arm of the interference optical system and a second reference light, (b) send the second reference light along a reference arm of the interference optical system for reflection off of a reference reflection of the interference optical system, and (c) generate interference light by causing reflected or scattered light of the first light with which the object or sample has been irradiated and the reflected second reference light to combine or recombine, and to interfere, with each other, the interference light generating one or more interference patterns; and one or more detectors that operate to continuously acquire the interference light and/or the one or more interference patterns to measure the interference or the one or more interference patterns between the combined or recombined light to obtain data for the one or more imaging modalities, wherein a wavelength of the first light is shorter than a wavelength of the reflected or scattered light and/or the generated interference light; and/or
(iii) the one or more imaging modalities include one or more of the following: Optical Coherence Tomography (OCT), single modality OCT, multi-modality OCT, swept source OCT, optical frequency domain imaging (OFDI), intravascular ultrasound (IVUS), another lumen image(s) modality, near-infrared spectroscopy (NIRS), near-infrared fluorescence (NIRF), near-infrared auto-fluorescence (NIRAF), near-infrared, fluorescence, and/or an intravascular imaging modality.

5. The imaging apparatus of claim 3, wherein the one or more processors further operate to display the one or more images or frames on a display, store the one or more images or frames in a memory, or use the one or more images or frames to train one or more models or AI-networks to auto-detect or to perform photo-bleaching and/or to automatically obtain one or more images or frames of the one or more imaging modalities; and wherein one or more of the following:
(i) the trained model is one or a combination of the following: a neural net model or neural network model, a deep convolutional neural network model, a recurrent neural network model with long short-term memory that can take temporal relationships across images or frames into account, a generative adversarial network (GAN) model, a consistent generative adversarial network (cGAN) model, a three cycle-consistent generative adversarial network (3cGAN) model, a model that can take temporal relationships across images or frames into account, a model that can take temporal relationships into account including tissue location(s) and/or photo-bleach location(s) during pullback in a vessel and/or including tissue and/or photo-bleach characterization data during pullback in a vessel, a model that can use prior knowledge about a procedure and incorporate the prior knowledge into the machine learning algorithm or a loss function, a model using feature pyramid(s) that can take different image resolutions into account, and/or a model using residual learning technique(s), a segmentation model, a segmentation model with post-processing, a model with pre-processing, a model with post-processing, a segmentation model with pre-processing, a deep learning or machine learning model, a semantic segmentation model or classification model, an object detection or regression model, an object detection or regression model with pre-processing or post-processing, a combination of a semantic segmentation model and an object detection or regression model, a model using repeated segmentation model technique(s), a model using feature pyramid(s), a genetic algorithm that operates to breed multiple models for improved performance, and/or a model using repeated object detection or regression model technique(s); and/or
(ii) the one or more processors further operate to use one or more neural networks or convolutional neural networks to one or more of: load a trained model of images including photo-bleached area(s); perform photo-bleaching on the optical probe and/or the catheter; determine whether the photo-bleached area(s) is/are accurate or correct; determine one or more of the characteristics of one or more objects, targets, or samples in the one or more images or frames; identify or detect the one or more objects, targets, or samples; overlay data on at least one of the one or more images or frames to show location(s) of intravascular image(s), the photo-bleached area(s), or the objects, targets, or samples; incorporate image processing and machine learning (ML) or deep learning to automatically identify and locate photo-bleached portions or components of the optical probe or the catheter; incorporate image processing and machine learning (ML) or deep learning to automatically identify and locate the one or more objects, targets, or samples; display the results for the photo-bleach identification/detection or characterization on a display; and/or acquire or receive image data during the pullback operation of the optical probe within the catheter.

6. The imaging apparatus of claim 1, further comprising one or more of the following:
(i) a light source that operates to produce the light;
(ii) a light source that operates to produce the light, the light source producing the light to operate as an excitation laser or light having a wavelength of 400 nm - 900 nm or 635 nm;
(iii) a light source that operates to produce the light, the light source producing the light as an excitation laser or light and coupling the excitation laser or light into the optical probe and/or one or more components of the optical probe;
(iv) a light source that operates to produce the light, the light source producing the light as an excitation laser or light and exciting the optical probe and/or one or more components of the optical probe with the excitation laser or light for more than or equal to a set or predetermined amount of time; and/or
(v) a light source that operates to produce the light, the light source producing the light as an excitation laser or light and exciting the optical probe and/or one or more components of the optical probe with the excitation laser or light for more than or equal to a set or predetermined amount of time, where the set or predetermined amount of time is one or more of the following: thirty (30) minutes, thirty (30) minutes or more, in a range of thirty (30) minutes to twenty-four (24) hours, twenty-four (24) hours, twenty-four (24) hours or more, an amount of time calculated or set/received by one or more processors of the imaging apparatus or by a user of the imaging apparatus, and/or an amount of time calculated or set by the one or more processors of the imaging apparatus or by the user of the imaging apparatus based on a size and shape to be photo-bleached or based a number of components or structure to be photo-bleached.

7. The imaging apparatus of claim 1, wherein, in a case where the optical probe or the one or more components of the optical probe include or are attached to a double clad fiber, one or more of the following exists:
(i) the imaging apparatus further comprises one or more processors that operate to perform a pullback of the optical probe within a catheter and/or obtain one or more images or frames of one or more imaging modalities from the pullback of the optical probe;
(ii) the imaging apparatus further comprises: one or more processors that operate to perform a pullback of the optical probe within a catheter and/or obtain one or more images or frames of one or more imaging modalities from the pullback of the optical probe, and the one or more processors further include or operate to be used with a core/clad ratio adjustment processor or unit that operates to control a ratio of excitation laser or light between a core and a clad of the double clad fiber;
(iii) the imaging apparatus further comprises: one or more processors that operate to perform a pullback of the optical probe within a catheter and/or obtain one or more images or frames of one or more imaging modalities from the pullback of the optical probe, and the one or more processors further include or operate to be used with a core/clad ratio adjustment processor or unit that operates to control a ratio of an excitation laser or light between a core and a clad of the double clad fiber, wherein the ratio is one or more of the following: 10% or more than 10% of the excitation laser or light being sent to the clad so that a ratio value for the amount of the excitation laser or light being sent to the core is 90% or less than 90%; 50% or about 50% of the excitation laser or light sent to the clad and 50% or about 50% or more of the excitation laser or light sent to the core; 47% to the clad and 53% to the core; and/or 50%-x% to the clad and 50%+x% to the core where x% is a value equal to a difference between 50% and the percentage value going to the clad; and/or
(iv) the imaging apparatus further comprises a fluorescence sub-system and a sub-system for another imaging modality.

8. The imaging apparatus of claim 1, further comprising a lens unit or one or more lens components that operate to filter an excitation laser or light of a light source and pass through the emission to and/or from the optical probe, the catheter, and/or the one or more components of the optical probe or the catheter, wherein one or more of the following:
(i) the one or more components of the optical probe include or comprise a double clad fiber; and/or
(ii) an optical power of the excitation laser or light into the optical probe and/or the one or more components of the optical probe in total is one of the following: same as a nominal intensity as compared to a case where the excitation laser or light is used as part of a system, the optical probe and/or the one or more components of the optical probe, which is at least 0.1 mW; two (2) times or more higher than the nominal intensity, which is at least 0.2 mW or at least 0.5 mW; ten (10) times or more higher than the nominal intensity, which is at least 1 mW; and/or a hundred (100) times or more higher than the nominal intensity, which is at least 10 mW.

9. A method for photo-bleaching an optical probe and/or one or more components of the optical probe of an imaging apparatus, the method comprising:
using an excitation laser or light with a wavelength of a predetermined range or value on or in the optical probe, the optical probe for use in a catheter and/or in one or more components of the optical probe for a predetermined or set amount of time or more to perform the photo-bleaching such that lower and stabilized background emission noise and/or a high signal to noise ratio is/are achieved for the optical probe and/or for the one or more components of the optical probe.

10. The method of claim 9, wherein:
the predetermined range or value is one or more of the following: 400 nm - 900 nm and/or 635 nm; and
the predetermined or set amount of time is one or more of the following: thirty (30) minutes, thirty (30) minutes or more, in a range of thirty (30) minutes to twenty-four (24) hours, twenty-four (24) hours, twenty-four (24) hours or more, an amount of time calculated or set/received by one or more processors of the imaging apparatus or by a user of the imaging apparatus, and/or an amount of time calculated or set by the one or more processors of the imaging apparatus or by the user of the imaging apparatus based on a size and shape to be photo-bleached or based a number of components or structure to be photo-bleached.

11. The method of claim 9, further comprising one or more of the following:
(i) controlling a ratio of the excitation laser or light between a core and a clad of a double clad fiber of the optical probe and/or the one or more components of the optical probe ; and/or
(ii) passing or coupling an optical power of the excitation laser or light on or in the optical probe and/or the one or more components of the optical probe for the predetermined amount of time to achieve the lower and stabilized background emission noise and/or the high signal to noise ratio.

12. The method of claim 9, wherein one or more of the following:
(i) an emission intensity of the photo-bleached one or more components of the optical probe or the catheter stabilizes within 10% or about 10% of an averaged intensity over a predetermined or set period of time;
(ii) an emission intensity of the photo-bleached one or more components of the optical probe or the catheter stabilize within 10% or about 10% of an averaged intensity over a predetermined or set period of time and/or such that the optical probe or the catheter has a higher signal to noise ratio as compared to an optical probe or catheter having an optical probe without being photo-bleached;
(iii) the method further comprises using one or more detectors of the imaging apparatus to continuously acquire the light or the excitation light in the photo-bleached optical probe and/or in the photo-bleached one or more components of the optical probe or the catheter such that an emission intensity of the photo-bleached optical probe and/or of the photo-bleached one or more components of the optical probe or the catheter stabilizes within 10% or about 10% of an averaged intensity over a predetermined or set period of time and/or such that the optical probe has a higher signal to noise ratio as compared to an optical probe without being photo-bleached;
(iv) an emission intensity of the photo-bleached optical probe and/or of the photo-bleached one or more components of the optical probe stabilizes within 10% or about 10% of an averaged intensity over a predetermined or set period of time, and the predetermined or set period of time is one of the following: two minutes, about two minutes, a period of time in a range of one minute to two minutes, and/or a period of time in a range of about one minute to about two minutes; and/or
(v) the method further comprises using the optical probe while including a double clad fiber.

13. The method of claim 9, further comprising performing a pullback of the optical probe within a catheter and/or obtaining one or more images or frames of one or more imaging modalities from the pullback of the optical probe, wherein one or more of the following:
(i) the imaging apparatus further includes an interference optical system operates to: (a) receive and divide light from a light source into a first light with which an object or sample is to be irradiated and which travels along a sample arm of the interference optical system and a second reference light, (b) send the second reference light along a reference arm of the interference optical system for reflection off of a reference reflection of the interference optical system, and (c) generate interference light by causing reflected or scattered light of the first light with which the object or sample has been irradiated and the reflected second reference light to combine or recombine, and to interfere, with each other, the interference light generating one or more interference patterns; and the imaging apparatus further includes one or more detectors that operate to continuously acquire the interference light and/or the one or more interference patterns to measure the interference or the one or more interference patterns between the combined or recombined light to obtain data for one or more imaging modalities;
(ii) the imaging apparatus further includes an interference optical system operates to: (a) receive and divide light from a light source into a first light with which an object or sample is to be irradiated and which travels along a sample arm of the interference optical system and a second reference light, (b) send the second reference light along a reference arm of the interference optical system for reflection off of a reference reflection of the interference optical system, and (c) generate interference light by causing reflected or scattered light of the first light with which the object or sample has been irradiated and the reflected second reference light to combine or recombine, and to interfere, with each other, the interference light generating one or more interference patterns; and the imaging apparatus further includes one or more detectors that operate to continuously acquire the interference light and/or the one or more interference patterns to measure the interference or the one or more interference patterns between the combined or recombined light to obtain data for one or more imaging modalities, wherein a wavelength of the first light is shorter than a wavelength of the reflected or scattered light and/or the generated interference light; and/or
(iii) the one or more imaging modalities include one or more of the following: Optical Coherence Tomography (OCT), single modality OCT, multi-modality OCT, swept source OCT, optical frequency domain imaging (OFDI), intravascular ultrasound (IVUS), another lumen image(s) modality, near-infrared spectroscopy (NIRS), near-infrared fluorescence (NIRF), near-infrared auto-fluorescence (NIRAF), near-infrared, fluorescence, and/or an intravascular imaging modality.

14. The method of claim 13, further comprising displaying the one or more images or frames on a display, storing the one or more images or frames in a memory, or using the one or more images or frames to train one or more models or AI-networks to auto-detect or to perform photo-bleaching and/or to automatically obtain one or more images or frames of the one or more imaging modalities, wherein one or more of the following:
(i) the trained model is one or a combination of the following: a neural net model or neural network model, a deep convolutional neural network model, a recurrent neural network model with long short-term memory that can take temporal relationships across images or frames into account, a generative adversarial network (GAN) model, a consistent generative adversarial network (cGAN) model, a three cycle-consistent generative adversarial network (3cGAN) model, a model that can take temporal relationships across images or frames into account, a model that can take temporal relationships into account including tissue location(s) and/or photo-bleach location(s) during pullback in a vessel and/or including tissue and/or photo-bleach characterization data during pullback in a vessel, a model that can use prior knowledge about a procedure and incorporate the prior knowledge into the machine learning algorithm or a loss function, a model using feature pyramid(s) that can take different image resolutions into account, and/or a model using residual learning technique(s), a segmentation model, a segmentation model with post-processing, a model with pre-processing, a model with post-processing, a segmentation model with pre-processing, a deep learning or machine learning model, a semantic segmentation model or classification model, an object detection or regression model, an object detection or regression model with pre-processing or post-processing, a combination of a semantic segmentation model and an object detection or regression model, a model using repeated segmentation model technique(s), a model using feature pyramid(s), a genetic algorithm that operates to breed multiple models for improved performance, and/or a model using repeated object detection or regression model technique(s); and/or
(ii) the method further comprises using one or more neural networks or convolutional neural networks to one or more of: load a trained model of images including photo-bleached area(s); perform photo-bleaching on the optical probe and/or the catheter; determine whether the photo-bleached area(s) is/are accurate or correct; determine one or more of the characteristics of one or more objects, targets, or samples in the one or more images; identify or detect the one or more objects, targets, or samples; overlay data on at least one of the one or more images to show location(s) of intravascular image(s), the photo-bleached area(s), or the objects, targets, or samples; incorporate image processing and machine learning (ML) or deep learning to automatically identify and locate photo-bleached portions or components of the optical probe or the catheter; incorporate image processing and machine learning (ML) or deep learning to automatically identify and locate the one or more objects, targets, or samples; display the results for the photo-bleach identification/detection or characterization on a display; and/or acquire or receive image data during the pullback operation of the optical probe.

15. The method of claim 9, further comprising one or more of the following:
(i) using a light source that operates to produce the excitation laser or light;
(ii) using a light source that operates to produce the excitation laser or light, the light source producing the excitation laser or light having a wavelength of 400 nm - 900 nm or 635 nm;
(iii) using a light source that operates to produce the excitation laser or light, the light source producing the excitation laser or light and coupling the excitation laser or light into the optical probe and/or one or more components of the optical probe;
(iv) using a light source that operates to produce the excitation laser or light, the light source producing the excitation laser or light and exciting the optical probe and/or one or more components of the optical probe and/or the catheter with the excitation laser or light for more than or equal to the predetermined or set amount of time; and/or
(v) using a light source that operates to produce the excitation laser or light, the light source producing the excitation laser or light and exciting the optical probe and/or one or more components of the optical probe with the excitation laser or light for more than or equal to the predetermined or set amount of time, where the predetermined or set amount of time is one or more of the following: thirty (30) minutes, thirty (30) minutes or more, in a range of thirty (30) minutes to twenty-four (24) hours, twenty-four (24) hours, twenty-four (24) hours or more, an amount of time calculated or set/received by one or more processors of the imaging apparatus or by a user of the imaging apparatus, and/or an amount of time calculated or set by the one or more processors of the imaging apparatus or by the user of the imaging apparatus based on a size and shape to be photo-bleached or based a number of components or structure to be photo-bleached.

16. The method of claim 9, wherein, in a case where the optical probe or the one or more components of the optical probe include or are attached to a double clad fiber, one or more of the following exists:
(i) the imaging apparatus further comprises one or more processors that operate to perform a pullback of the optical probe within a catheter and/or obtain one or more images or frames of one or more imaging modalities from the pullback of the optical probe;
(ii) the imaging apparatus further comprises: one or more processors that operate to perform a pullback of the optical probe within a catheter and/or obtain one or more images or frames of one or more imaging modalities from the pullback of the optical probe, and the one or more processors further include or operate to be used with a core/clad ratio adjustment processor or unit that operates to control a ratio of the excitation laser or light between a core and a clad of the double clad fiber;
(iii) the imaging apparatus further comprises: one or more processors that operate to perform a pullback of the optical probe within a catheter and/or obtain one or more images or frames of one or more imaging modalities from the pullback of the optical probe, and the one or more processors further include or operate to be used with a core/clad ratio adjustment processor or unit that operates to control a ratio of the excitation laser or light between a core and a clad of the double clad fiber, wherein the ratio is one or more of the following: 10% or more than 10% of the excitation laser or light being sent to the clad so that a ratio value for the amount of the excitation laser or light being sent to the core is 90% or less than 90%; 50% or about 50% of the excitation laser or light sent to the clad and 50% or about 50% or more of the excitation laser or light sent to the core; 47% to the clad and 53% to the core; and/or 50%-x% to the clad and 50%+x% to the core where x% is a value equal to a difference between 50% and the percentage value going to the clad; and/or
(iv) the imaging apparatus further comprises a fluorescence sub-system and a sub-system for another imaging modality.

17. The method of claim 9, further comprising using a lens unit or one or more lens components that operate to filter the excitation laser or light of a light source and pass through the emission to and/or from the optical probe and/or the one or more components of the optical probe, wherein one or more of the following:
(i) the one or more components of the optical probe include or comprise a double clad fiber; and/or
(ii) an optical power of the excitation laser or light into the optical probe and/or one or more components of the optical probe in total is one of the following: same as a nominal intensity as compared to a case where the excitation laser or light is used as part of a system, the optical probe, and/or the one or more components of the optical probe, which is at least 0.1 mW; two (2) times or more higher than the nominal intensity, which is at least 0.2 mW or at least 0.5 mW; ten (10) times or more higher than the nominal intensity, which is at least 1 mW; and/or a hundred (100) times or more higher than the nominal intensity, which is at least 10 mW.

18. A computer-readable storage medium storing at least one program that operates to cause one or more processors to execute a method for photo-bleaching an optical probe, a catheter, and/or one or more components of the optical probe and/or the catheter of an imaging apparatus, the method comprising:
using an excitation laser or light with a wavelength of a predetermined range or value on or in the optical probe and/or in one or more components of the optical probe for a predetermined or set amount of time or more to perform the photo-bleaching such that lower and stabilized background emission noise and/or a high signal to noise ratio is/are achieved for the optical probe and/or for the one or more components of the optical probe.
